(19) Europäisches Patentamt
European Patent Office
Office européen des brevets

(11) Veröffentlichungsnummer: **0 261 520 B1**

(12) **EUROPÄISCHE PATENTSCHRIFT**

(45) Veröffentlichungstag der Patentschrift: **30.12.92**

(51) Int. Cl.$^5$: **C07F 7/08**, C08F 30/08, C08G 77/38, A61K 6/08

(21) Anmeldenummer: **87113306.2**

(22) Anmeldetag: **11.09.87**

(54) **(Meth)-acrysäureester von Tricyclodecangruppen enthaltenden Siloxanen.**

(30) Priorität: **26.09.86 DE 3632657**
**26.09.86 DE 3632792**
**12.03.87 DE 3707908**

(43) Veröffentlichungstag der Anmeldung:
**30.03.88 Patentblatt 88/13**

(45) Bekanntmachung des Hinweises auf die Patenterteilung:
**30.12.92 Patentblatt 92/53**

(84) Benannte Vertragsstaaten:
**AT BE CH DE ES FR GB IT LI NL SE**

(56) Entgegenhaltungen:
**GB-A- 2 023 628**

**CHEMICAL ABSTRACTS, Band 85, Nr. 19, 8. November 1976, Seite 534, Spalte 2, Zusammenfassung Nr. 143184d, Columbus, Ohio, US; A.I. NOGAIDELI et al.: "Hydrosilylation of dicyclopentadiene by some organochlorosilanes and siloxanes", & SOOBSHCH. AKAD. NAUK GRUZ. SSR 1976, 82(3), 589-92**

(73) Patentinhaber: **BAYER AG**

**W-5090 Leverkusen 1 Bayerwerk(DE)**

(72) Erfinder: **Reiners, Jürgen, Dr.**
**Carl-Rumpff-Strasse 57**
**W-5090 Leverkusen 1(DE)**
Erfinder: **Schlak, Ottfired, Dr.**
**Carl-Duisberg-Strasse 331**
**W-5090 Leverkusen 1(DE)**
Erfinder: **Podszun, Wolfgang, Dr.**
**Wolfskaul 4**
**W-5000 Köln 80(DE)**
Erfinder: **Winkel, Jens, Dr.**
**Hahnenweg 6**
**W-5000 Köln 80(DE)**

**EP 0 261 520 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft (Meth)-acrylsäureester von Tricyclodecangruppen enthaltenden Siloxanen, Verfahren zu ihrer Herstellung und ihre Verwendung als Monomere in Dentalmaterialien.

Aus der DE-A 2 922 932 sind Zahnfüllungsmaterialien bekannt, die aus Hydrolysaten von 3-Methacryloyloxypropyl-trialkoxysilanen hergestellt werden.

In der DE-A 3 038 153 wird ein Prothesenbasismaterial beschrieben, das aus Methylmethacrylat, einer Silanverbindung wie 3-Methacryloyloxypropyl-triethoxysilan und einer ungesättigten Carbonsäure erhalten wird.

Die unzureichenden mechanischen Eigenschaften der bekannten Materialien aus Polysiloxanen schließt jedoch ihre Anwendung als Matrix in Dentalmaterialien in der Praxis aus.

Es wurden neue (Meth)-acrylsäureester von Tricyclodecangruppen enthaltenden Siloxanen der Formel

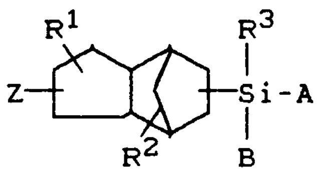

(I),

in der

| | |
|---|---|
| $R^1$ und $R^2$ | gleich oder verschieden sind und Wasserstoff, Niederalkyl, Halogen oder Trifluormethyl bedeuten, |
| $R^3$ | Niederalkyl, Cycloalkyl, Cycloalkyl-alkyl oder gegebenenfalls substituiertes Aryl oder Aralkyl bedeutet, |
| Z | für die Gruppe |

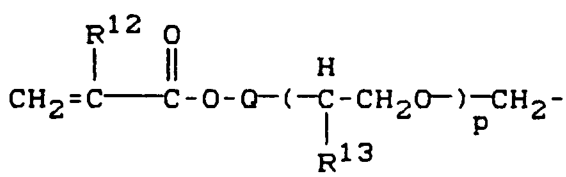

steht,
in der

| | |
|---|---|
| $R^{12}$ und $R^{13}$ | gleich oder verschieden sind und Wasserstoff oder Methyl bedeuten, |
| p | für eine Zahl von 0 bis 20 steht und |
| Q | für eine Einfachbindung steht, einem Rest der Formel |

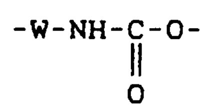

bedeutet,
in der

| | |
|---|---|
| W | eine Alkylenkette bedeutet, oder einen Rest der Formel |

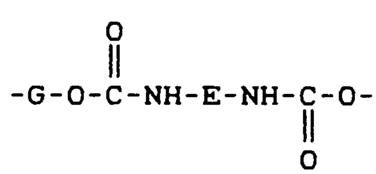

bedeutet,

in der

E ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, aromatischen, araliphatischen und/oder cycloaliphatischen Reste über gegebenenfalls substituierte Methylengruppen verbunden sein können,

und

G einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acryloyloxy-Reste substituiert sein kann, bedeutet,

A für eine Siloxankette steht, die aus m Strukturelementen der Formel

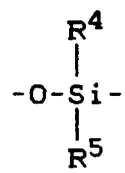

und/oder

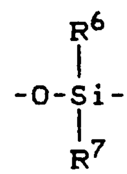

und der Endgruppe

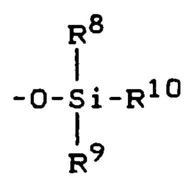

besteht,

wobei

$R^4$, $R^5$, $R^6$, $R^7$, $R^8$ und $R^9$ gleich oder verschieden sind und Niederalkyl, Cycloalkyl, Cycloalkyl-alkyl oder gegebenenfalls substituiertes Aryl oder Aralkyl bedeuten,

wobei die Reste $R^5$, $R^7$ und $R^8$ auch für die Gruppe

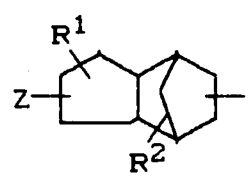

in der

$R^1$, $R^2$ und Z die obengenannte Bedeutung haben,

stehen,

$R^{10}$ für Niederalkyl steht und

wobei die Summe der Strukturelemente m unabhängig voneinander für eine Zahl von 0 bis 600 steht, und

B den Bedeutungsumfang wie A haben kann,

wobei die Reste $R^4$ bis $R^{10}$ in den Ketten A und B unterschiedlich sein können,

oder für Niederalkyl steht,

gefunden.

Die neuen (Meth)-acrylsäureester von Tricyclodecangruppen enthaltenden Siloxanen ergeben nach der Polymerisation Kunststoffe, die die Anforderungen an Dentalmaterialien in der Praxis erfüllen. Insbesondere zeigen sie einen sehr niedrigen Polymerisationsschrumpf und hervorragende mechanische Eigenschaften und eine hohe Stabilität gegenüber der physikalischen und chemischen Degradation im Mundmilieu. Die Monomere zeigen auch bei geringem Siloxangehalt z.B. als Disiloxan, überraschenderweise eine niedrige Viskosität.

Im Rahmen der vorliegenden Erfindung haben die Substituenten im allgemeinen die folgende Bedeutung:

Niederalkyl kann für einen geradkettigen oder verzweigten Alkylrest mit 1 bis etwa 6 Kohlenstoffatomen stehen. Beispielsweise seien Methyl, Ethyl, n-Propyl, iso-Propyl, n-Butyl, iso-Butyl, n-Pentyl, iso-Pentyl, n-Hexyl und iso-Hexyl genannt. Bevorzugte Niederalkylreste sind Methyl und Ethyl.

Halogen kann Fluor, Chlor, Brom oder Iod, bevorzugt Fluor oder Chlor, bedeuten.

Cycloalkyl kann für einen cyclischen, bevorzugt monocyclischen, Kohlenwasserstoffrest mit 5 bis 7 Kohlenstoffatomen stehen. Beispielsweise seien Cyclopentyl, Cyclohexyl und Cycloheptyl genannt. Bevorzugt sind Cyclopentyl und Cyclohexyl.

Cycloalkyl-alkyl kann für einen Rest mit 6 bis 13 Kohlenstoffatomen stehen, wobei ein geradkettiger oder verzweigter Alkylrest ($C_1$ bis $C_6$) durch einen Cycloalkylrest ($C_5$ bis $C_7$) substituiert sein kann. Beispielsweise seien Cyclohexylmethyl, 2-Cyclohexyl-1-ethyl, Cycloheptylmethyl und 2-Cycloheptyl-1-ethyl genannt. Bevorzugt sind Cyclohexylmethyl, 2-Cyclohexyl-1-ethyl.

Aryl kann für einen aromatischen Kohlenwasserstoffrest mit 6 bis 12 Kohlenstoffatomen stehen. Beispielsweise seien Phenyl, Napthyl und Biphenyl genannt. Bevorzugt ist Phenyl.

Aralkyl kann für einen Rest mit 7 bis 18 Kohlenstoffatomen stehen, wobei ein geradkettiger oder verzweigter Alkylrest ($C_1$ bis $C_6$) durch einen aromatischen Rest ($C_6$ bis $C_{12}$) substituiert sein kann. Beispielsweise seien Benzyl, Phenyl-ethyl und Phenyl-propyl genannt. Bevorzugt ist Benzyl.

Die Aryl- und Aralkylreste können gegebenenfalls substituiert sein. Als Substituenten seien beispielsweise Niederalkyl ($C_1$ bis etwa $C_6$), Aryl ($C_6$ bis $C_{12}$) und Halogen, bevorzugt Fluor und Chlor, genannt.

Die Siloxankette (A) besteht aus m Strukturelementen der Formel

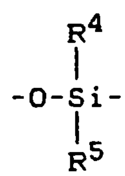

und/oder

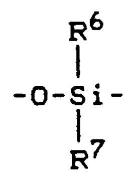

und der Endgruppe

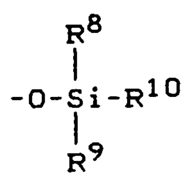

wobei die Substituenten $R^4$ bis $R^{10}$ die obengenannte Bedeutung haben.

Die Strukturelemente können statistisch verteilt oder zu größeren Strukturbereichen (Blöcken) zusammengefaßt sein. Es ist auch möglich, daß die Siloxankette nur aus einer Sorte der Strukturelemente besteht.

Eine Alkylenkette (W) steht im allgemeinen für einen zweibindigen, geradkettigen oder verzweigten Kohlenwasserstoffrest mit 2 bis 10 Kohlenstoffatomen. Bevorzugt werden Alkylenketten mit 2 bis 6 Kohlenstoffatomen. Beispielsweise seien die folgenden Alkylenketten genannt: Ethylen, Propylen, iso-Propylen, 1-Methyl-propylen-(1,3), 1,2-Dimethyl-propylen-(1,3).

Ein zweiwertiger, geradkettiger oder verzweigter aliphatischer Rest E kann einen Kohlenwasserstoffrest mit 2 bis 24 Kohlenstoffatomen, bevorzugt 2 bis 12 Kohlenstoffatomen, bedeuten. Beispielsweise seien die folgenden zweiwertigen aliphatischen Reste genannt: Ethylen, Propylen, 1,4-Tetramethylen, 1,6-Hexamethylen oder 2,2,4-Trimethyl-1,6-hexamethylen und Isomere.

Ein zweiwertiger aromatischer Rest E kann einen Kohlenwasserstoffrest mit 6 bis 26, bevorzugt 6 bis 18, Kohlenstoffatomen bedeuten. Beispielsweise seien die folgenden aromatischen Reste genannt:

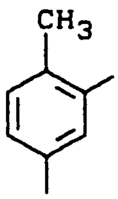 , 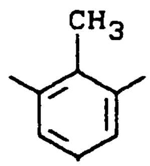 , 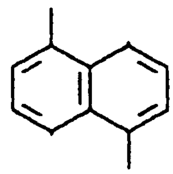 , 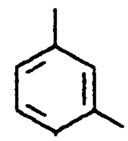 ,

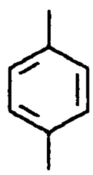 , 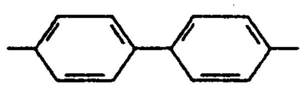 , 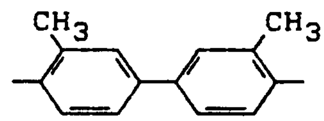 ,

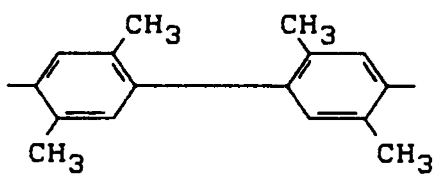 , 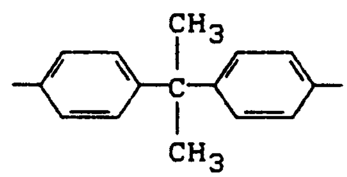 ,

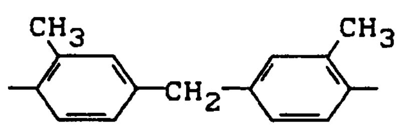 , 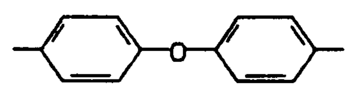 .

Ein zweiwertiger araliphatischer Rest E kann einen Kohlenwasserstoffrest mit einem geradkettigen oder verzweigten aliphatischen und einem aromatischen Teil mit 7 bis 20 Kohlenstoffatomen bedeuten, wobei der aromatische Teil bevorzugt 6 bis 12 und der aliphatische bevorzugt 1 bis 8 Kohlenstoffatome enthält. Beispielsweise seien die folgenden araliphatischen Reste genannt:

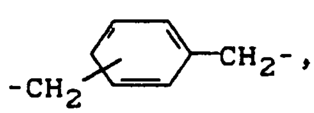 , 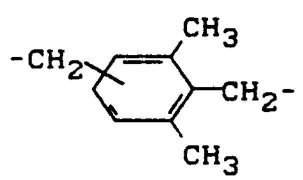 ,

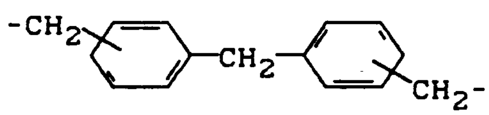 , 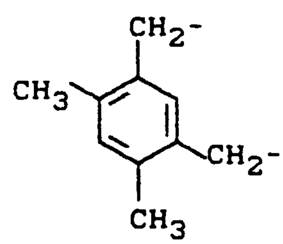 .

Ein zweiwertiger cycloaliphatischer Rest E kann einen Kohlenwasserstoffrest mit 6 bis 26, bevorzugt 6 bis 14 Kohlenstoffatomen bedeuten. Beispielsweise seien genannt:

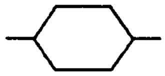 , 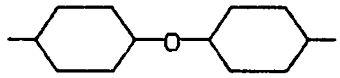 , 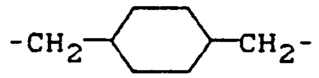 ,

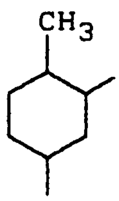 , 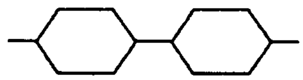 , 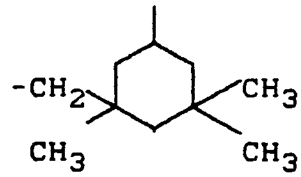,

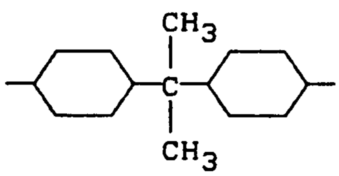 , 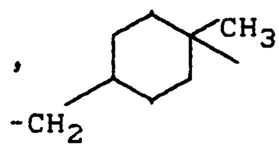 , 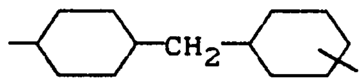 ,

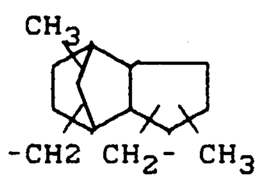 , 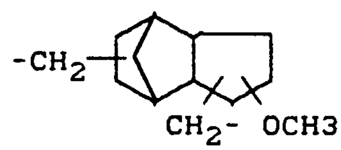

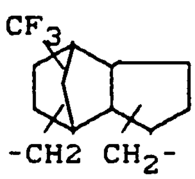 , 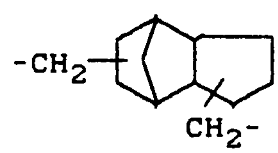 .

Es ist auch möglich, daß mehrere (vorzugsweise 1 bis 3) der genannten aromatischen, araliphatischen und/oder cycloaliphatischen Reste über gegebenenfalls substituierte Methylengruppen verbunden sind.

Gegebenenfalls substituierte Methylengruppen können beispielsweise die Gruppen

$-CH_2-$, 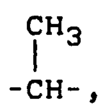, 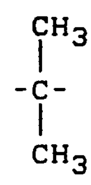 , 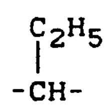 , 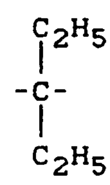

sein.

Ein zweiwertiger Kohlenwasserstoffrest G kann ein geradkettigen oder verzweigten aliphatischen Kohlenwasserstoff mit 3 bis 15 Kohlenstoffatomen, bevorzugt 3 bis 10 Kohlenstoffatomen bedeuten. Der Rest G kann gegebenenfalls 1 bis 3 Sauerstoffbrücken, bevorzugt 1 bis 2 Sauerstoffbrücken enthalten. Es ist auch möglich, daß der Rest G durch 1 bis 4, bevorzugt 1 oder 2 (Meth)-acrylatreste substituiert ist. Beispielsweise seien die folgenden Reste genannt:

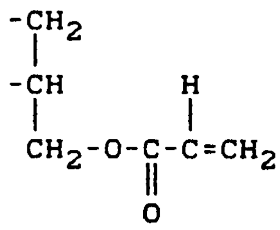 ,

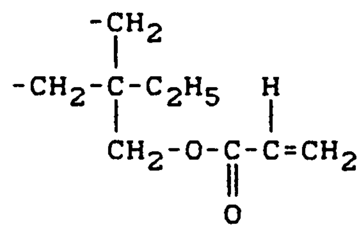 ,

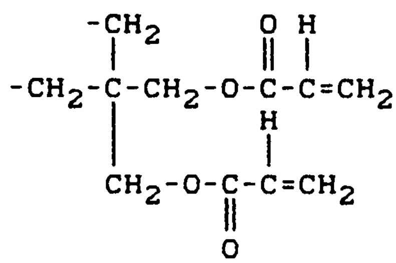 ,

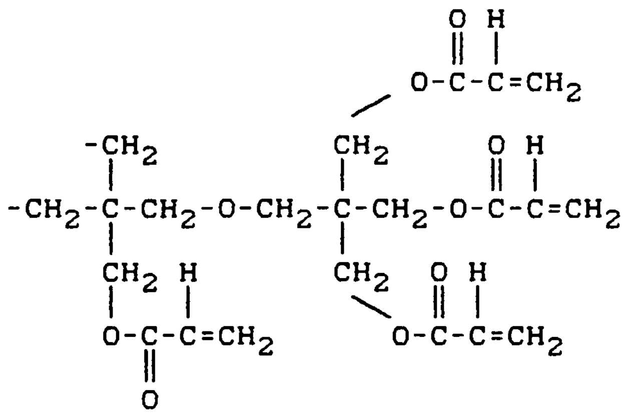

,

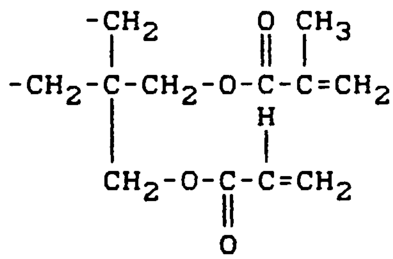

,

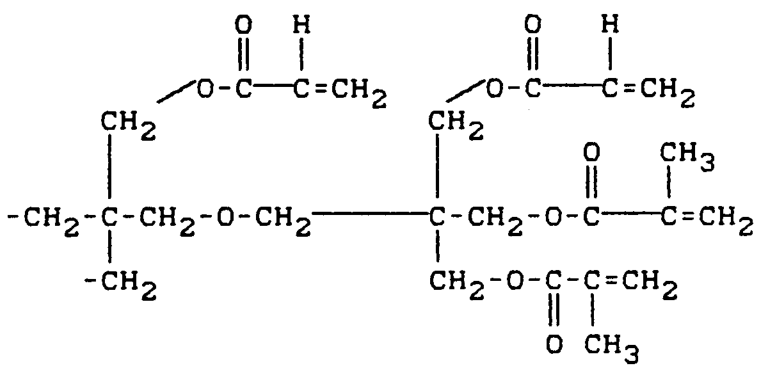

,

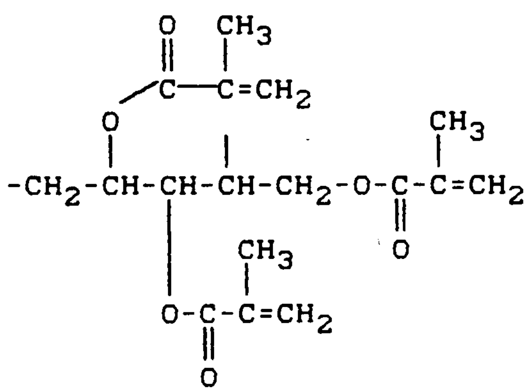

,

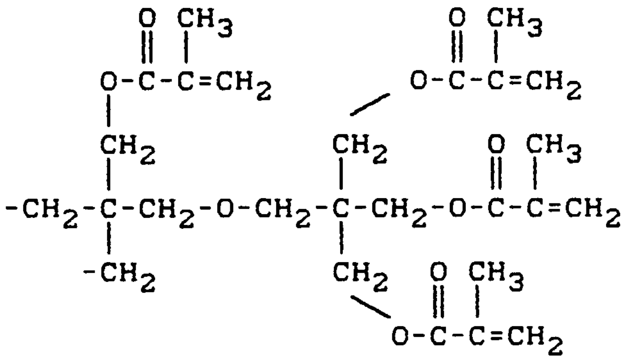 .

Im allgemeinen besteht die Siloxankette aus insgesamt 0 bis 600, bevorzugt 0 bis 200, insbesondere bevorzugt 0 bis 50, Strukturelementen (m). Für den Fall, daß die Zahl der Strukturelemente (m) gleich 0 ist und B für Niederalkyl steht, besteht die Siloxankette nur aus der Endgruppe (Disiloxan).

Für die erfindungsgemäßen Verfahren ist eine Substitution von 0,5 bis 100 Mol-%, besonders von 10 bis 100 Mol-%, aller Silicium-Atome mit Tricyclo[5.2.1.$0^{2,6}$]-decanyl-Gruppen bevorzugt.

Im Rahmen der vorliegenden Erfindung bevorzugt werden (Meth)-acrylsäureester von Tricyclodecangruppen enthaltenden Siloxanen der Formel

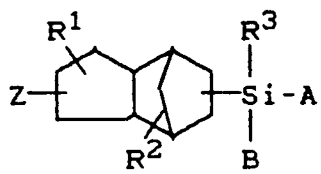 ,

in der

$R^1$ und $R^2$ gleich oder verschieden sind und Wasserstoff, Niederalkyl, Halogen oder Trifluormethyl bedeuten,

$R^3$ Niederalkyl, Cycloalkyl ($C_5$ bis $C_7$), Cycloalkyl-alkyl ($C_6$ bis $C_{13}$) oder gegebenenfalls substituiertes Aryl ($C_6$ bis $C_{12}$) oder Aralkyl ($C_7$ bis $C_{18}$) bedeutet,

Z für die Gruppe

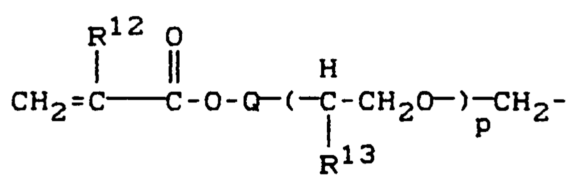

steht,

in der

$R^{12}$ und $R^{13}$ gleich oder verschieden sind und Wasserstoff oder Methyl bedeuten,

p für eine Zahl von 0 bis 20 steht und

Q für eine Einfachbindung steht oder einen Rest der Formel

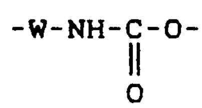

bedeutet,

in der

W eine Alkylenkette mit 2 bis 10 Kohlenstoffatomen bedeutet, oder einen Rest der Formel

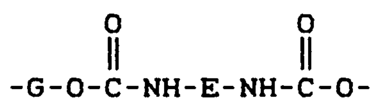

bedeutet,

in der

E ein zweiwertiger, geradkettiger oder verzweigter aliphatischer Rest mit 2 bis 12 Kohlenstoffatomen, ein aromatischer Rest mit 6 bis 18 Kohlenstoffatomen oder ein cycloaliphatischer Rest mit 6 bis 14 Kohlenstoffatomen ist, wobei die aliphatischen, aromatischen und/oder cycloaliphatischen Reste 1 oder 2 Sauerstoffbrücken enthalten können, und wobei mehrere der aliphatischen, aromatischen und/oder cycloaliphatischen Reste über gegebenenfalls substituierte Methylengruppen verbunden sein können,

und

G einen zweiwertigen geradkettigen oder verzweigten aliphatischen Kohlenwasserstoffrest mit 3 bis 10 Kohlenstoffatomen, der gegebenenfalls 1 bis 3 Sauerstoffbrücken enthalten kann und gegebenenfalls durch 1 oder 2 zusätzliche (Meth)-acryloyloxy-Reste substituiert sein kann, bedeutet,

A für eine /Siloxankette steht, die aus m Strukturelementen der Formel

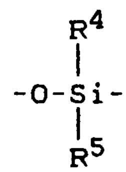

und/oder

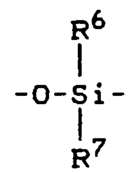

und der Endgruppe

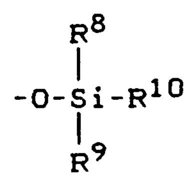

besteht,

wobei

$R^4$, $R^5$, $R^6$, $R^7$, $R^8$ und $R^9$ gleich oder verschieden sind und Niederalkyl, Cycloalkyl ($C_5$ bis $C_7$), Cycloalkyl-alkyl ($C_6$ bis $C_{13}$) oder gegebenenfalls substituiertes Aryl ($C_6$ bis $C_{12}$) oder Aralkyl ($C_7$ bis $C_{18}$) bedeuten,

wobei die Reste $R^5$, $R^7$ und $R^8$ auch für die Gruppe

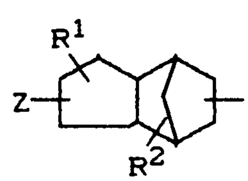

| | |
|---|---|
| | in der |
| $R^1$, $R^2$ und Z | die obengenannte Bedeutung haben, |
| | stehen, |
| $R^{10}$ | für Niederalkyl steht und |
| | wobei die Summe der Strukturelemente m unabhängig voneinander für eine Zahl von 0 bis 200 steht, und |
| B | den Bedeutungsumfang wie A haben kann, |
| | wobei die Reste $R^4$ bis $R^{10}$ in den Ketten A und B unterschiedlich sein können, |
| | oder für Niederalkyl steht. |

Insbesondere bevorzugt werden außerdem (Meth)acrylsäureester von Tricyclodecangruppen enthaltenden Siloxanen der Formel

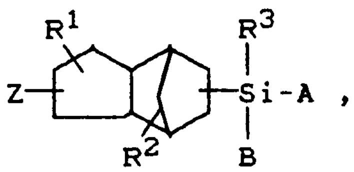

in der

| | |
|---|---|
| $R^1$ und $R^2$ | gleich oder verschieden sind und Wasserstoff oder Niederalkyl bedeuten, |
| $R^3$ | Niederalkyl, Cycloalkyl ($C_5$ bis $C_7$), Cycloalkyl-alkyl ($C_6$ bis $C_{10}$) oder gegebenenfalls substituiertes Aryl ($C_6$ bis $C_{11}$) oder Aralkyl ($C_7$ bis $C_{11}$) bedeutet, |
| Z | für die Gruppe |

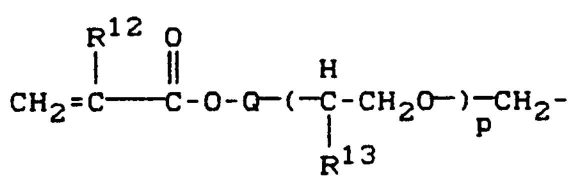

| | |
|---|---|
| | steht, |
| | in der |
| $R^{12}$ und $R^{13}$ | gleich oder verschieden sind und Wasserstoff oder Methyl bedeuten, |
| p | für eine Zahl von 0 bis 4 steht und |
| Q | für eine Einfachbindung steht oder einen Rest der Formel |

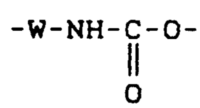

| | |
|---|---|
| | bedeutet, |
| | in der |
| W | eine Alkylenkette mit 2 bis 6 Kohlenstoffatomen bedeutet, |
| | oder einen Rest der Formel |

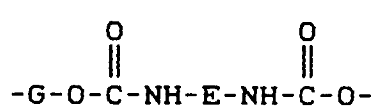

bedeutet,
in der

E ein zweiwertiger, geradkettiger oder verzweigter aliphatischer Rest mit 2 bis 8 Kohlenstoffatomen oder ein cycloaliphatischer Rest mit 6 bis 14 Kohlenstoffatomen ist,
und

G einen zweiwertigen geradkettigen oder verzweigten aliphatischen Kohlenwasserstoffrest mit 3 bis 10 Kohlenstoffatomen, der gegebenenfalls 1 Sauerstoffbrücke enthalten kann und gegebenenfalls durch 1 oder 2 zusätzliche (Meth)-acryloyloxy-Reste substituiert sein kann, bedeutet,

A für eine Siloxankette steht, die aus m Sturkturelementen der Formel

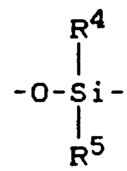
und/oder
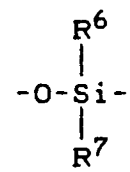

und der Endgruppe

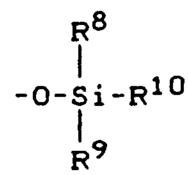

besteht,
wobei

$R^4$, $R^5$, $R^6$, $R^7$, $R^8$ und $R^9$ gleich oder verschieden sind und Niederalkyl, Cycloalkyl ($C_5$ bis $C_7$), Cycloalkyl-alkyl ($C_6$ bis $C_{10}$) oder gegebenenfalls substituiertes Aryl ($C_6$ bis $C_{11}$) oder Aralkyl ($C_7$ bis $C_{11}$) bedeuten,
wobei die Reste $R^5$, $R^7$ und $R^8$ auch für die Gruppe

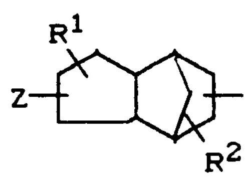

in der

$R^1$, $R^2$ und Z die obengenannte Beduetung haben,
stehen,

$R^{10}$ für Niederalkyl steht und
wobei die Summe der Strukturelemente m unabhängig voneinander für eine Zahl von 0 bis 200 steht, und

B den Bedeutungsumfang wie A haben kann,
wobei die Reste $R^4$ bis $R^{10}$ in den Ketten A und B unterschiedlich sein können,
oder für Niederalkyl steht.

Beispielsweise seien die folgenden (Meth)acrylsäureester von Tricyclodecangruppen enthaltenden Siloxanen genannt:

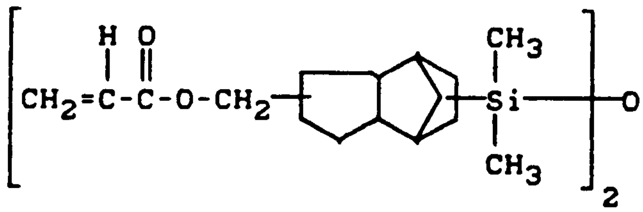

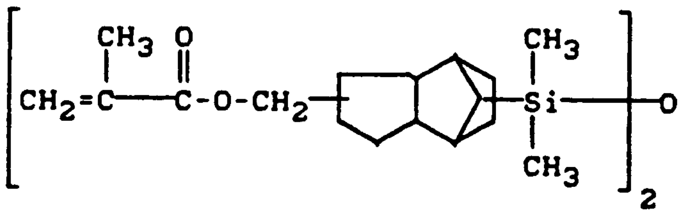

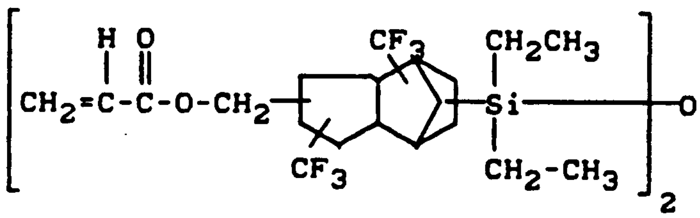

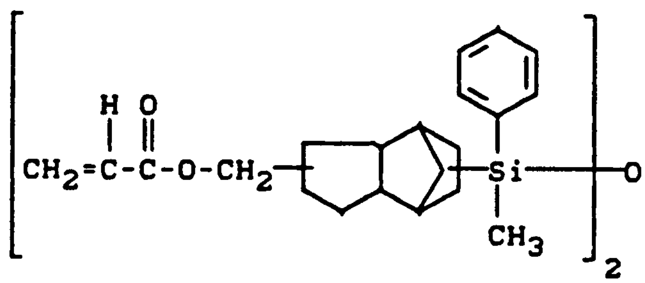

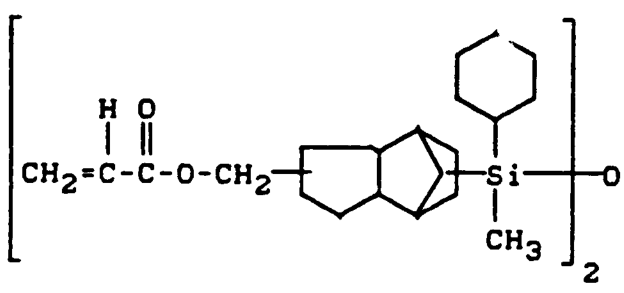

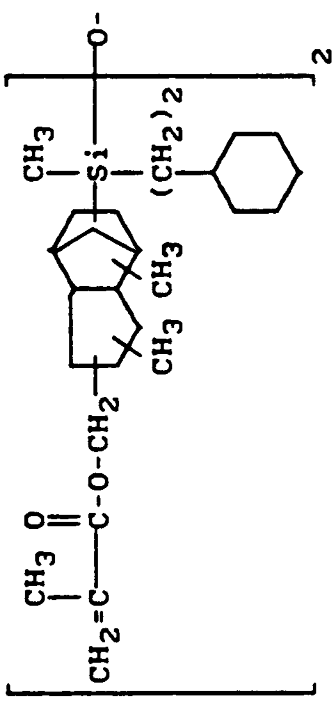

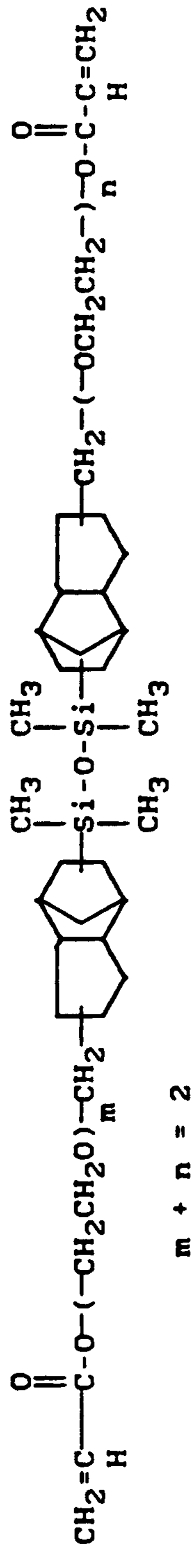

m + n = 2

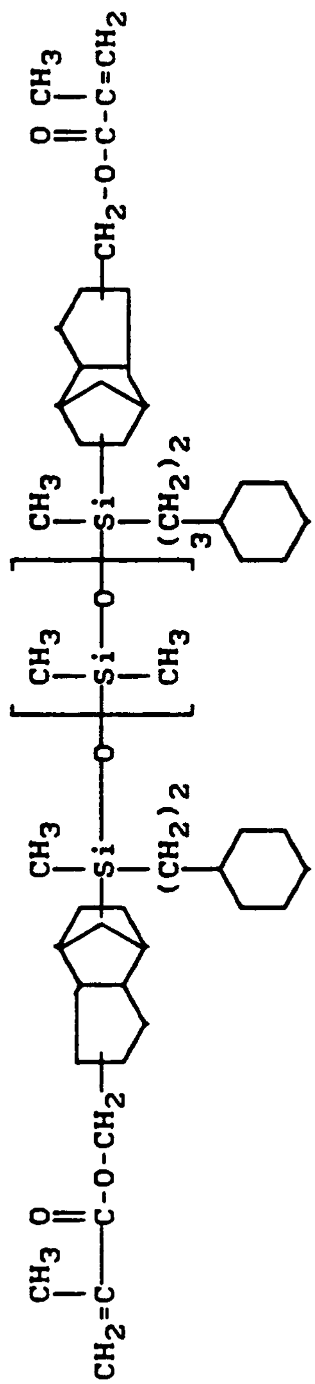

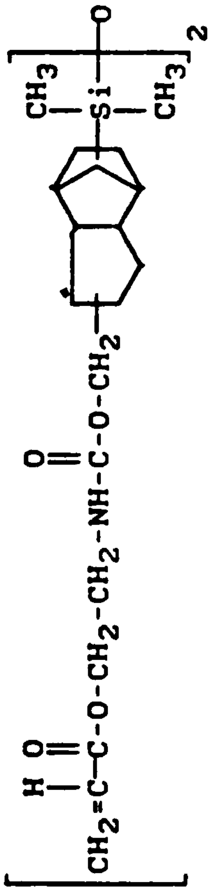

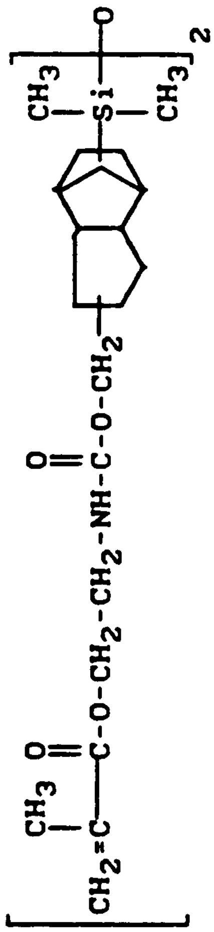

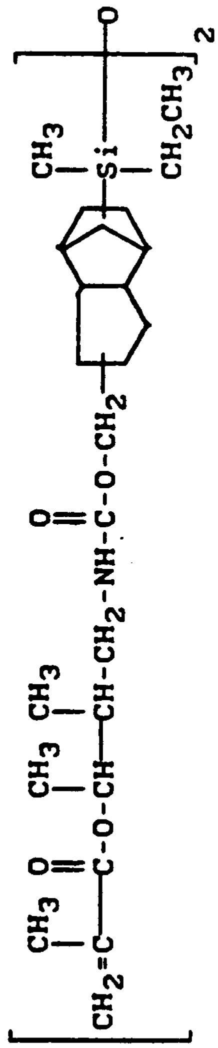

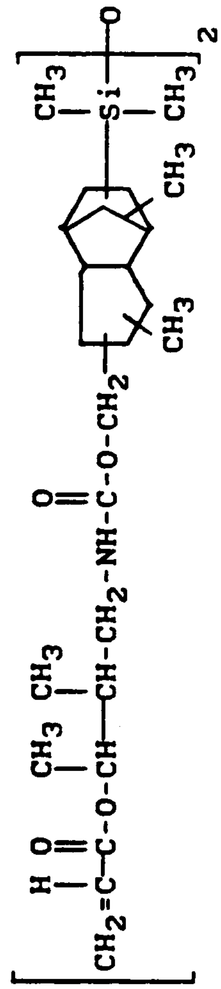

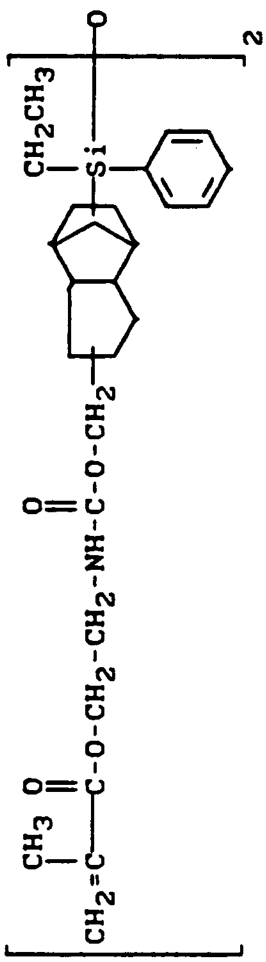

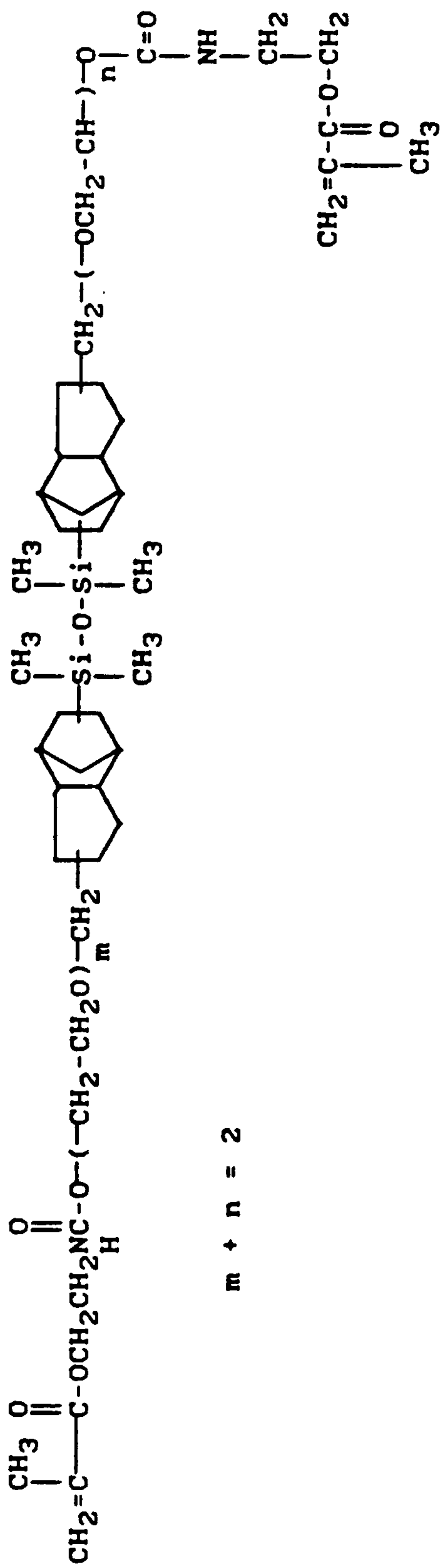

m + n = 2

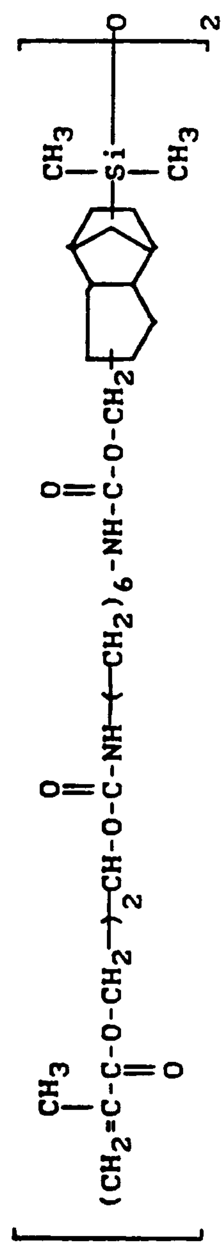

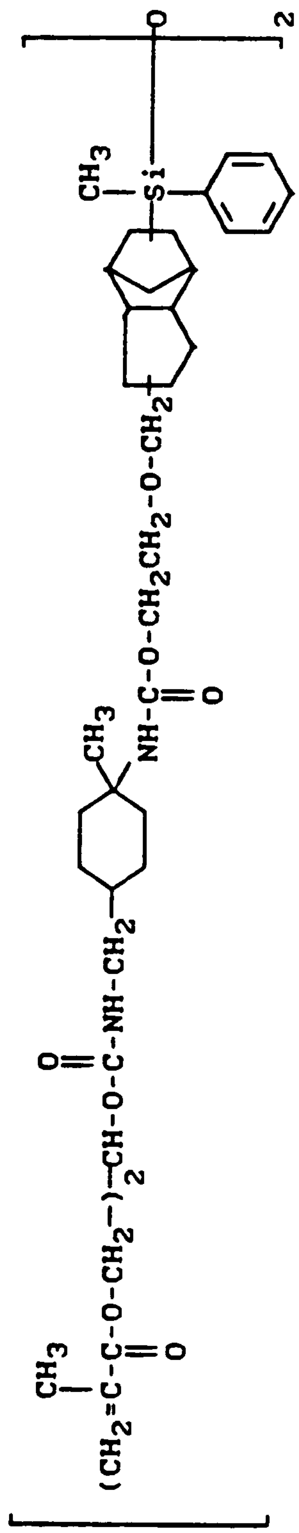

Es wurde auch ein Verfahren zur Herstellung (Meth)acrylsäureester von Tricyclodecangruppen enthaltenden Siloxanen der Formel

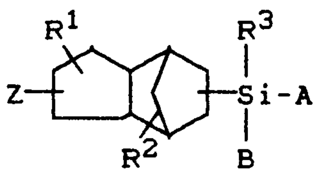 (I),

in der

$R^1$ und $R^2$ gleich oder verschieden sind und Wasserstoff, Niederalkyl, Halogen oder Trifluormethyl bedeuten,

$R^3$ Niederalkyl, Cycloalkyl, Cycloalkyl-alkyl oder gegebenenfalls substituiertes Aryl oder Aralkyl bedeutet,

Z für die Gruppe

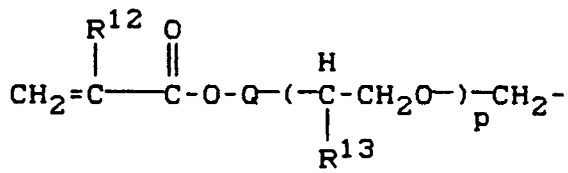

steht,

in der

$R^{12}$ und $R^{13}$ gleich oder verschieden sind und Wasserstoff oder Methyl bedeuten,

p für eine Zahl von 0 bis 20 steht und

Q für eine Einfachbindung steht oder einen Rest der Formel

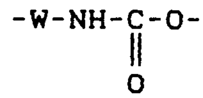

bedeutet,

in der

W eine Alkylenkette bedeutet,

oder einen Rest der Formel

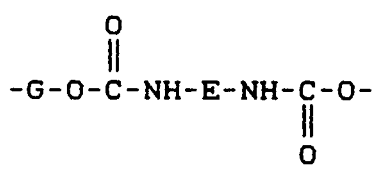

bedeutet,

in der

E ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, aromatischen, araliphatischen und/oder cycloaliphatischen Reste über gegebenenfalls substituierte Methylengruppen verbunden sein können,

und

G — einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)acryloyloxy-Reste substituiert sein kann, bedeutet,

A — für eine Siloxankette steht, die aus m Strukturelementen der Formel

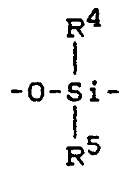

und/oder

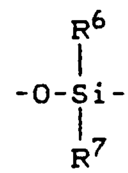

und der Endgruppe

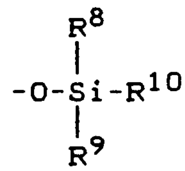

besteht,
wobei

$R^4$, $R^5$, $R^6$, $R^7$, $R^8$ und $R^9$ — gleich oder verschieden sind und Niederalkyl, Cycloalkyl, Cycloalkyl-alkyl oder gegebenenfalls substituiertes Aryl oder Aralkyl bedeuten,
wobei die Reste $R^5$, $R^7$ und $R^8$ auch für die Gruppe

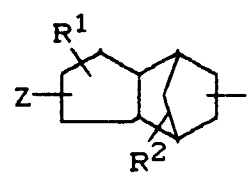

in der

$R^1$, $R^2$ und Z — die obengenannte Bedeutung haben,
stehen,

$R^{10}$ — für Niederalkyl steht und
wobei die Summe der Strukturelemente m unabhängig voneinander für eine Zahl von 0 bis 600 steht, und

B — den Bedeutungsumfang wie A haben kann,
wobei die Reste $R^4$ bis $R^{10}$ in den Ketten A und B unterschiedlich sein können,
oder für Niederalkyl steht,

gefunden, das dadurch gekennzeichnet ist, daß man Poly[Hydroxy-(alkylenoxy)methyl-tricyclo-[5.2.1.0$^{2.6}$]-decanyl]-siloxane der Formel

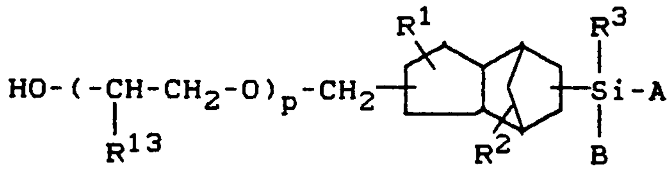

(II),

in der

$R^1$, $R^2$, $R^3$, $R^{13}$, p, A und B die obengenannte Bedeutung haben,

für den Fall der Herstellung von Verbindungen, in denen Q für eine Einfachbindung steht, mit einem (Meth)acrylsäure-Derivat der Formel

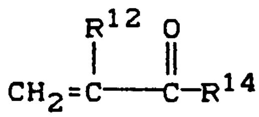

(III),

in der

$R^{12}$ die obengenannte Bedeutung hat, und

$R^{14}$ für Hydroxy, Chlor, Methoxy, Ethoxy oder (Meth)Acryloyloxy steht, verestert,

oder

für den Fall der Herstellung von Verbindungen, in denen Q für einen Rest der Formel

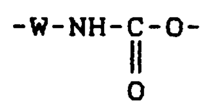

steht,

in der

W für eine Alkylenkette steht,

mit einem Isocyanat der Formel

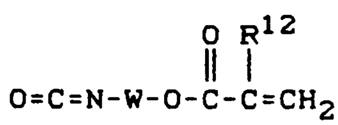

(IV),

in der

$R^{12}$ und W die obengenannte Bedeutung haben,

in einem inerten Lösungsmittel umsetzt,

oder für den Fall der Herstellung von Verbindungen, in denen Q für einen Rest der Formel

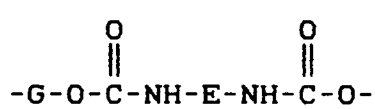

steht,

in der

E ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, aromatischen, araliphatischen und/oder cycloaliphatischen Reste über gegebenenfalls substituierte Methylengruppen verbunden sein können,

und

G einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acryloyloxy-Reste substituiert sein kann, bedeutet,

mit dem Additionsprodukt aus 1 Mol eines Diisocyanats der Formel

O = C = N-E-N = C = O (V),

in der

E die oben angegebene Bedeutung hat,

und 1 Mol eines Hydroxyalkyl(meth)acrylsäureesters der Formel

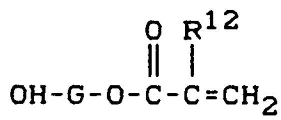

(VI),

in der

G und $R^{12}$ die oben angegebene Bedeutung haben,

wobei eine stöchiometrische Äquivalenz zwischen den NCO-Gruppen des Adduktes aus V und VI und den OH-Gruppen von II bestehen soll,

in einem inerten Lösungsmittel umsetzt.

Das erfindungsgemäße Verfahren kann durch das folgende Formelschema erläutert werden:

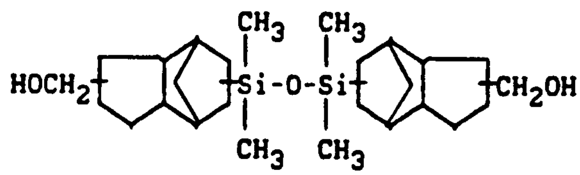

1

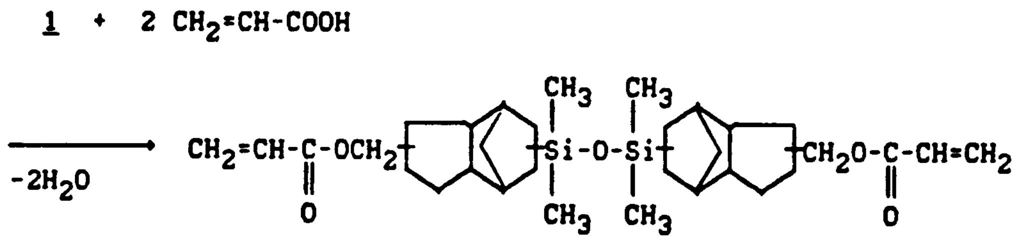

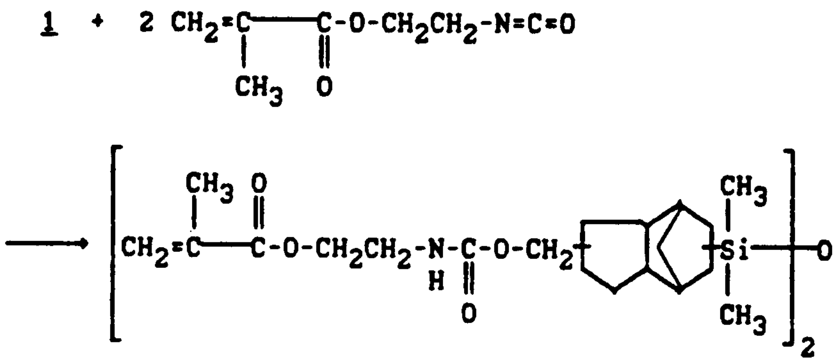

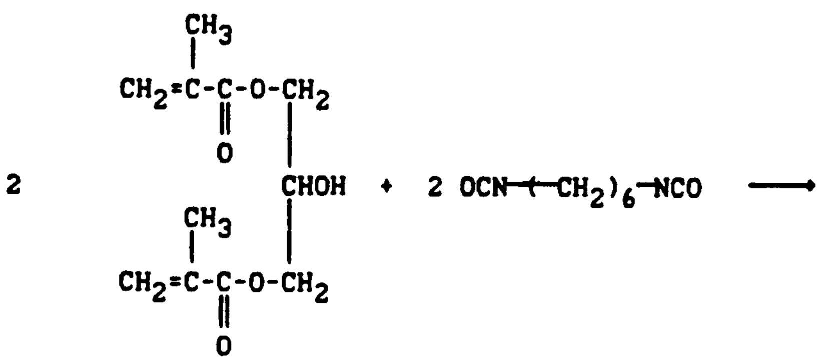

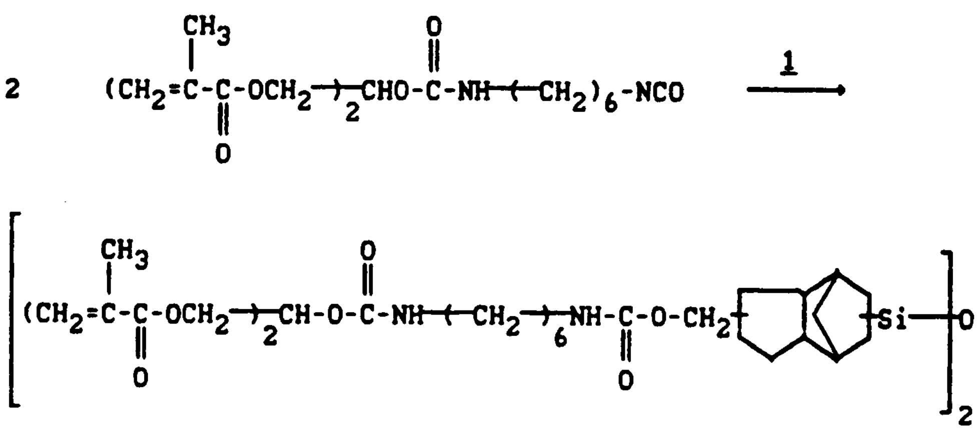


Poly[Hydroxy(alkylenoxy)methyl(tricyclo-[5.2.1.$0^{2.6}$]-decanyl)]-siloxane der Formel

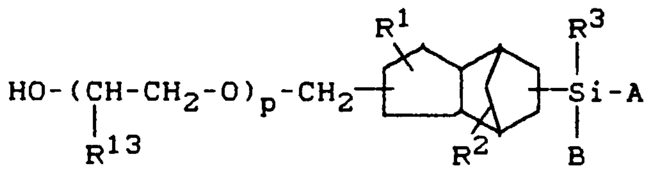 (II),

in der

$R^1$ und $R^2$ gleich oder verschieden sind und Wasserstoff, Niederalkyl, Halogen oder Trifluormethyl bedeuten,

$R^3$ Niederalkyl, Cycloalkyl, Cycloalkyl-alkyl oder gegebenenfalls substituiertes Aryl oder Aralkyl bedeutet,

$R^{13}$ Wasserstoff oder Methyl bedeutet,

p für eine ganze Zahl von 0 bis 20 steht,

A für eine Siloxankette steht, die aus m Strukturelementen der Formel

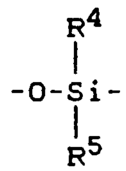 und/oder 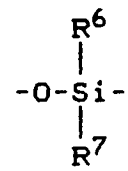

und der Endgruppe

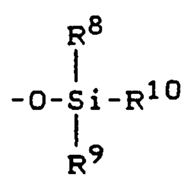

besteht,

wobei

$R^4$, $R^5$, $R^6$, $R^7$, $R^8$ und $R^9$ gleich oder verschieden sind und Niederalkyl, Cycloalkyl, Cycloalkyl-alkyl oder gegebenenfalls substituiertes Aryl oder Aralkyl bedeuten,

wobei die Reste $R^5$, $R^7$ und $R^8$ auch für die Gruppe

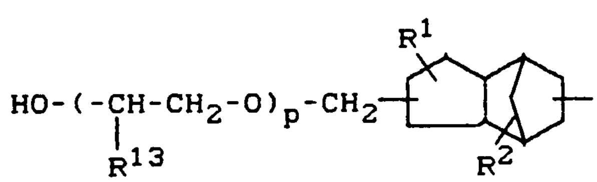

in der

$R^1$, $R^2$, $R^{13}$ und p die obengenannte Bedeutung haben,

stehen,

$R^{10}$ für Niederalkyl steht und

wobei die Anzahl der Stukturelemente m unabhängig voneinander jeweils für eine Zahl von 0 bis 600 steht, und

B den Bedeutungsumfang wie A haben kann,

wobei die Reste $R^4$ bis $R^{10}$ in den Ketten A und B unterschiedlich sein können,

oder für Niederalkyl steht,

können hergestellt werden, indem man ein Tricyclo-[5.2.1.0$^{2.6}$]-decenyl-

silan der Formel

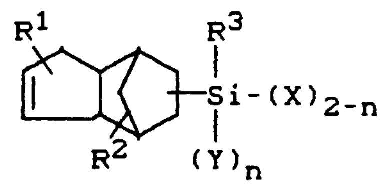 (VII),

in der

| | |
|---|---|
| $R^1$, $R^2$ und $R^3$ | die obengenannte Bedeutung haben, |
| X | für einen hydrolysierbaren Rest steht, |
| Y | für Niederalkyl steht, und |
| n | für die Zahl 0 oder 1 steht, |

in Gegenwart von Wasser und einem Kondensationskatalysators zuerst homokondensiert oder mit Diorgano-Silanen der Formeln

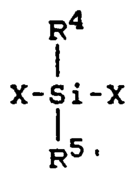 (VIII) und/oder 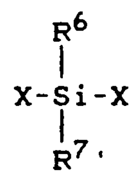 (IX),

cokondensiert
und anschließend, für den Fall, daß n gleich 0 ist, mit Triorgano-Silanen der Formel

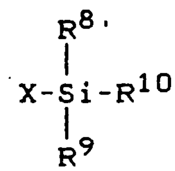 (X),

wobei X, $R^4$, $R^6$, $R^9$ und $R^{10}$ die obengenannte Bedeutung haben, und wobei $R^{5\prime}$, $R^{7\prime}$, $R^{8\prime}$ gleich oder verschieden sind und Niederalkyl, Cycloalkyl, Cycloalkylalkyl oder gegebenenfalls substituiertes Aryl oder Aralkyl bedeuten oder auch für die Gruppe

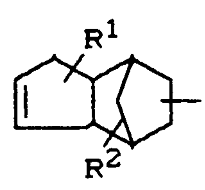 ,

in der $R^1$ und $R^2$ die obengenannte Bedeutung haben,
stehen,
umsetzt,
wobei das Molverhältnis der Silane VII zu den Silanen X nach der Homokondensation der Silane VII bzw. nach der Cokondensation der Silane VII mit den Silanen VIII und/oder IX größer oder gleich 0,5 beträgt und wobei für die Cokondensation m Mol der Silane VIII und/oder IX, bezogen auf 1 Mol Silan der Formel VII eingesetzt werden,

wobei m die oben genannte Bedeutung hat,
und dann das erhaltene Poly(tricyclo[-5.2.1.$0^{2.6}$] decenyl)-siloxan in Gegenwart eines Hydroformylierungskatalysators mit Kohlenmonoxid und Wasserstoff (im Verhältnis von etwa 1:1) umsetzt,
das erhaltene Formyl-Derivat in einer weiteren Stufe zum Hydroxymethyl-Derivat reduziert, und gegebenenfalls (für p>0) das erhaltene Hydroxymethyl-Derivat mit p Mol bezogen auf 1 Mol Hydroxymethylgruppen eines Alkylenoxids der Formel

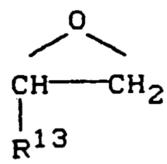

(XI)

wobei
$R^{13}$ und p die obengenannte Bedeutung haben,
in Gegenwart eines basischen Katalysators umsetzt.

Das Herstellungsverfahren kann durch das folgende Reaktionsschema erläutert werden:

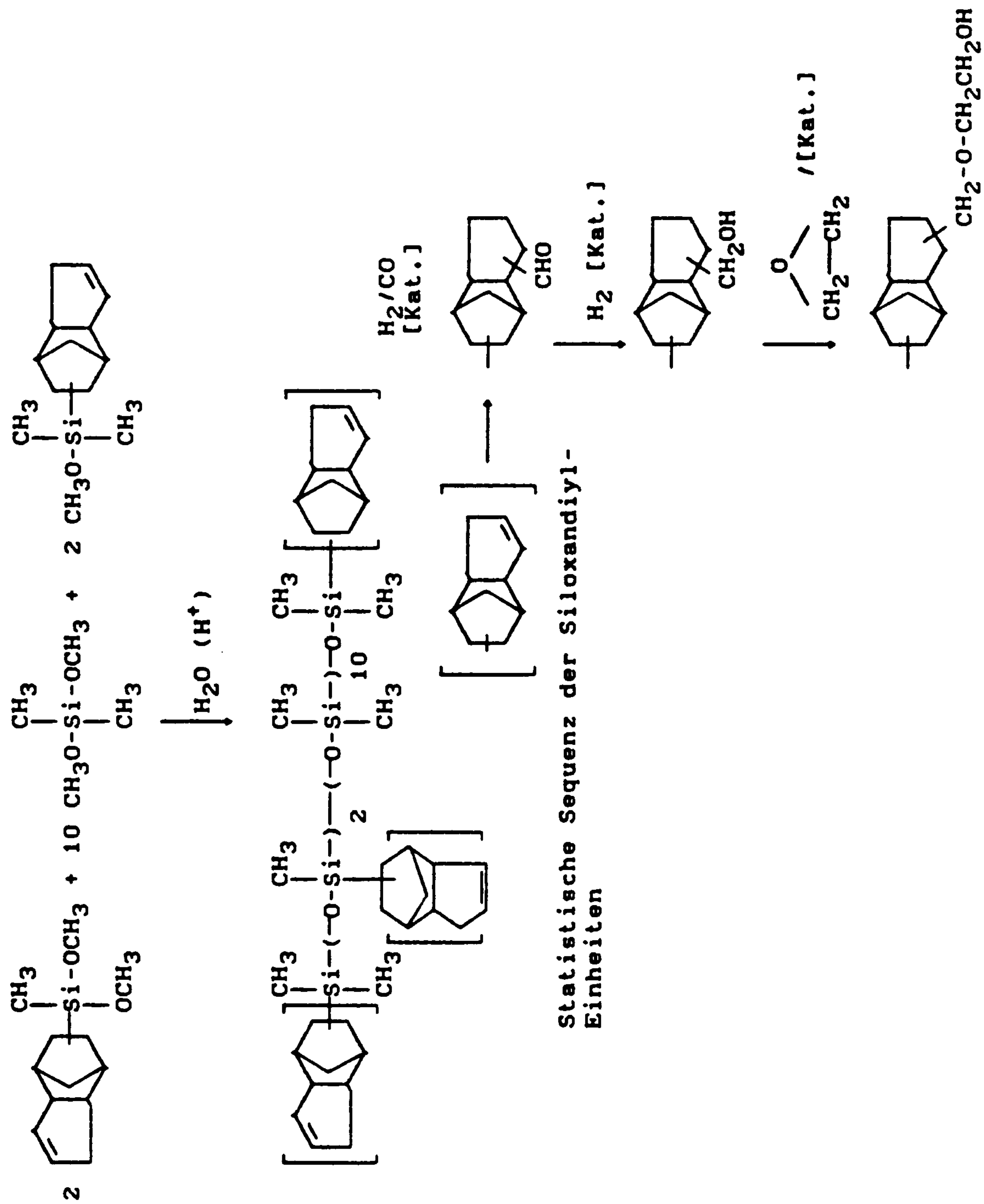


Tricyclo-[5.2.1.$0^{2.6}$]-decenyl-silane (VII) sind an sich bekannt (Chem. Abstr. 94, 15799 und 85, 143 184, 77, 88573), Die Tricyclo-[5.2.1.$0^{2.6}$]decenyl-silane enthalten einen hydrolysierbaren Rest, der bei der Kondensation abgespalten wird. Als hydrolysierbare Reste seien beispielsweise Halogene (Chlor, Brom und Iod, bevorzugt Chlor), Alkoxy ($C_1$ bis $C_6$, bevorzugt Methoxy und Ethoxy), Acyloxy, Dialkylamino (Alkyl, bevorzugt Methyl und Ethyl) genannt.

Die Tricyclo-[5.2.1.$0^{2.6}$]-decenyl-silane können beispielsweise durch Umsetzung von 3(4),8-Tricyclo-[5.2.1.$0^{2.6}$]-decadien-Derivaten mit entsprechenden Hydrogensilanen in Gegenwart von Hydrosilylierungskatalysatoren, z.B. $H_2PtCl_6$, hergestellt werden.

Diorgano-Silane (VIII, IX) sind handelsüblich oder können für den Fall, daß $R^{5'}$ und $R^{7'}$ für einen Tricyclo-[5.2.1.$0^{2,6}$]-decenyl-Rest stehen, durch Umsetzung von 3(4),8-Tricyclo[5.2.1.$0^{2,6}$]decadien-Derivaten mit Hydrogensilanen, z.B. Methyldichlorsilan hergestellt werden. Beispielsweise seien die folgenden Diorganosilane genannt:

Dimethyldichlorsilan, Dimethoxydimethylsilan, Ethylmethyldichlorsilan, Methylphenyldimethoxysilan, Diphenyldiethoxysilan, (2-Cyclohexylethyl-1-yl)-ethyldichlorsilan, Dibutyl-dimethoxysilan, Di-n-propyldichlorsilan, Methyl-Tricyclo[5.2.1.$0^{2,6}$]decenyl-dichlor-silan, Methyl-tricyclo[5.2.1.$0^{2,6}$]decenyl-dimethoxysilan.

Triorganosilane (X) sind handelsüblich oder können für den Fall, daß $R^{8'}$ für einen Tricyclo[5.2.1.$0^{2,6}$]-decenyl-Rest steht, in der für VIII und IX beschriebenen Weise analog hergestellt werden.

Beispielsweise seien die folgenden Triorganosilane genannt: Trimethylchlorsilan, Trimethylmethoxysilan, Diethylpropyl-ethoxysilan, Diphenyl-methyl-methoxysilan, Tricyclo[5.2.1.$0^{2,6}$]decenyl-dimethylchlorsilan, Tricyclo[5.2.1.$0^{2,6}$]decenyldimethylmethoxysilan.

Kondensationskatalysatoren (W. Noll, Chemie und Technologie der Silicone, Verlag Chemie (1968)) für die Homo/Cokondensation sind beispielsweise Halogenwasserstoffsäuren, Schwefelsäure, Alkali-Metallsalze, Alkali- und Erdalkalimetall-hydroxide, -carbonate und -oxide, Trifluoressigsäure, Perfluorbutansulfonsäure und Essigsäure.

Die Homo/Co-Kondensation wird im allgemeinen bei Normaldruck und bei Temperaturen von -20 bis +80 °C mit einem Überschuß Wasser in Gegenwart des Kondensationskatalysators, der z.B. bei Halogensilanen während der Hydrolyse in situ gebildet wird, durchgeführt. Die Homo(co)kondensation kann auch in Gegenwart von Lösungsmitteln, die mit Wasser mischbar oder nicht mischbar sind, durchgeführt werden. Beispielsweise seien folgende Lösungsmittel genannt: Toluol, Dichlormethan, Chloroform, Diethylether, Tetrahydrofuran, Methylethylketon, Aceton, Hexan, Ethanol, Isopropanol, Methanol, Essigsäureethylester.

Nach der Homokondensation von Tricyclo[5.2.1.$0^{2,6}$]decenylsilanen VII, für den Fall, daß n gleich 0 ist, und nach der Cokondensation mit Diorganosilanen VIII und/oder IX wird die wäßrige Phase abgetrennt und das Reaktionsgemisch mit sauren oder basischen Katalysatoren der obengenannten Art, vorzugsweise Perfluorbutansulfonsäure, in einem inerten Lösungsmittel equilibriert. Dabei werden die zunächst entstandenen Gemische von cyclischen und linearen Polysiloxanen in Gemische von linearen Polysiloxanen mit höherer Einheitlichkeit und höherem Molekulargewicht überführt. Der Verlauf der Reaktion kann z.B. durch Messung der Viskosität verfolgt werden.

Nach dem Equilibrierungsschritt entstehen überwiegend Polysiloxane mit Silanol-Endgruppen. Diese Polysiloxane werden anschließend mit Triorganosilanen X in Gegenwart von Kondensations-Katalysatoren umgesetzt, um vorhandene Silanol-Endgruppen in Triorganosiloxy-Endgruppen umzuwandeln.

Es ist aber auch möglich ein Triorganosilan X direkt bei der Homo-/Co-Kondensation einzusetzen, wobei es als Kettenabbrecher wirkt und als Triorganosiloxy-Endgruppe in das Polysiloxan eingeführt wird.

Für den Fall, daß n in Formel VII gleich 1 ist, d.h. das Tricyclo[5.2.1.$0^{2,6}$]decenylsilan VII nur eine hydrolysierbare Gruppe besitzt, erübrigt sich die Umsetzung mit Triorganosilanen X, da aus einem solchen Silan VII, in dem n gleich 1 ist, bei der Homokondensation das entsprechende Disiloxan entsteht und im Fall der Cokondensation mit Diorganosilanen der Formel VI und/oder IX Polysiloxane entstehen, die bereits den Silylrest des Silans VII als Endgruppe enthalten.

Die weitere Umsetzung der Poly-(tricyclo[5.2.1.$0^{2,6}$]decenyl)-Verbindungen zu den Formyl-Derivaten wird im allgemeinen in Gegenwart eines Hydroformylierungskatalysators mit Kohlenmonoxid und Wasserstoff durchgeführt. Kohlenmonoxid und Wasserstoff werden in der Regel im Verhältnis von etwa 1 zu 1 eingesetzt.

Als Hydroformylierungskatalysatoren werden (Houben-Weyl, Methoden der organischen Chemie, Bd. E3, S. 180 ff. (1983)) im allgemeinen Rhodiumkatalysatoren verwandt. Bevorzugt wird beispielsweise der sogenannte Wilkinson-Komplex oder das Bis-(dicarbonylchlororhodium). Insbesondere bevorzugt wird ein heterogener Katalysator, der beispielsweise 5 % Rhodium auf Aluminiumoxid enthält.

Im allgemeinen setzt man $10^{-3}$ bis $10^{-7}$ Mol, bevorzugt $10^{-3}$ bis $10^{-5}$ Mol Rhodium, bezogen auf ein Mol Tricyclo[5.2.1.$0^{2,6}$]decenyl-Gruppen, ein.

Die Hydroformylierung wird im allgemeinen im Druckbereich von 30 bis 250 bar Kohlenmonoxid/Wasserstoff und im Temperaturbereich von 50 bis 180 °C, bevorzugt 70 bis 150 °C durchgeführt.

Die Hydroformylierung kann bevorzugt in Gegenwart eines Lösungsmittels durchgeführt werden. Als Lösungsmittel seien beispielsweise Methylcyclohexan, Toluol und Xylol genannt.

Die erhaltene erfindungsgemäße Formylverbindung kann in an sich bekannter Weise, beispielsweise durch Entfernen des Lösungsmittels und Reinigung über ein Adsorbens, isoliert werden.

Die Hydrierung der Formylverbindungen zu dem Alkohol wird in Gegenwart eines Hydrierungs-Katalysators bei einem Wasserstoffdruck von 5 bis 250 bar, vorzugsweise von 50 bis 200 bar und im Temperaturbereich von 20 bis 180 °C, bevorzugt von 60-150 °C in inerten Lösungsmitteln, z.B. gesättigten Kohlenwasserstoffen oder aliphatischen Alkoholen, durchgeführt. Als gesättigte Kohlenwasserstoffe seien beispielsweise genannt: Cyclohexan, n-Hexan, n-Heptan, Methylcyclohexan. Geeignete Alkohole sind niedere Alkohole, z.B. Methanol, Ethanol, Isopropanol, n-Propanol, n-Butanol.
Aliphatische Alkohole sind als Lösungsmittel bevorzugt.

Der Hydrierungskatalysator ist beispielsweise ein Übergangsmetall, das in Form seiner Salze, Komplexe oder in Form eines Trägerkatalysators eingesetzt wird. Beispielsweise seien folgende Hydrierungskatalysatoren genannt: Tris(triphenylphosphin)chloro-rhodium, Raney-Nickel, Bis(dicarbonylchlororhodium), 5 % Rhodium auf Aluminiumoxid.

Gegebenenfalls können Aktivatoren zur Beschleunigung der Hydrierung zugesetzt werden. Geeignete Aktivatoren sind beispielsweise: Natriumhydroxid, Triethylamin, Tri-n-butylamin, Pyridin, Hexachlorplatinsäure. Der Katalysator wird in einer Menge von 1 bis $10^{-6}$ Mol, bevorzugt $10^{-1}$ bis $10^{-4}$ Mol Metall, bezogen auf 1 Mol Formylgruppen, eingesetzt. Der Aktivator wird in einer Menge von $10^{-1}$ bis $10^{-6}$ Mol, bezogen auf 1 Mol Metall, eingesetzt.

Die Hydrierung wird IR-spektroskopisch verfolgt und solange fortgesetzt, bis eine quantitative Reduktion aller Formylgruppen erreicht ist.

Das enstandene Poly(Hydroxymethyl-tricyclo[5.2.1.$0^{2,6}$]decanyl)siloxan kann nach beendeter Hydrierung durch Filtration und/oder unter Anwendung von Adsorbentien wie Celite®, Silicagel oder Aluminiumoxid von Katalysator und Verunreinigungen befreit und durch Entfernen des Lösungsmittels im Vakuum isoliert werden. Man erhält die Siloxanderivate als farblose hochviskose bis wachsartige Substanzen. Gegebenenfalls kann eine Reinigung auch durch Vakuumdestillation vorgenommen werden, sofern die Produkte z.B. Disiloxane sind.

Dieses Poly(hydroxymethyl-tricyclo[5.2.1.$0^{2,6}$]decanyl)siloxan kann gegebenenfalls in Gegenwart von basischen Katalysatoren mit 1-20 Mol (bezogen auf 1 Mol Hydroxymethylgruppen) eines Alkylenoxids, z.B. Oxiran oder Methyloxiran, bei Temperaturen von 70 bis 150 °C, vorzugsweise 90 bis 120 °C, zu Verbindungen der Formel II umgesetzt werden, wobei das Alkylenoxid zweckmäßigerweise nach Maßgabe seines Verbrauchs zudosiert wird.

Als basische Katalysatoren werden vorzugsweise Alkali-oder Erdalkali-Alkoholate verwendet, welche auch in situ hergestellt werden können.

Ein weiteres Verfahren zur Herstellung von α,ω-Bis[hydroxy(alkylenoxy)methyl-tricyclo[5.2.1.$0^{2,6}$]-decanyl]siloxanen der Formel

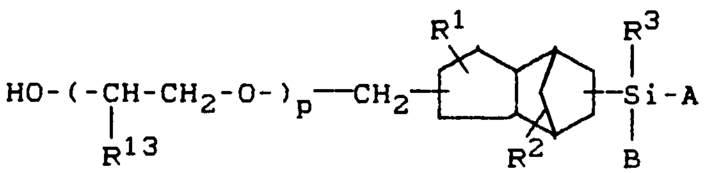

(II),

in der

$R^1$ und $R^2$ gleich oder verschieden sind und Wasserstoff, Niederalkyl, Halogen oder Trifluormethyl bedeuten,

$R^3$ Niederalkyl, Cycloalkyl, Cycloalkyl-alkyl oder gegebenenfalls substituiertes Aryl oder Aralkyl bedeutet,

$R^{13}$ Wasserstoff oder Methyl bedeutet,

p für eine ganze Zahl von 0 bis 20 steht,

A für eine Siloxankette steht, die aus m Strukturelementen der Formel

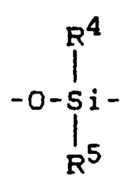

und/oder

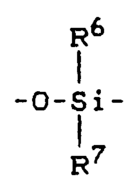

und der Endgruppe

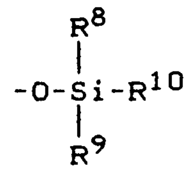

besteht
wobei

$R^4$, $R^5$, $R^6$, $R^7$ und $R^9$ gleich oder verschieden sind und Niederalkyl, Cycloalkyl ($C_5$ bis $C_7$), Cycloalkyl-alkyl ($C_6$ bis $C_{10}$) oder gegebenenfalls substituiertes Aryl ($C_6$ bis $C_{11}$) oder Aralkyl ($C_7$ bis $C_{11}$) bedeuten,

$R^8$ für die Gruppe

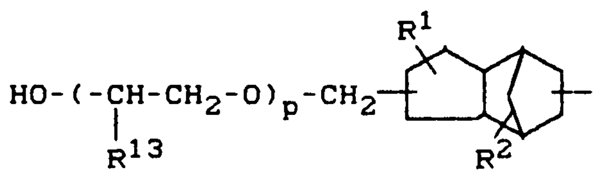

in der

$R^1$, $R^2$, $R^{13}$ und p die obengenannte Bedeutung haben, steht,

$R^{10}$ für Niederalkyl steht und

wobei die Summe der Stukturelemente m unabhängig voneinander für eine Zahl von 0 bis 600 steht, und

B für Niederalkyl steht,

ist dadurch gekennzeichnet, daß man in einer ersten Stufe ein $\alpha,\omega$-Bishydrogensiloxan der Formel

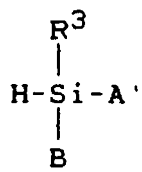
(XII),

in der

A' für eine Siloxankette steht, die aus m Stukturelementen der Formel

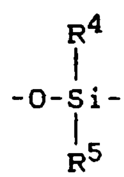
und/oder
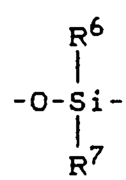

und der Endgruppe

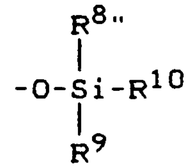

besteht

wobei

$R^4$, $R^5$, $R^6$, $R^7$, $R^9$ und $R^{10}$ die obengenannte Bedeutung haben und

$R^{8''}$ für Wasserstoff steht, wobei die Summe der Stukturelemente m unabhängig für eine Zahl von 0 bis 600 steht und

B und $R^3$ die obengenannte Bedeutung haben,

mit einem Tricyclo-[5.2.1.0$^{2.6}$]-decadien-Derivat der Formel

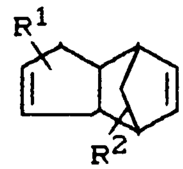

(XIII),

in der

$R^1$ und $R^2$ die obengenannte Bedeutung haben,

in Gegenwart von Edelmetall-Katalysatoren umsetzt, oder, daß man Bis-(tricyclo[5.2.1.0$^{2.6}$]decenyl)-disiloxane der Formel

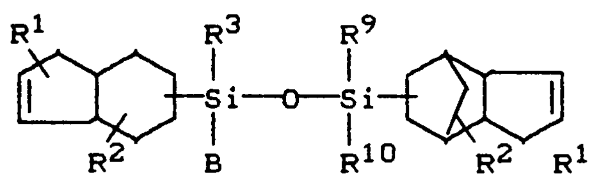

(XIV),

in der

$R^1$, $R^2$, $R^3$, $R^9$, $R^{10}$ und B die obengenannte Bedeutung haben,

in Gegenwart von Säuren oder Basen mit einem Cyclosiloxan der Formel

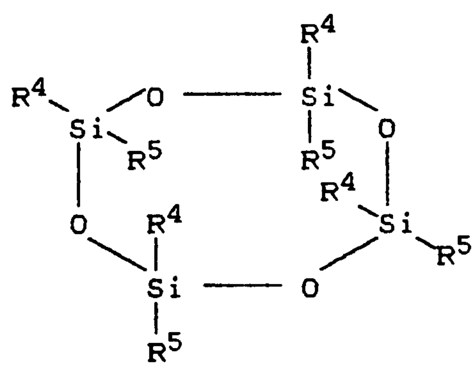

(XV),

in der

$R^4$ und $R^5$ die obengenannte Bedeutung haben,

und/oder mit einem Cyclo-siloxan der Formel

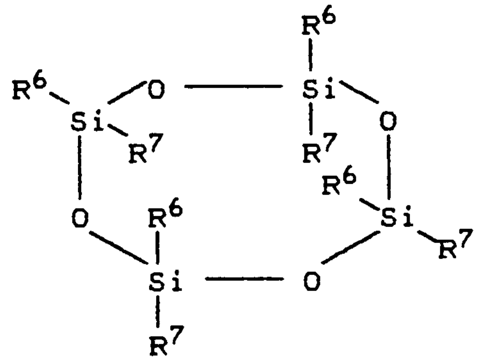

(XVI),

in der

$R^6$ und $R^7$ die obengenannte Bedeutung haben,

equilibriert,

und dann das nach den Varianten erhaltene $\alpha,\omega$-Bis-(tricyclo-[5.2.1.$0^{2.6}$]-decenyl)-siloxan in einer folgenden Stufe in Gegenwart eines Hydroformylierungskatalysators mit Kohlenmonoxid und Wasserstoff im Verhältnis von etwa 1:1 umsetzt,

das erhaltene Formyl-Derivat in einer weiteren Stufe zum entsprechenden Hydroxymethyl-Derivat reduziert und gegebenenfalls (für p>0) das erhaltene Hydroxymethyl-Derivat mit p Mol (bezogen auf 1 Mol Hydroxymethylgruppen) eines Alkylenoxids der Formel

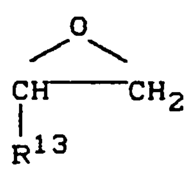

,

wobei

$R^{13}$ und p die obengenannte Bedeutung haben,

in Gegenwart eines basischen Katalysators umsetzt.

Das Herstellungsverfahren kann durch das folgende Reaktionsschema erläutert werden.

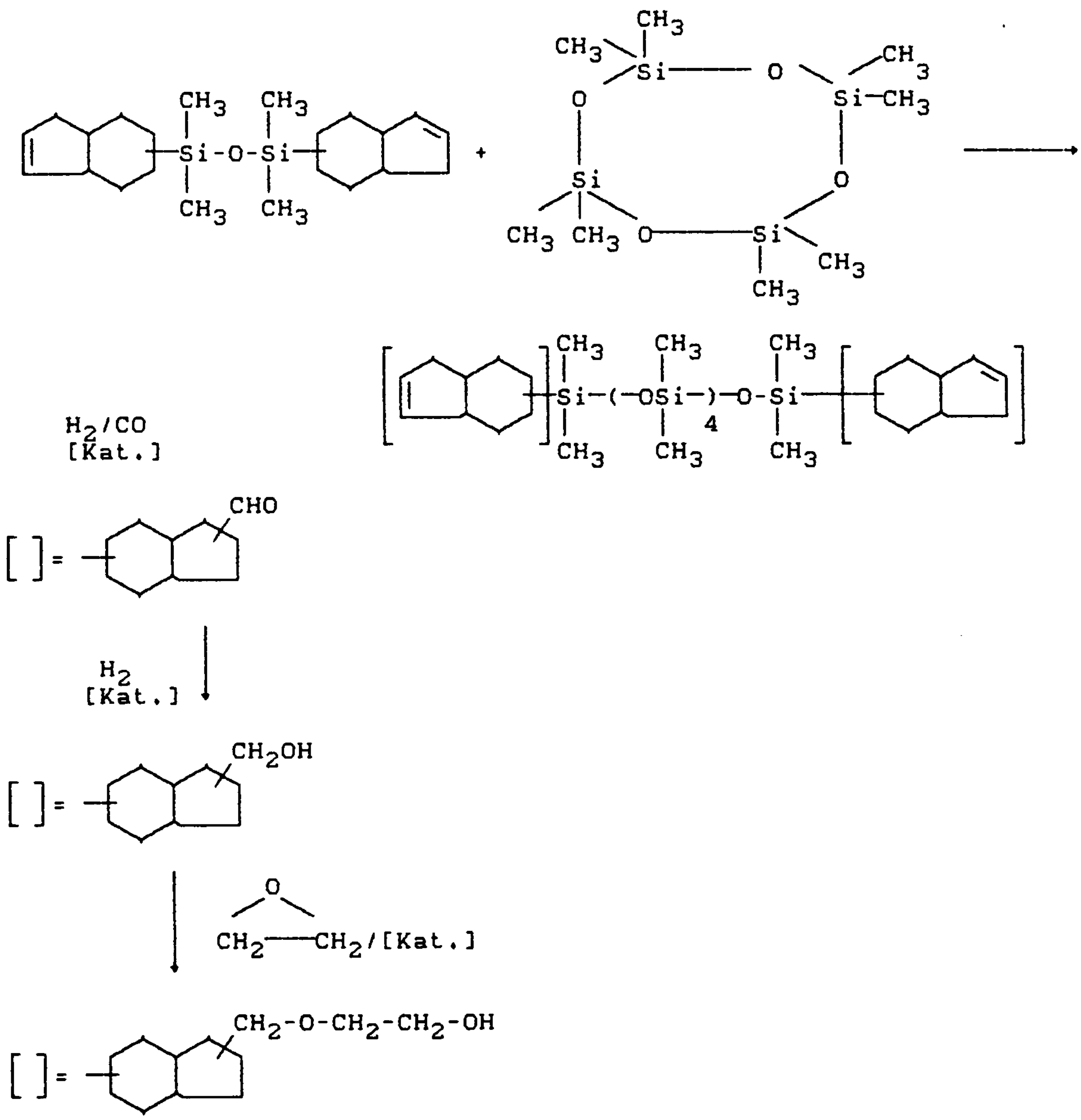


Die erste Stufe dieses Verfahrens kann in zwei Varianten durchgeführt werden:
Nach der ersten Variante setzt man α,ω-Bishydrogensiloxane (XII) mit einem Tricyclo-[5.2.1.0$^{2.6}$]-decadien-Derivat (XIII) um.

α,ω-Bishydrogensiloxane (XII) sind handelsüblich und können beispielsweise durch Equilibrierung von Dihydrogentetraorganodisiloxanen mit Cyclotrisiloxanen hergestellt werden.

Beispielsweise seien die folgenden α,ω-Bishydrogensiloxane (XII) genannt:

$H\text{-}Si(CH_3)_2\text{-}O\text{-}Si(CH_3)_2\text{-}H$,
$H\text{-}Si(CH_3)_2\text{-}OSi(CH_3)_2\text{-}OSi(CH_3)_2\text{-}H$
$H\text{-}(CH_3)_2Si[\text{-}O\text{-}Si(CH_3)_2]_{10}\text{-}OSi(CH_3)_2\text{-}H$
$H\ Si(CH_3)_2[\text{-}OSi(CH_3)_2]_{150}\text{-}O\text{-}Si(CH_3)_2H$

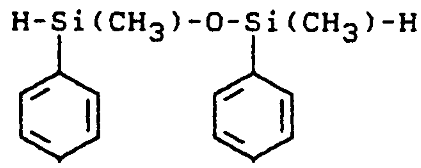

Tricyclo-[5.2.1.0$^{2.6}$]-decadien-Derivate (XIII) sind handelsüblich und können beispielsweise durch Diels-Alder-Reaktion der entsprechenden Cyclopentadien-Derivate hergestellt werden (Houben-Weyl, Methoden der organischen Chemie, Band V/1b, S. 438-447 (1972)).

Beispielsweise seien die folgenden Tricyclo-[5.2.1.0$^{2.6}$] -decadien-Derivate genannt:

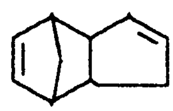

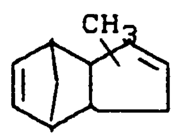

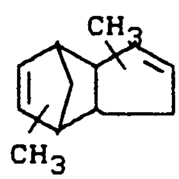

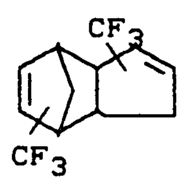

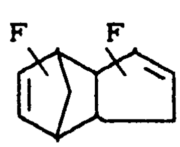

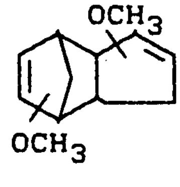

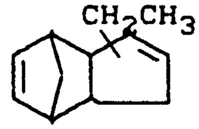

Die Umsetzung der $\alpha,\omega$-Bishydrogensiloxane mit den Tricyclo-[5.2.1.0$^{2.6}$]-decadien-Derivaten

(Hydrosilylierung) wird in Gegenwart von Hydrosilylierungskatalysatoren durchgeführt. Bevorzugt werden Platinverbindungen verwendet, wie beispielsweise Hexachloroplatinsäure in Isopropanol, der Lamoreaux-Katalysator (US 3 220 972) oder der Karstedt-Katalysator (US 3 775 452). Es ist auch möglich, die Hydrosilylierung mit einem Platin-Träger-Katalysator, beispielsweise mit Platin auf Aktivkohle, durchzuführen.

Der Hydrosilylierungskatalysator wird im allgemeinen in einer Menge von $10^{-7}$ bis $10^{-3}$ Mol Platin, vorzugsweise $10^{-6}$ bis $10^{-3}$ Mol Platin, bezogen auf ein Mol SiH-Gruppen des $\alpha,\omega$-Bishydrogensiloxans eingesetzt.

Die Hydrosilylierung wird im allgemeinen im Temperaturbereich von 20 bis 180 ° C, bevorzugt im Bereich von 50 bis 150 ° C durchgeführt.

Im allgemeinen wird die Hydrosilylierung bei Normaldruck durchgeführt. Es ist jedoch möglich die Umsetzung auch bei erhöhtem Druck (beispielsweise im Druckbereich von 1,5 bis 10 bar) durchzuführen.

Im allgemeinen führt man die Hydrosilylierung unter Feuchtigkeitsausschluß durch.

Es ist möglich, die Hydrosilylierung mit oder ohne Lösungsmittel durchzuführen.

Als Lösungsmittel seien solche genannt, die unter den Reaktionsbedingungen inert sind. Beispielsweise seien Toluol, Chlorbenzol, Xylol, Octahydronaphthalin und Ethylenglykoldimethylether genannt.

Im allgemeinen werden 1,0 bis 2,2 Mol des Tricyclo-[5.2.1.$0^{2,6}$]decadien-Derivats pro 1 Mol an SiH-Gruppen im $\alpha,\omega$-Bishydrogensiloxan eingesetzt.

Die Umsetzung bei der Hydrosilylierung kann IR-spektroskopisch verfolgt werden. Das Reaktionsende kann man beispielsweise dadurch feststellen, daß keine Absorption der SiH-Gruppe bei etwa 2100 $cm^{-1}$ mehr beobachtet wird.

Bei der Hydrosilylierung erhält man $\alpha,\omega$-Bis(tricyclo-[5.2.1.$0^{2,6}$]-decenyl)-siloxane. Vor der weiteren Umsetzung kann es zweckmäßig sein, das Reaktionsprodukt dieser Reaktionsstufe zu reinigen. Niedrigsiedende Siloxane, bevorzugt Disiloxane, mit Tricyclo[5.2.1.$0^{2,6}$]-decenyl-Substituenten können durch Vakuumdestillation gereinigt werden. Dabei kann es zweckmäßig sein, vor der Destillation einen Polymerisationsinhibitor in einer Menge von 100 bis 1000 ppm bezogen auf die Reaktanden hinzuzufügen. Als Polymerisationsinhibitoren seien beispielsweise 2,6-Di-tert.-butyl-4-methyl-phenol und Hydrochinonmonomethylether genannt. Den Polymerisationsinhibitor setzt man im allgemeinen in einer Menge von 0,01 bis 1 Gew.-% bezogen auf die Reaktanden zu. $\alpha,\omega$-Bis(tricyclo[5.2.1.$0^{2,6}$]decenyl)siloxane mit z.B. mehr als zwei Siloxandiylgruppen werden durch Adsorbentien, wie Aktivkohle, Aluminiumoxid oder Kieselgel, Celite usw. vom Katalysator und anschließend von flüchtigen Komponenten durch Vakuumbehandlung befreit.

Unter den im Rahmen des erfindungsgemäßen Verfahrens angegebenen Bedingungen der Hydrosilylierung erfolgt die Addition der SiH-Gruppe bevorzugt in der 8- oder 9-Stellung der 3(4),8-Tricyclo[5.2.1.$0^{2,6}$]-decadien-Derivate.

Nach der zweiten Variante setzt man Bis-(tricyclo-[5.2.1.$0^{2.6}$]-decenyl)-disiloxane (XIV) mit Cyclosiloxanen (XV) und/oder (XVI) um.

Bis-(tricyclo-[5.2.1.$0^{2.6}$]-decenyl)-disiloxane sind an sich bekannt (Chem. Abst. 85, 143 184) und können beispielsweise durch Umsetzung von Tricyclo[5.2.1.$0^{2,6}$]-decadien-Derivaten mit Dihydrogentetraorganodisiloxanen hergestellt werden.

Beispielsweise seien die folgenden Bis-(tricyclo[5.2.1.$0^{2.6}$]-decenyl)-disiloxane genannt:

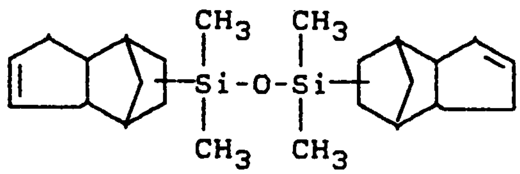

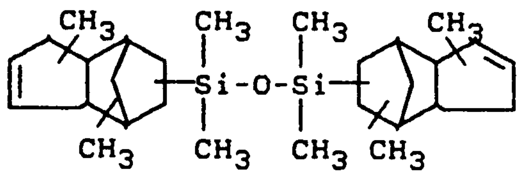

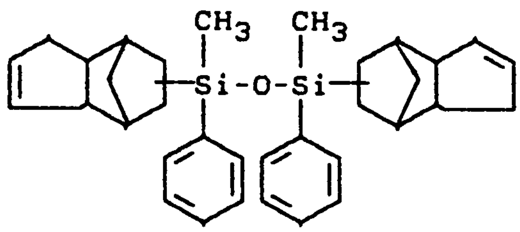

Cyclo-siloxane (XV) und (XVI) sind handelsüblich und können beispielsweise durch Kondensation von Diorganodihalogensilanen erhalten werden (Preparative Methods in Polymer Chemistry, Wiley (1969), S. 384 oder W. Noll, Chemie und Technologie der Silicone, Verlag Chemie (1968)).

Beispielsweise seien die folgenden Cyclo-siloxane genannt: Hexamethylcyclotrisiloxan, Octamethylcyclotetrasiloxan, Tetramethyltetraethylcyclotetrasiloxan und Tetramethyltetraphenylcyclotetrasiloxan.

Die Equilibrierung wird im allgemeinen in Gegenwart einer Säure oder Base durchgeführt. Als Säuren werden bevorzugt starke Säuren mit einem $p_k$-Wert kleiner 2 eingesetzt. Beispielsweise seien die folgenden Säuren genannt: Schwefelsäure, Trifluoressigsäure, Trifluormethansulfonsäure und Perfluorbutansulfonsäure.

Die Säure wird im allgemeinen in einer Menge von $10^{-3}$ bis 5 Mol.-%, bevorzugt $10^{-2}$ bis 1,0 Mol.-%, der Reaktanden eingesetzt.

Als Basen werden (Erd)Alkalihydroxide, Tetraalkylammoniumhydroxide, (Erd)Alkalioxide oder (Erd)-Alkalicarbonate in einer Menge von $10^{-3}$ bis 2 Mol.-% der Reaktanden eingesetzt.

Die Equilibrierung wird im allgemeinen im Temperaturbereich von 20 bis 120 ° C, bevorzugt von 20 bis 80 ° C, durchgeführt.

Im allgemeinen wird die Equilibrierung bei Normaldruck durchgeführt. Es ist aber auch möglich die Equilibrierung bei einem Über- oder Unterdruck (beispielsweise im Druckbereich von $10^{-2}$ bis 100 bar) durchzuführen.

Die Equilibrierung kann mit oder ohne Lösungsmittel durchgeführt werden. Lösungsmittel für diese Verfahrensstufe sind inerte Lösungsmittel, die sich unter den Reaktionsbedingungen nicht verändern. Beispielsweise seien Chloroform, Toluol, Chlorbenzol und Hexan genannt.

Der Reaktionsverlauf kann durch Messung der Viskosität verfolgt werden. Das Gleichgewicht ist erreicht, wenn keine Änderung der Viskosität mehr beobachtet wird.

Die Anzahl m der verschiedenen Strukturelemente im Polysiloxan kann durch das eingesetzte Molverhältnis der Cyclo-siloxane der Formel (XV) und/oder (XVI), bezogen auf das Bis(tricyclo[5.2.1.$0^{2,6}$]decenyl)-disiloxan (XIV), festgelegt werden.

Nach beendeter Umsetzung wird der Equilibrierungskatalysator neutralisiert und/oder extrahiert; flüchtige Bestandteile des Reaktionsgemisches können im Vakuum bis etwa 200 ° C abdestilliert werden.

Das nach beiden Varianten erhaltene $\alpha,\omega$-Bis-tricyclo[5.2.1.$0^{2.6}$]-decenyl-siloxan kann im allgemeinen nach den oben genannten Reinigungsverfahren in den weiteren Reaktionsstufen eingesetzt werden.

Die Hydroformylierung zu Formyl-Derivaten, deren Hydrierung zu den entsprechenden Hydroxymethyl-Derivaten und die Umsetzung mit Alkylenoxiden können in der oben beschriebenen Weise durchgeführt werden.

Ein weiteres Verfahren zur Herstellung von Poly[hydroxy(alkylenoxy)methyltricyclo-[5.2.1.$0^{2,6}$]-decanyl]-siloxanen der Formel

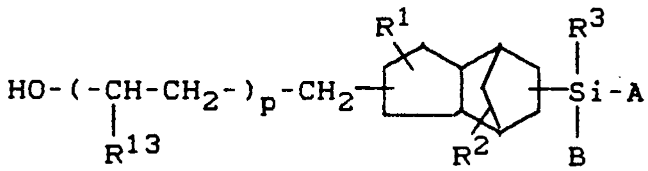

in der

$R^1$ und $R^2$ gleich oder verschieden sind und Wasserstoff, Niederalkyl, Halogen oder Trifluormethyl bedeuten,

$R^3$ Niederalkyl, Cycloalkyl, Cycloalkyl-alkyl oder gegebenenfalls substituiertes Aryl oder Aralkyl bedeutet,

$R^{13}$ Wasserstoff oder Methyl bedeutet,

p für eine ganze Zahl von 0 bis 20 steht,

A für eine Siloxankette steht, die aus m Strukturelementen der Formel

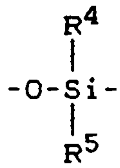

und/oder

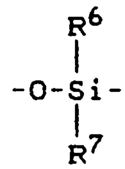

und der Endgruppe

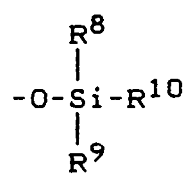

besteht

wobei

$R^4$, $R^5$, $R^6$, $R^7$, $R^8$ und $R^9$ gleich oder verschieden sind oder Niederalkyl, Cycloalkyl, Cycloalkyl-alkyl und gegebenenfalls substituiertes Aryl oder Aralkyl bedeuten,

wobei die Reste $R^5$ und/oder $R^7$ auch für die Gruppe

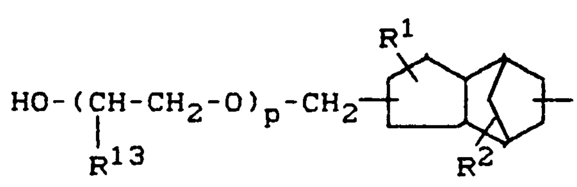

in der

$R^1$, $R^2$, $R^{13}$ und p die obengenannte Bedeutung haben, stehen,

$R^{10}$ für Niederalkyl steht, und,

wobei die Summe der Strukturelemente m unabhängig voneinander für eine Zahl von 0 bis 600 steht, und

B den Bedeutungsumfang wie A haben kann,

wobei die Reste $R^4$ bis $R^{10}$ in den Ketten A und B unterschiedlich sein können,

ist dadurch gekennzeichnet, daß man in einer ersten Stufe ein Polyhydrogensiloxan der Formel

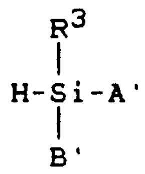

(XVII)

in der

A' für eine Siloxankette steht, die aus m Strukturelementen der Formel

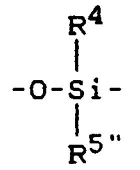

und/oder

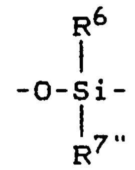

und der Endgruppe

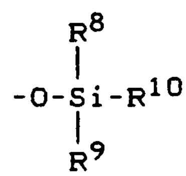

besteht,

wobei

$R^{5''}$ und $R^{7''}$ gleich oder verschieden sind und Wasserstoff, Niederalkyl, Cycloalkyl, Cycloalkyl-alkyl oder gegebenenfalls substituiertes Aryl oder Aralkyl bedeuten, wobei mindestens einer der Reste für Wasserstoff steht,

m, $R^4$, $R^6$, $R^8$, $R^9$ und $R^{10}$ die obengenannte Bedeutung haben und

B' den Bedeutungsumfang von A' haben kann,

wobei die Reste $R^4$, $R^{5''}$, $R^6$, $R^{7''}$, $R^8$, $R^9$ und $R^{10}$ in den Ketten A' und B' unterschiedlich sein können,

mit einem Tricyclo-[5.2.1.0$^{2.6}$]-decadien-Derivat der Formel

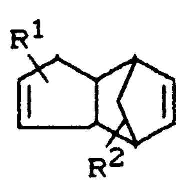

(XVIII),

in der

$R^1$ und $R^2$ die obengenannte Bedeutung haben,

in Gegenwart von Edelmetall-Katalysatoren umsetzt,

und dann das erhaltene Poly(tricyclo-[5.2.1.0$^{2.6}$]-decenyl)-siloxan in Gegenwart eines Hydroformylierungskatalysators mit Kohlenmonoxid und Wasser im Verhältnis von etwa 1:1 umsetzt,

das erhaltene Formyl-Derivat in einer weiteren Stufe zum Hydroxymethyl-Derivat reduziert,

und gegebenenfalls (für p >0) das erhaltene Hydroxymethyl-Derivat mit p Mol (bezogen auf 1 Mol Hydroxymethylgruppen) eines Alkylenoxids der Formel

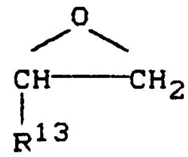,

wobei $R^{13}$ und p die obengenannte Bedeutung haben,
in Gegenwart eines basischen Katalysators umsetzt.

Das Herstellungsverfahren kann durch das folgende Reaktionsschema erläutert werden:

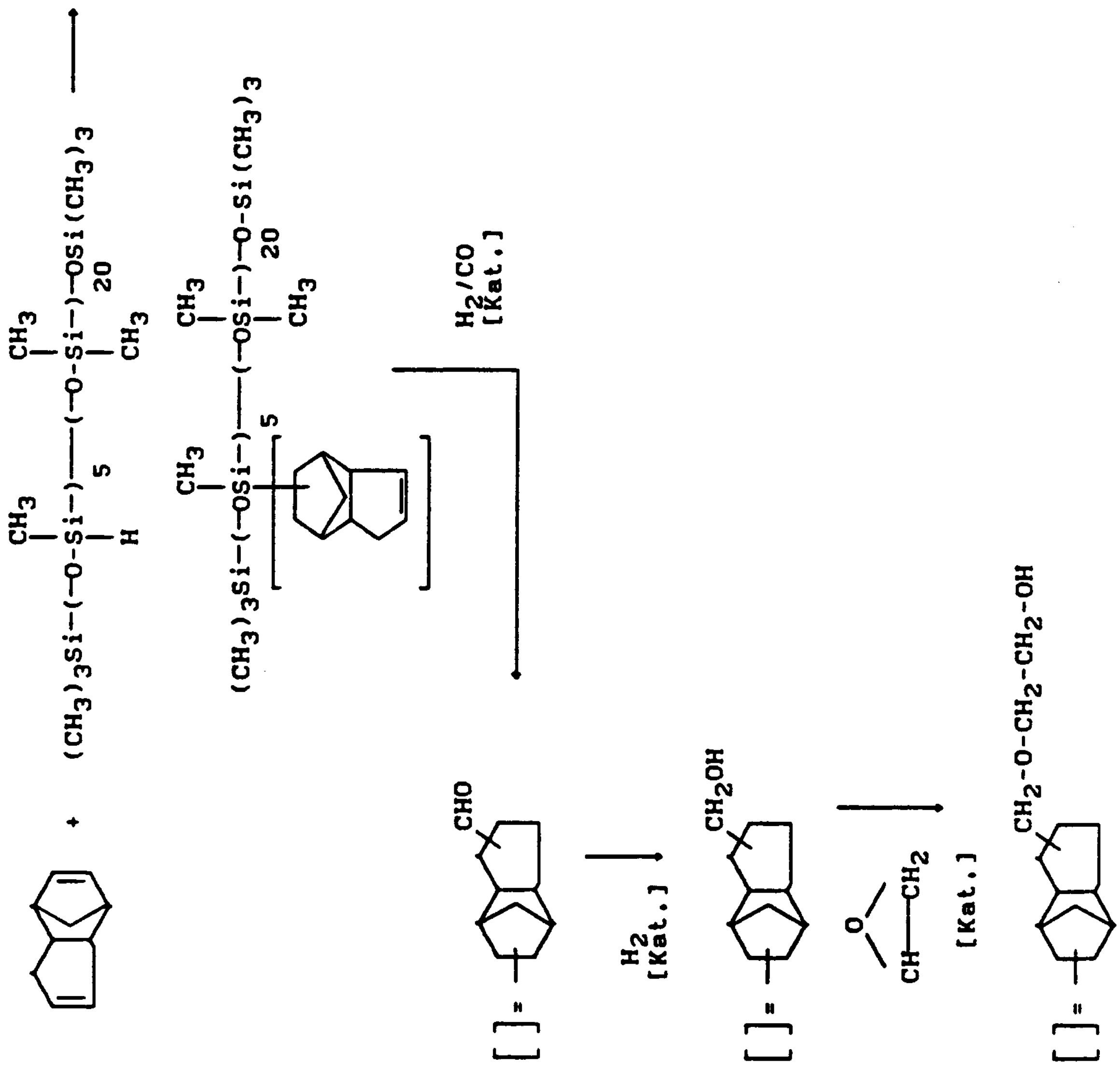


Poly-hydrogensiloxane (XVII) sind handelsüblich und können beispielsweise durch Equilibrierung von Hexaorganodisiloxanen mit Tetrahydrogen-tetraorgano-cyclosiloxanen und gegebenenfalls Octaorganocyclotetrasiloxanen hergestellt werden. Beispielsweise seien die folgenden Poly-hydrogensiloxane genannt:

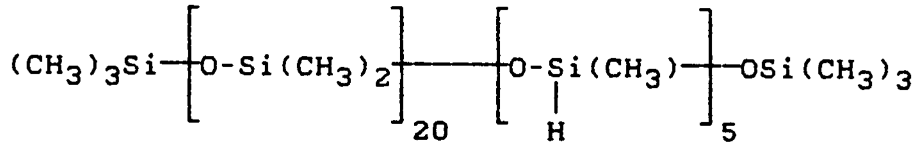

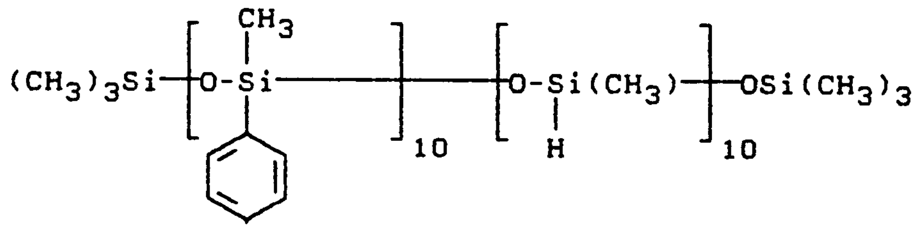

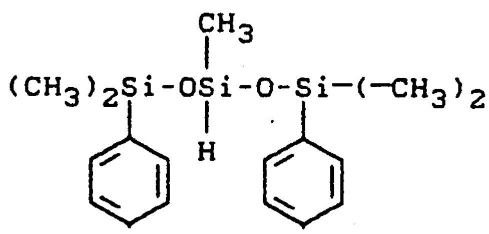

Die Hydrosilylierung und alle nachfolgenden Reaktionsstufen zu den erfindungsgemäßen Derivaten können in Analogie zum Verfahren, das α,ω-Bis[hydroxy(alkylenoxy)methyltricyclo[5.2.1.$0^{2,6}$]decenyl]-siloxane ergibt, durchgeführt werden.

Beispielsweise seien die folgenden Poly[hydroxy(alkylenoxy)methyltricyclo-[5.2.1.$0^{2,6}$]-decanyl]-siloxan-Derivate genannt:

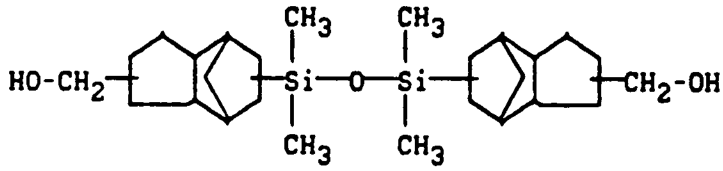

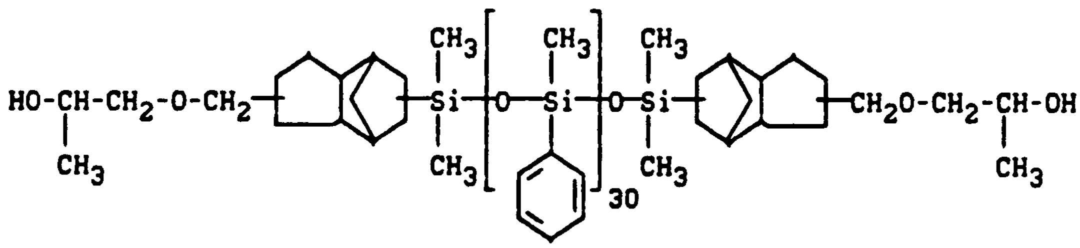

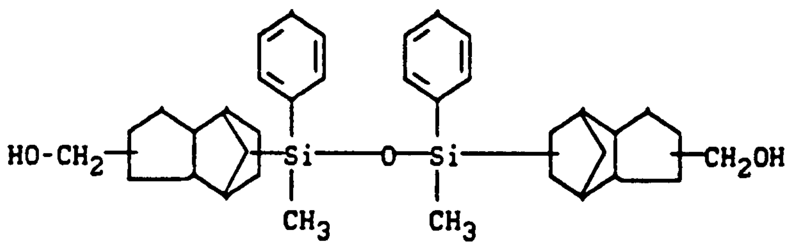

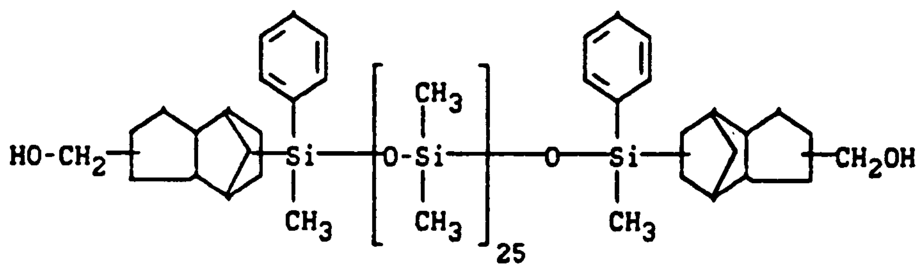

Die erfindungsgemäßen (Meth)Acrylsäureester (I) werden aus den Hydroxylverbindungen der Formel (II) durch Veresterung mit (Meth)acrylsäure oder seinen reaktiven Derivaten (III) oder durch Umsetzung mit Isocyanatoalkyl(meth)-acrylaten (IV) oder durch Umsetzung mit 1:1-Additionsprodukten aus Diisocyanaten (V) und Hydroxyalkyl(meth)acrylsäureestern (VI) erhalten.

Zur Veresterung können (Meth)-Acrylsäure, (Meth)acrylsäurechlorid, (Meth)-Acrylsäureanhydrid oder beispielsweise Ester wie (Meth)Acrylsäuremethylester oder -ethylester eingesetzt werden. Die Veresterung erfolgt vorzugsweise mit (Meth)Acrylsäure in Anwesenheit eines sauren Katalysators, z.B. p-Toluolsulfonsäure, Schwefelsäure oder Ionenaustauschern in der $H^{\oplus}$-Form in einem Lösungsmittel, das mit Wasser nicht mischbar ist, z.B. Toluol, Chloroform, Xylol, usw.

Die Veresterung kann beispielsweise wie folgt durchgeführt werden:

Die Hydroxylverbindung (II) und ein Überschuß von (Meth)Acrylsäure werden im Lösungsmittel gelöst und mit dem sauren Katalysator sowie einem Polymerisations-Inhibitor versetzt. Das während der Veresterung gebildete Wasser wird durch azeotrope Destillation aus dem Gleichgewicht entfernt. Die Reaktion wird im allgemeinen im Temperaturbereich von 50 ° C bis etwa 120 ° C durchgeführt.

Geeignete Polymerisations-Inhibitoren sind beispielsweise 2,6-Di-tert.-Butyl-4-methyl-phenol, Methylenblau und Hydrochinon in einer Menge von 0,01 bis 1 Gew.-%.

Nach beendeter Veresterung wird nicht umgesetzte (Meth)-Acrylsäure durch Extraktion mit einer basischen wäßrigen Lösung entfernt. Der Inhibitor wird beispielsweise durch Zusatz von Adsorbentien abgetrennt. Die erfindungsgemäßen Reaktionsprodukte werden durch Abdestillieren der Lösungsmittel isoliert.

Es ist auch möglich, die Veresterung der Hydroxylverbindung (II) mit (Meth)-Acrylsäurechlorid in Gegenwart von tertiären Aminen, wie Pyridin, Triethylamin, Dicyclohexylmethylamin, p-Dimethylaminopyridin, Tri-n-butylamin, N-Methylpiperidin, Cyclohexyldimethylamin usw. in inerten Lösungsmittel durchzuführen.

Bevorzugt werden 1,0 bis 1,5 Mol (Meth)-Acrylsäurechlorid, bezogen auf jedes Mol Hydroxylgruppen, eingesetzt. Das tertiäre Amin wird in der den OH-Gruppen von (II) äquivalenten Menge zugegeben. Die Reaktion wird vorzugsweise in Gegenwart von 0,01 bis 1 Gew.-% eines der obengenannten Polymerisationsinhibitors unter wasserfreien Bedingungen durchgeführt. Als Lösungsmittel sind solche geeignet, die mit dem (Meth)Acrylsäurechlorid nicht reagieren. Beispielsweise seien genannt: Dichlormethan, Chloroform, Toluol, n-Hexan, Methylcyclohexan, Xylol, Aceton usw.

Die Umsetzung wird im allgemeinen im Temperaturbereich von 0 bis 80 ° C, bevorzugt von 10 bis 50 ° C durchgeführt.

Nach beendeter Reaktion wird das ausgefallene Hydrochlorid des tertiären Amins abfiltriert und das Filtrat mit wäßrigen Mineralsäuren, mit wäßriger Alkalihydrogencarbonat oder -Carbonat-Lösung und Wasser gewaschen. Die erfindungsgemäßen Verbindungen werden beispielsweise nach Reinigung der getrockneten Lösung durch Aktivkohle, Celite, Bleicherde oder andere Adsorbentien, durch Abdestillieren der Lösungsmittel im Vakuum isoliert.

Weiterhin ist es möglich, die Veresterung von (II) mit (Meth)acrylsäureestern in Gegenwart von Umesterungskatalysatoren wie Tetrabutoxytitan, Tetra(isopropoxy)titan usw. durchzuführen, wobei der dem (Meth)Acrylsäureester zugrundeliegende Alkohol aus dem Gleichgewicht durch Destillation entfernt wird.

Für die Umsetzung der Hydroxylverbindungen (II) zum Urethan werden Isocyanate (IV) (Meth)-acrylsäure-isocyanatoalkylester) eingesetzt.

Isocyanate (IV) sind handelsüblich oder können beispielsweise durch Umsetzung von 5,6-Dihydrooxazinen mit Phosgen (DE-OS 3 338 077) oder durch Phosgenierung der entsprechenden Aminoverbindungen (US-P 2 821 544) hergestellt werden.

Beispielsweise seien die folgenden Isocyanate genannt: 2-Isocyanatoethylmethacrylat, 3-Isocyanato-1,2-dimethylpropyl-methacrylat, 3-Isocyanatopropylmethacrylat, 3-Isocyanatopropylacrylat.

Das erfindungsgemäße Verfahren wird im allgemeinen unter Wasserausschluß in einem inerten Lösungsmittel durchgeführt. Beispielsweise seien Chloroform, Tetrahydrofuran, Aceton, Dioxan, Dichlormethan, Toluol und Acetonitril genannt. Bevorzugte Lösungsmittel sind Chloroform,Toluol und Dichlormethan.

Das erfindungsgemäße Verfahren wird im allgemeinen im Tempraturbereich von 20 bis 100 ° C, vorzugsweise von 30 bis 70 ° C, durchgeführt.

Das erfindungsgemäße Verfahren wird im allgemeinen bei Normaldruck durchgeführt. Es ist jedoch auch möglich, das Verfahren im Druckbereich von 1 bis 15 bar durchzuführen.

Bei der erfindungsgemäßen Umsetzung zum Urethan wird vorzugsweise unter Wasserausschluß gearbeitet (vorzugsweise unter 0,1 % Wasser).

Zur Beschleunigung der Umsetzung werden vorzugsweise zinnhaltige Katalysatoren wie Dibutylzinndilaurat, Zinn(II)-octoat oder Dibutylzinndimethoxid verwendet.

Es ist auch möglich als Katalysatoren Verbindungen mit tertiären Aminogruppen oder Titanverbindungen einzusetzen. Beispielsweise seien die folgenden Katalysatoren genannt: Diazabicyclo[2.2.2]octan, Triethylamin, N-Methylpiperidin, Tetrabutoxy-titan (Ullmann, Encyclopädie der technischen Chemie, Bd. 19, S. 306 (1981)).

Im allgemeinen wird der Katalysator in einer Menge von 0,01 bis 2,5 Gew.-%, bevorzugt von 0,1 bis 1,5 Gew.-%, bezogen auf die Gesamtmenge der Reaktanten eingesetzt.

Die Umsetzung zu Urethan wird im allgemeinen in Gegenwart von 0,01 bis 0,2 Gew.-% eines Polymerisationsinhibitors, beispielsweise 2,6-Di-tert.-butyl-4-methylphenol durchgeführt.

Das erfindungsgemäße Verfahren kann beispielsweise wie folgt durchgeführt werden:
Die Reaktanden werden in dem Lösungsmittel gelöst und unter Rühren mit dem Katalysator versetzt. Der zeitliche Verlauf der Umsetzung kann beispielsweise durch Messung der IR-Spektren verfolgt werden. Nach vollständiger Umsetzung der Isocyanatgruppen werden die Reaktionsprodukte durch Entfernen des Lösungsmittels isoliert. Eine vorherige Reinigung mit Hilfe von Absorbentien, beispielsweise Aktivkohle, Bleicherde, Kieselgel oder Aluminiumoxid ist selbstverständlich auch möglich.

Die Umsetzung mit Acrylsäure-Derivaten kann in analoger Weise erfolgen.

Für die Umsetzung der Hydroxylverbindungen (II) zum Urethanmethacrylat können auch Additionsprodukte aus 1 Mol eines Diisocyanats (V) und 1 Mol eines Hydroxyalkyl-(Meth)acrylsäureesters(VI) eingesetzt werden.

Diisocyanate der Formel (V) sind an sich bekannt und können beispielsweise durch Umsetzung der Diamine mit Phosgen hergestellt werden. Beispielsweise seien die folgenden Diisocyanate genannt: Hexamethylendiisocyanat, Trimethyl-hexamethylendiisocyanat, Isophorondiisocyanat, 1,4-Diisocyanato-cyclohexan, 1-Isocyanato-4-methyl-4-isocyanato-cyclohexan, 3(4),8-Bis-(isocyantomethyl)-tricyclo[5.2.1.0$^{2,6}$]decan,

2,4-Diisocyanatotoluol, 1,5-Diisocyanatonaphthalin.

Hydroxyalkyl(meth)acrylsäureester der Formel (VI) sind an sich bekannt und können beispielsweise durch partielle Veresterung der entsprechenden Polyole erhalten werden. Beispielsweise seien folgende Hydroxyalkyl(meth)acrylsäureester genannt: 2-Hydroxyethylacrylat, 2-Hydroxypropyl-methacrylat, Glycerindimethacrylat, 1-Methacryloyloxy-3-acryloyloxy-propanol-2, Trimethylolpropandimethacrylat, Dipentaerythritpentaacrylat.

Die Herstellung der Additionsverbindungen aus 1 Mol eines Diisocyanats (V) und 1 Mol eines Hydroxyalkyl(meth)acrylsäureesters (VI) erfolgt in an sich bekannter Weise durch Umsetzung der beiden Reaktanden im Molverhältnis 1:1 bis 10:1 und bei Verwendung von überschüssigem Diisocyanat durch anschließende Reinigung des entstandenen Isocyanatourethans.

Die Reinigung des Adduktes von (V) und (VI) erfolgt bevorzugt durch Extraktion mit aliphatischen Lösungsmitteln mit Siedepunkten unter 120 ° C bei Normaldruck, z.B. mit Pentan, n-Hexan, Isopentan.

Die Herstellung der Additionsverbindungen wird im allgemeinen in einem inerten Lösungsmittel durchgeführt. Beispielsweise seien Aceton, Chloroform, Tetrahydrofuran, Dioxan, Methylenchlorid, Toluol, Acetonitril genannt. Besonders bevorzugt sind Chloroform, Toluol, Acetonitril und Aceton.

Das erfindungsgemäße Verfahren kann beispielsweise wie folgt durchgeführt werden:
Der (Meth)-acrylsäureester der Formel (VI) und gegebenenfalls der Polymerisationsinhibitor werden im inerten Lösungsmittel gelöst und unter Rühren zu dem gegebenenfalls gelösten Diisocyanat (V) zugetropft. Der Katalysator wird dabei einem der beiden Reaktanden zugesetzt. Die Reaktanden werden im Molverhältnis V:VI von etwa 1:1 bis 10:1 zur Umsetzung gebracht und bis zum vollständigen Umsatz der OH-Gruppen bzw. zum entsprechenden Umsatz der Isocyanatgruppen geführt. Die Umsetzung der Isocyanatgruppen kann in bekannter Weise durch IR-Spektroskopie und/oder durch Titration kontrolliert werden.

Ein Überschuß Diisocyanat kann anschließend mit n-Hexan, n-Pentan oder anderen aliphatischen Lösungsmitteln mit einem Siedepunkt unter 120 ° C (bei Normaldruck) extrahiert werden.

In der zweiten Stufe des erfindungsgemäßen Verfahrens wird das in der ersten Stufe erhaltene Isocyanatourethan gegebenenfalls nach Extraktion eventuell vorhandenen überschüssigen Diisocyanats, mit der Hydroxylverbindung (II) umgesetzt, so daß die Anzahl der Hydroxyläquivalente der Anzahl der noch vorhandenen NCO-Äquivalente entspricht.

Die Reaktion wird im allgemeinen bis zum vollständigen Umsatz geführt, so daß weder freies Isocyanat noch freies II im Reaktionsgemisch verbleiben. Nach beendeter Umsetzung wird das Reaktionsprodukt durch Entfernen des Lösungsmittels isoliert. Eine vorherige Filtration oder Reinigung mit Hilfe von Adsorbentien, z.B. Aktivkohle, Bleicherde, Kieselgel oder Aluminiumoxid ist möglich.

Nach dem erfindungsgemäßen Verfahren entsteht in der Regel ein Gemisch von Urethangruppen enthaltenden (Meth)-acrylsäurenderivaten, die an Adsorbentien aufgetrennt werden können.

Es ist auch möglich, die erste und die zweite Stufe des obengenannten Verfahrens in ihrer Reihenfolge zu vertauschen. In diesem Fall werden in der ersten Stufe Diisocyanat V und Hydroxyverbindung II im Molverhältnis NCO:OH = 2 bis 4, vorzugsweise im Molverhältnis NCO:OH = 2,0 bis 2,3, bis zur Umsetzung aller Hydroxylgruppen in Urethangruppen zur Reaktion gebracht.

Anschließend wird ein eventuell vorhandener Überschuß an Diisocyanat (sofern dieses im Überschuß eingesetzt wurde) in der oben beschriebenen Weise mit den genannten Lösungsmitteln extrahiert. Die verbleibenden NCO-Gruppen werden dann in der zweiten Stufe mit einem Hydroxyalkyl(meth)acrylat VI zum erfindungsgemäßen (Meth)acrylsäureester I umgesetzt, wobei eine stöchiometrische Äquivalenz von NCO- und OH-Gruppen besteht.

Mit Hilfe von Diisocyanaten mit unterschiedlicher Reaktivität der NCO-Gruppe ist es jedoch durchaus möglich, die erfindungsgemäßen Urethangruppen enthaltenden (Meth)-acrylsäurederivate der Formel I selektiv herzustellen. Hierfür geeignete Diisocyanate sind vor allem solche, die neben einer sterisch ungehinderten, aliphatisch gebundenen, eine sterisch gehinderte, cycloaliphatisch gebundene Isocyanatgruppe aufweisen. Beispielsweise seien 1-Isocyanato-1-methyl-4-isocyanatomethylcyclohexan, bevorzugt Isophorondiisocyanat usw. genannt.

Bei Einsatz dieser Diisocyanate ergeben sich naturgemäß unterschiedliche Reaktionsgeschwindigkeiten für die erste und zweite Synthesestufe, so daß in der ersten Stufe eine Stöchiometrie von NCO:OH = 2,0:1 bis 2,05:1 bevorzugt ist.

Für den erfindungsgemäßen Einsatz der neuen Urethan(meth)acrylate auf dem Dentalgebiet ist eine Auftrennung der erhaltenen Reaktionsgemische nicht erforderlich.

Die erfindungsgemäßen (Meth)-acrylsäureester von Tricyclodecangruppen enthaltenden Siloxanen können als Monomere zur Herstellung von polymeren Werkstoffen verwendet werden. Die Polymerisation kann in an sich bekannter Weise durch radikalische Initiierung durchgeführt werden und ergibt Polymerisate, die eine hohe Vernetzungsdichte aufweisen.

Die erfindungsgemäßen (Meth)-acrylsäureester von Tricyclodecangruppen enthaltenden Siloxanen können insbesondere als Monomere für Dentalmaterialien verwendet werden, aus denen durch die Polymerisation Dentalformkörper hergestellt werden. Als Dentalmaterialien seien beispielsweise Füllungsmaterialien für Zähne, Beschichtungsmittel für Zähne und Komponenten für die Herstellung von Zahnersatz genannt. Je nach Anwendungsgebiet können Dentalmaterialien weitere Hilfsstoffe enthalten.

Die erfindungsgemäßen Dentalmaterialien enthalten im allgemeinen als polymerisierbare Verbindungen (Monomere) 30 bis 100 Gew.-%, bevorzugt 60 bis 100 Gew.-%, an (Meth)-acrylsäureestern von Tricyclodecangruppen enthaltenden Siloxanen.

Für die Anwendung als Monomere für Zahnfüllmassen oder Beschichtungsmittel (Zahnlacke) im Dentalbereich können die erfindungsgemäßen (Meth)-acrylsäureester von Tricyclodecangruppen enthaltenden Siloxanen mit an sich bekannten Comonomeren gemischt werden. So kann beispielsweise die Viskosität dem Verwendungszweck angepaßt werden. Diese Monomermischungen haben im allgemeinen eine Viskosität im Bereich von 60 bis 10.000 mPas.

Beispielsweise seien die folgenden Comonomere genannt:

Triethylenglykoldimethacrylat, Tetraethylenglykoldimethacrylat, 1,12-Dodecandioldimethacrylat, 1,6-Hexandioldimethacrylat, Diethylenglykoldimethacrylat, 2,2-Bis(p-(2′-hydroxy-3′-methacryloyloxypropoxy)phenyl)-propan, 2,2-Bis(p-(2′-methacryloyloxyethoxy)phenyl)propan, Trimethylolpropan-tri(meth)-acrylat, Bis-(meth)-acryloyloxyethoxymethyl)-tricyclo[5.2.1.$0^{2.6}$]decan (gemäß DE-OS 2 931 925 und 2 931 926), 1,3-Di((meth)-acryloyloxypropyl)-1,1,3,3-tetra-methyldisiloxan, 1,3-Bis(3-(meth)acryloyloxyethylcarbamoyloxy-propyl)-1,1,3,3-tetramethyl-disiloxan. Insbesondere werden Comonomere bevorzugt, die bei 13 mbar einen Siedepunkt über 100° C besitzen.

Im Rahmen der vorliegenden Erfindung ist es ebenfalls bevorzugt, Mischungen verschiedener erfindungsgemäßer (Meth)-acrylsäureester einzusetzen.

Es ist auch möglich, Monomermischungen einzusetzen, die mehrere Comonomere enthalten.

Die erfindungsgemäßen (Meth)-acrylsäureester lassen sich, gegebenenfalls in Mischung mit den genannten Monomeren, mit an sich bekannten Methoden zu vernetzten Polymerisaten aushärten (G.M. Brauer, H. Argentar, Am. Chem. Soc., Symp. Ser. 212, S. 359-371 (1983)). Für die sogenannte Redoxpolymerisation ist ein System aus einer peroxidischen Verbindung und einem Reduktionsmittel, beispielsweise auf Basis tertiärer aromatischer Amine, geeignet. Beispiele für Peroxide sind:

Dibenzoylperoxid, Dilauroylperoxid und Di-4-Chlorbenzoylperoxid.

Als tertiäre aromatische Amine seien beispielsweise N,N-Dimethyl-p-toluidin, Bis-(2-hydroxyethyl)-p-toluidin, Bis(2-hydroxyethyl)-3,5-dimethylanilin und das in der DE-PS 2 759 239 beschriebene N-Methyl-N-(2-methylcarbamoyloxypropyl)-3,5-dimethylanilin genannt.

Die Konzentration des Peroxids bzw. des Amins werden vorteilhaft so gewählt, daß sie 0,1 bis 5 Gew.-%, bevorzugt 0,5 bis 3 Gew.-%, bezogen auf die Monomermischung betragen. Die Peroxid- bzw. aminhaltige Monomermischungen werden bis zur Anwendung getrennt gelagert.

Die erfindungsgemäßen Monomeren können auch durch Bestrahlung mit UV-Licht oder sichtbarem Licht (beispielsweise im Wellenlängenbereich von 230 bis 650 nm) zur Polymerisation gebracht werden. Als Initiatoren für die fotoinitiierte Polymerisation eignen sich beispielsweise Benzil, Benzildimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls in Gegenwart von synergistisch wirkenden Fotoaktivatoren, wie N,N-Dimethylaminoethylmethacrylat, Triethanolamin, 4-N,N-Dimethylaminobenzolsulfonsäurebisallylamid.

Die Durchführung des Fotopolymerisationsverfahrens ist beispielsweise in der DE-A 3 135 115 beschrieben.

Neben den oben beschriebenen Initiatoren können den erfindungsgemäßen (Meth)-acrylsäureestern an sich für diesen Einsatzzweck bekannte Lichtschutzmittel und Polymerisations-Inhibitoren zugesetzt werden.

Das Lichtschutzmittel und der Polymerisations-Inhibitor werden jeweils im allgemeinen in einer Menge von 0,01 bis 0,50 Gew.-Teilen, bezogen auf 100 Gew.-Teile der Monomermischung eingesetzt. Die Monomermischungen können ohne Zusatz von Füllstoffen als Beschichtungsmittel (Zahnlacke) eingesetzt werden.

Bei der Verwendung als Zahnfüllmassen setzt man den erhaltenen Monomermischungen im allgemeinen Füllstoffe zu. Um einen hohen Füllgrad erreichen zu können, sind Monomermischungen, die eine Viskosität im Bereich von 60 bis 10.000 mPas besitzen, besonders vorteilhaft. Den die erfindungsgemäßen Verbindungen der Formel I enthaltenden Monomermischungen können vorzugsweise anorganische Füllstoffe zugemischt werden. Beispielsweise seien Bergkristall, Quarzit, Kristobalit, Quarzglas, hochdisperse Kieselsäure, Aluminiumoxid und Glaskeramiken, beispielsweise Lanthan und Zirkon enthaltende Glaskeramiken (DE-A 2 347 591) genannt.

Die anorganischen Füllstoffe werden zur Verbesserung des Verbundes zur Polymermatrix des Dentalformkörpers vorzugsweise mit einem Haftvermittler vorbehandelt. Die Haftvermittlung kann beispielsweise durch eine Behandlung mit Organosiliciumverbindungen erreicht werden (E.P. Plueddemann, Progress in Organic coatings, 11, 297 bis 308 (1983)). Bevorzugt wird 3-Methacryloyloxypropyltrimethoxysilan eingesetzt.

Die Füllstoffe für die erfindungsgemäßen Zahnfüllmassen weisen im allgemeinen einen mittleren Teilchendurchmesser von 0,01 bis 100 µm, vorzugsweise von 0,05 bis 50 µm auf, besonders bevorzugt 0,05 bis 5 µm. Es kann auch vorteilhaft sein, mehrere Füllstoffe nebeneinander einzusetzen, die einen voneinander verschiedenen Teilchendurchmesser und verschiedenen Silanisierungsgrad besitzen.

Der Füllstoffanteil in den Zahnfüllmassen beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen verarbeitet.

Der Anteil der erfindungsgemäßen (Meth)-acrylsäureester in den Füllmassen beträgt im allgemeinen 10 bis 90 Gew.-%, vorzugsweise 45 bis 85 Gew.-%, bezogen auf die Füllmasse.

Die erfindungsgemäßen (Meth)-acrylsäureester von Tricyclodecangruppen enthaltenden Siloxanen können auch als Komponenten bei der Herstellung von Zahnersatz eingesetzt werden.

Dabei werden die erfindungsgemäßen Monomeren mit den üblicherweise verwendeten, an sich bekannten Bestandteilen kombiniert. Vorzugsweise werden die Monomeren im Gemisch mit Alkylmethacrylaten, wie Methylmethacrylat eingesetzt. Es können auch zusätzlich an sich bekannte Perlpolymerisate zugesetzt werden. Zur Einstellung der Zahnfarbe können bekannte anorganische und organische Farbpigmente und Trübungsmittel zugesetzt werden. Auch die Anwendung von Stabilisatoren und Lichtschutzmitteln ist möglich.

Die Kunstoffzähne werden durch radikalische Polymerisation der Dentalmassen unter Formgebung hergestellt. Die Verarbeitung ist sowohl nach Injektionsverfahren als auch Prägeverfahren möglich und erfolgt im allgemeinen nach den üblichen Herstellungsmethoden für Zähne auf Basis von Poly-(methylmethacrylat), z.B. durch thermische Polymerisation unter Verwendung von an sich bekannten Polymerisationsinitiatoren, beispielsweise auf Basis von Peroxiden und Azoverbindungen, wie Dibenzoylperoxid, Dilauroylperoxid, Cyclohexylpercarbonat, Azoisobuttersäuredinitril. Gut geeignet sind auch Mischungen von Polymerisationsinitiatoren mit unterschiedlichen Zerfallstemperaturen.

Die unter Verwendung der erfindungsgemäßen (Meth)acrylsäureester von Tricyclo[5.2.1.$0^{2.6}$]-decanylsiloxan-Derivaten hergestellten Kunststoffe besitzen einen sehr niedrigen Polymerisationsschrumpf, eine geringe Wasseraufnahme, sehr gute mechanische Eigenschaften und eine gegenüber bekannten (Meth)-acrylsäureestern, die in Dentalmaterialien angewendet werden, deutlich verbesserte Abrasionsfestigkeit. Überraschend ist neben diesen Vorteilen die sehr niedrige Viskosität der reinen Monomeren.

## Beispiele

### Beispiel 1

1,3-Bis(tricyclo[5.2.1.$0^{2,6}$]dec-3-en-8(9)-yl)-1,1,3,3-tetramethyldisiloxan

#### Syntheseweg A

Einer Lösung von 924 g destilliertem Dicyclopentadien in 2 l Chlorbenzol werden $10^{-4}$ Mol Platin, in Form des Lamoreaux-Katalysators gemäß US 3 220 972 zugesetzt. In die auf 80 °C erwärmte Reaktionslösung werden unter intensivem Rühren 469 g 1,1,3,3-Tetramethyldisiloxan langsam eingetropft, wobei die Temperatur der Mischung 100 °C nicht überschreiten sollte. Nach beendeter Zugabe wird die Abnahme der SiH-Bande IR-spektroskopisch verfolgt. Nach beendeter Reaktion (ca. 24 h bei 100 bis 120 °C) wird die Mischung abgekühlt und im Wasserstrahlvakuum vom Lösungsmittel befreit. Der Rückstand wird im Hochvakuum nach Zugabe von 500 ppm Hydrochinonmonomethylether fraktioniert. Neben einem geringen Vorlauf von nicht umgesetztem Dicyclopentadien und einer Zwischenfraktion, die als Hauptkomponente das Monoaddukt des Disiloxans enthält, werden 1091 g des Bisaddukts erhalten. Das Produkt ist eine farblose, niedrigviskose Flüssigkeit und stellt ein Gemisch der endo- und exo-Isomeren dar.
Ausbeute: 78 %.
Siedepunkt (0,07 mm Hg): 155 bis 160 °C.

#### Syntheseweg B

1) 68,9 g Dicyclopentadien und $5.10^{-5}$ Mol Platin in Form des Lamoreaux-Katalysator werden in 780 ml Chlorbenzol gelöst und auf 80 °C erwärmt. Bei dieser Temperatur werden 59,5 g Chlordimethylsilan zugetropft. Nach beendeter Zugabe wird 24 Stunden bei 120 °C gerührt und anschließend destilliert.

Es werden 38 g 8(9)-(Chlordimethylsilyl)-tricyclo[5.2.1.$0^{2,6}$]dec-3-en erhalten.

Siedepunkt (0,12 mm Hg): 80 bis 85 °C.

2) 8,9 g 8(9)-(Chlordimethylsilyl)-tricyclo[5.2.1.$0^{2,6}$]dec-3-en werden in 50 ml Toluol gelöst. Bei Raumtemperatur werden 1,2 g Methanol und 3,7 g Triethylamin gleichzeitig zugetropft. Man rührt 1 Stunde bei 40 °C nach und fraktioniert nach Entfernen des Lösungsmittels und Abtrennung des Triethylaminhydrochlorids den Rückstand im Vakuum.

Ausbeute: 8,5 g 8(9)-(Methoxydimethylsilyl)-tricyclo[5.2.1.$0^{2,6}$]dec-3-en

Siedepunkt (0,12 mm Hg): 88 bis 91 °C.

3) Hydrolyse

10 mMol 8(9)-(Methoxydimethylsilyl)-tricyclo[5.2.1.$0^{2,6}$]dec-3-en bzw. 10 mMol 8(9)-(Chlordimethylsilyl)-tricyclo[5.2.1.$0^{2,6}$]dec-3-en werden in 50 ml Ether gelöst und in 100 ml 0,01 n-Salzsäure oder in 100 ml gesättigte Natriumhydrogencarbonatlösung eingetropft. Man läßt bei Raumtemperatur 2 Stunden nachrühren und trennt die organische Phase ab. Die Disiloxan-Lösung wird neutral gewaschen, getrocknet und vom Lösungsmittel befreit. Das Produkt ist aufgrund der analytischen Daten identisch mit dem nach Syntheseweg A erhaltenen 1,3-Bis(tricyclo[5.2.1.$0^{2,6}$]dec-3-en-8(9)-yl)-1,1,3,3-tetramethyldisiloxan.

Beispiel 2

1,3-Bis[3(4)-(hydroxymethyl)tricyclo[5.2.1.$0^{2,6}$]decan-8(9)-yl]-1,1,3,3-tetramethyl-disiloxan.

244 g des Disiloxans aus Beispiel 1 werden in 700 ml Toluol gelöst. Es wird 1 g eines Hydroformylierungskatalysators (5 % Rh auf Aluminiumoxid) zugegeben. Die Suspension wird in einen Rührautoklaven gegeben und bei 140 °C und 180 bis 200 bar mit einem (1:1)-$H_2$/CO-Gemisch zur Reaktion gebracht. Nach 2,5 Stunden ist die Umsetzung beendet. Der Katalysator wird abfiltriert und das Filtrat gegebenenfalls mit Aktivkohle behandelt und filtriert. Das Lösungsmittel wird im Vakuum entfernt. Das $^1$H-NMR-Spektrum des Rückstandes zeigt keine olefinischen Protonen. Das Produkt enthält nach gaschromatographischer Analyse den Dialdehyd mit einem Anteil > 98 %.

Zur Reduktion werden 200 g des Rohproduktes in 500 ml Ethanol gelöst und mit 2 ml Triethylamin versetzt. Dann werden 10 g Raney-Nickel und anschließend 0,5 ml einer 0,1 %igen Lösung von Hexachloroplatinsäure in Isopropanol zugegeben. In einem Rührautoklaven wird bei 120 °C und 100 bar Wasserstoffdruck 3 Stunden hydriert. Die Hydrierung wird IR-spektroskopisch kontrolliert. Gegebenenfalls wird nachhydriert, bis die Carbonylbande im IR-Spektrum nicht mehr vorhanden ist.

Das Produkt wird nach Filtration über Aktivkohle und Entfernen des Lösungsmittels als zähflüssiger, farbloser Rückstand mit nahezu quantitativer Ausbeute erhalten.

Das Diol ist gut löslich in Toluol und polaren Lösungsmitteln wie Alkoholen, Aceton, chlorierten Kohlenwasserstoffen und Essigsäureethylester.

Siedepunkt (0,05 mm Hg): 220-230 °C

Molmasse (osmometrisch):

gef. 475

ber. 462

| Elementaranalyse | | | IR(Film auf KBr): | |
|---|---|---|---|---|
| | C(%) | H(%) | | |
| ber. | 67,53 | 9,96 | 3600-3200 $cm^{-1}$ | ν(O-H) |
| gef. | 67,8 | 10,0 | 1000-1100 $cm^{-1}$ | ν(C-O) und ν(Si-O-Si) |
| OH-Zahl: 238 mg KOH/g | | | | |
| | | | 1250 $cm^{-1}$ | ν(Si-$CH_3$) |

Beispiel 3

1,3-Bis[3(4)(methacryloyloxymethyl)-tricyclo[5.2.1.$0^{2,6}$]decan-8(9)-yl]-1,1,3,3-tetramethyl-disiloxan

492,5 g des Produkts aus Beispiel 2 werden in 1000 ml trockenem Dichlormethan gelöst. Nach Zugabe von 247,7 g destilliertem Triethylamin und 383 mg 2,6-Di-tert.-butyl-4-methyl-phenol werden bei 10 °C 257,5 g frisch destilliertes Methacrylsäurechlorid zugetropft. Nach beendeter Zugabe wird 6 Stunden bei Raumtemperatur nachgerührt. Anschließend wird filtriert. Das Filtrat wird mit gesättigter Natriumhydrogencarbonat-Lösung, mit 10 %iger Salzsäurelösung und anschließend bis zur neutralen Reaktion der wäßrigen Phase mit Wasser gewaschen. Die über Natriumsulfat getrocknete organische Phase wird mit Aktivkohle verrührt, filtriert und danach über Kieselgel 60 filtriert. Das Filtrat wird im Vakuum bis zur Gewichtskonstanz eingeengt. Man erhält 503 g des Dimethacrylats als niedrigviskose, farblose Flüssigkeit ($\eta$25 °C - 803 mPas).
Hydroxylzahl: < 5 mg KOH/g

Beispiel 4

Bis-Methacrylat des Kondensationsproduktes aus 1 Mol des Diols aus Beispiel 2 und 2 Mol Ethylenoxid

A) Herstellung des Kondensates

In einem heizbaren Rührautoklaven, der mit einer Vorrichtung zur Azeotrop-Entwässerung versehen ist, werden 462 g 1,3-Bishydroxymethyl-tricyclo[5.2.1.$0^{2.6}$]decanyl-1,1,3,3-tetramethyldisiloxan (Isomerengemisch) und 600 g Toluol vorgelegt und die Luft gegen Stickstoff ausgetauscht. Man gibt bei 80 °C 2,9 g 50 %ige wäßrige Kaliumhydroxidlösung zu. Anschließend werden bei 100 bis 115 °C und 0,4 bis 0,6 bar 88 g Ethylenoxid langsam zudosiert und das Gemisch 3 Stunden bei 100 bis 105 °C nachgerührt. Das Reaktionsprodukt wird mit 29 g Wasser und 10,2 g einer 12,5 %igen wäßrigen Schwefelsäure neutralisiert. Anschließend wird nach Zugabe eines Filterhilfsmittels und eines Antioxydans (0,05 % 2,6-Bis-t-butyl-p-kresol) das Wasser im Vakuum bei 70 bis 90 °C abdestilliert und die abgeschiedenen Salze zusammen mit dem Filterhilfsmittel abfiltriert.

Das neutrale Produkt wird durch Vakuumdestillation bis 150 °C Innentemperatur von etwa 1 g flüchtiger Bestandteile befreit.
Molmasse: 545
Hydroxylzahl: 208 mg KOH/g

Acrylsäureester von Produkt A:

200 g Produkt A werden in 500 ml Toluol gelöst. Man setzt 0,125 g Jonol und 75,7 g Triethylamin zu und tropft bei Raumtemperatur 67,9 g Acrylsäurechlorid ein. Nach 24 Stunden bei Raumtemperatur wird das Produkt analog Beispiel 3 isoliert.
Ausbeute: 191 g
Viskosität: (25 °C) ~ 300 mPas
Hydroxylzahl: < 2 mg KOH/g

Beispiel 5

1,3-Bis[(2-methacryloyloxyethyl)-1-carbamoyloxymethyltricyclo(5.2.1.$0^{2,6}$)decan-8(9)-yl]-1,1,3,3-tetramethyldisiloxan

21,1 g des Produkts aus Beispiel 2 werden in 100 ml über $P_4O_{10}$ destilliertem Chloroform gelöst. Nach Zugabe von 20 mg 2,6-Di-tert.-butyl-4-methylphenol und 0,1 g Zinnoctoat werden 14,16 g 2-Isocyanatoethylmethacrylat zugetropft. Man rührt bei 50 °C nach, bis die NCO-Gruppen (IR-spektroskopische Kontrolle) vollständig umgesetzt sind (etwa nach 15 Stunden). Nach Entfernen des Lösungsmittels erhält man quantitativ ein farbloses Öl.

| IR-Spektrum (Film auf KBr): | |
|---|---|
| 3200 bis 3500 $cm^{-1}$ | V(N-H) |
| 1710 bis 1720 $cm^{-1}$ | V(C=O), Ester + Amid I |
| 1640 $cm^{-1}$ | V(C=C) |
| 1510 $cm^{-1}$ | Amid II |
| 1265 $cm^{-1}$ | $Si-CH_3$ |
| 1050 $cm^{-1}$ | Si-O-Si |

Beispiel 6

1,3-Bis-[3(4)-(acryloyloxymethyl)-tricyclo[5.2.1.$0^{2,6}$]decan-8(9)-yl]-1,1,3,3-tetramethyldisiloxan

300 g des Produkts aus Beispiel 2 werden in 500 ml (über $P_4O_{10}$ destilliertem) Dichlormethan gelöst. Nach Zugabe von 222 mg 2,6-Di-tert.-butyl-4-methyl-phenol und 158 g frisch destilliertem Triethylamin werden bei 10°C 145 g Acrylsäurechlorid zugetropft. Nach beendeter Zugabe wird 6 Stunden bei Raumtemperatur gerührt. Die Aufarbeitung des Reaktionsansatzes geschieht analog zu Beispiel 3. Man erhält 317 g des Diacrylats. Der Ester ist farblos und besitzt eine dem Methacrylat aus Beispiel 3 vergleichbare Viskosität.

Elementaranalyse:

| % | C | H | Si | O |
|---|---|---|---|---|
| ber. | 67,4 | 8,8 | 9,8 | 14,0 |
| gef. | 67,8 | 8,9 | 9,6 | 13,7 |

Molmasse (osmometrisch)
ber. 570
gef. 562

Beispiel 7 Herstellung eines Urethanmethacrylats

72,73 g Hexamethylendiisocyanat und 0,2 g Dibutylzinndilaurat werden in 100 ml Chloroform gelöst und auf 40°C erwärmt. Bei dieser Temperatur wird langsam eine Mischung von 0,14 g 2,6-di-tert.-butyl-4-methyl-phenol und 98,7 g Glycerindimethacrylat zugetropft. Nachdem die Hälfte der NCO-Äquivalente umgesetzt ist (NCO-Bestimmung durch Reaktion mit Dibutylamin und Rücktitration des überschüssigen Dibutylamins mit 0,1 n Salzsäure), werden 100 g Bis-(tricyclo[5,2,1,$0^{2,6}$]decan-8(9)-yl)-1,1,3,3-tetramethyl-disiloxan, gelöst in 200 ml Chloroform, zugetropft. Man rührt die Mischung bis zum vollständigen Umsatz der NCO-Gruppen bei 40-60°C. Das Produkt wird nach Zugabe von Triethylenglykoldimethacrylat vom Lösungsmittel befreit, so daß eine Monomermischung mit einem Gehalt von 72 Gewichtsprozent des erfindungsgemäßen Methacrylsäureesters entsteht.

Anwendungsbeispiele

Beispiel 8

Herstellung eines Zahnfüllungsmaterials

Es wird eine Monomerlösung hergestellt, die folgende Zusammensetzung besitzt:

| Verbindung | Gewichtsteile |
|---|---|
| Monomer aus Beispiel 3 | 100 |
| p-Dimethylaminobenzolsulfon-säure-N,N-diallylamid | 0,5 |
| 2,3-Bornandion | 0,2 |
| Benzildimethylketal | 0,125 |

Die Monomerlösung härtet durch Bestrahlen mit einer handelsüblichen Dentallampe (Translux®) bei einer Belichtungsdauer von 60 Sekunden zu einem farblosen Kunststoff aus, der im Vergleich zu bekannten Dentalwerkstoffen eine deutlich verbesserte Abrasionsbeständigkeit aufweist.

Die Monomerlösung kann als Zahnlack verwendet werden.

Beispiel 9

Herstellung einer lichthärtenden Zahnfüllungsmasse

45 Gew.-Teilen der Monomerlösung aus Beispiel 8 wird 0,1 Gew.-Teil Tinuvin P zugesetzt.

Diese Mischung wird mit 55 Gew.-Teilen einer mit 5 % 3-Methacryloylpropyltrimethoxysilan silanierten pyrogenen Kieselsäure (spez. Oberfläche: 50 $m^2/g$) in einem Duplex-Kneter im Vakuum zu einer Paste verarbeitet.

Das erhaltene Material läßt sich durch Belichtung mit einer Dentallampe unter Verwendung von sichtbarem Licht (60 Sekunden) zu einem Formkörper aushärten.

Beispiel 10

Es wird eine Monomerlösung analog Beispiel 8 hergestellt, wobei jedoch an Stelle des Monomers aus Beispiel 3 das Urethanmethacrylat aus Beispiel 5 verwendet wird.

An einer lichtgehärteten Probe dieser Monomerlösung werden eine Biegefestigkeit von 64 $N/mm^2$ und ein Biegemodul von 1.852 $N/mm^2$ gemessen. (Prüfung gemäß DIN 13 922).
Die diametrale Zugfestigkeit beträgt 35 $N/mm^2$.

Auch bei diesem Kunststoff ergibt sich eine verbesserte Abrasionsbeständigkeit.
Er eignet sich besonders als Zahnfüllungsmaterial.

Beispiel 11

Herstellung einer lichthärtenden Zahnfüllungsmasse

Es wird eine Lösung aus 100 Gewichtsteilen der Monomermischung aus Beispiel 7, 0,2 Gew.-Teil Campherchinon, 0,1 Gew.-Teil Benzildimethylketal und 0,5 Gew.-Teilen 4-N,N-Dimethylamino-benzolsulfonsäure-bisallylamid hergestellt. 40 Gew.-Teile dieser Mischung werden mit 60 Gew.-Teilen einer mit 5 % 3-Methacryloyloxypropyltrimethoxysilan silanisierten hochdispersen Kieselsäure (BET-Oberfläche 50 $m^2/g$) in einem Duplex-Kneter im Vakuum zu einer Paste verarbeitet.

Das Material läßt sich durch Belichtung mit einer Dentallampe unter Verwendung von sichtbarem Licht (60 Sekunden) zum fertigen Dentalwerkstoff aushärten.

Man erhält einen Formkörper mit folgenden Eigenschaften:
Biegefestigkeit 93 $N/mm^2$, Biegemodul 4700 $N/mm^2$.

Durch Verwendung von Füllstoffmischungen aus silanisierter hochdisperser Kieselsäure und silanisierter Keramik (mit einem mittleren Partikeldurchmesser von etwa 4 $\mu m$) können je nach Verwendungszweck die mechanischen Eigenschaften angepaßt werden.

**Patentansprüche**

**Patentansprüche für folgende Vertragsstaaten : AT, BE, CH, DE, FR, GB, IT, LI, NL, SE**

**1.** (Meth)-acrylsäureester von Tricyclodecangruppen enthaltenden Siloxanen der Formel

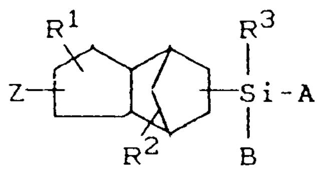 (I),

in der

$R^1$ und $R^2$ gleich oder verschieden sind und Wasserstoff, Niederalkyl, Halogen oder Trifluormethyl bedeuten,

$R^3$ geradkettiges oder verzweigtes $C_1$-$C_6$-Alkyl, Cycloalkyl, Cycloalkylalkyl oder gegebenenfalls substituiertes Aryl oder Aralkylbedeutet,

Z für die Gruppe

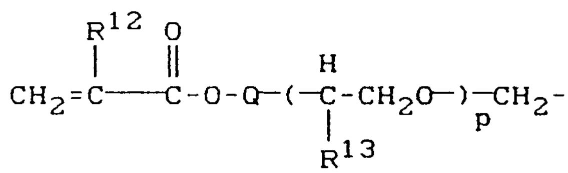

steht,

in der

$R^{12}$ und $R^{13}$ gleich oder verschieden sind und Wasserstoff oder Methyl bedeuten,

p für eine Zahl von 0 bis 20 steht und

Q für eine Einfachbindung steht oder einen Rest der Formel

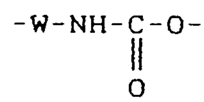

bedeutet,

in der

W eine Alkylenkette bedeutet, oder einen Rest der Formel

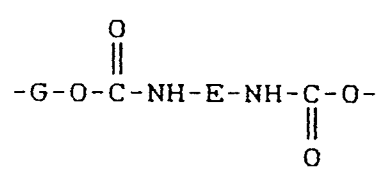

bedeutet,

in der

E ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, aromatischen, araliphatischen und/oder cycloaliphatischen Reste über gegebenenfalls substituierte Methylengruppen verbunden sein können,

und

G einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)acryloyloxy-Reste substituiert sein kann, bedeutet,

A für eine Siloxankette steht, die aus m Strukturelementen der Formel

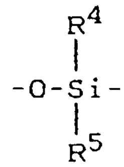 und/oder 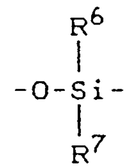

und der Endgruppe

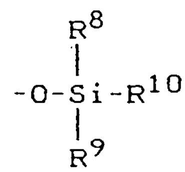

besteht,

wobei

$R^4$, $R^5$, $R^6$, $R^7$, $R^8$ und $R^9$ gleich oder verschieden sind und geradkettiges oder verzweigtes $C_1$-$C_6$-Alkyl, Cycloalkyl, Cycloalkyl-alkyl oder gegebenenfalls substituiertes Aryl oder Aralkyl bedeuten,

wobei die Reste $R^5$, $R^7$ und $R^8$ auch für die Gruppe

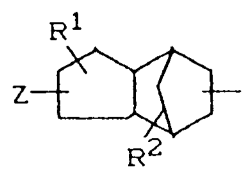

in der

$R^1$, $R^2$ und Z die obengenannte Bedeutung haben,

stehen,

$R^{10}$ für geradkettiges oder verzweigtes $C_1$-$C_6$-Alkyl steht und wobei die Summe der Strukturelemente m unabhängig voneinander für eine Zahl von 0 bis 600 steht, und

B den Bedeutungsumfang wie A haben kann, wobei die Reste $R^4$ bis $R^{10}$ in den Ketten A und B unterschiedlich sein können, oder für geradkettiges oder verzweigtes $C_1$-$C_6$-Alkyl steht.

**2.** (Meth)acrylsäureester von Tricyclodecangruppen enthaltenden Siloxanen nach Anspruch 1, worin

$R^1$ und $R^2$ gleich oder verschieden sind und Wasserstoff, geradkettiges oder verzweigtes $C_1$-$C_6$-Alkyl, Halogen oder Trifluormethyl bedeuten,

$R^3$ geradkettiges oder verzweigtes $C_1$-$C_6$-Alkyl, Cycloalkyl ($C_5$ bis $C_7$), Cycloalkyl-alkyl ($C_6$-$C_{13}$) oder gegebenenfalls substituiertes Aryl ($C_6$ bis $C_{12}$) oder Aralkyl ($C_7$ bis $C_{18}$) bedeutet,

Z für die Gruppe

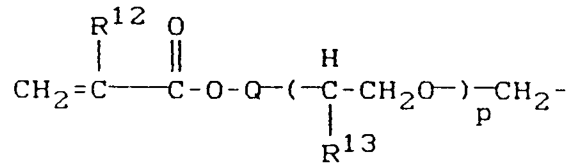

steht,

in der

$R^{12}$ und $R^{13}$ gleich oder verschieden sind und Wasserstoff oder Methyl bedeuten,

p für eine Zahl von 0 bis 20 steht und

Q für eine Einfachbindung steht oder einen Rest der Formel

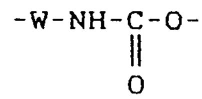

bedeutet,

in der

W eine Alkylenkette mit 2 bis 10 Kohlenstoffatomen bedeutet, oder einen Rest der Formel

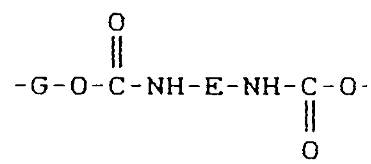

bedeutet,

in der

E ein zweiwertiger, geradkettiger oder verzweigter aliphatischer Rest mit 2 bis 12 Kohlenstoffatomen, ein aromatischer Rest mit 6 bis 18 Kohlenstoffatomen oder ein cycloaliphatischer Rest mit 6 bis 14 Kohlenstoffatomen ist, wobei die aliphatischen, aromatischen und/oder cycloaliphatischen Reste 1 oder 2 Sauerstoffbrücken enthalten können, und wobei mehrere der aliphatischen, aromatischen und/oder cycloaliphatischen Reste über gegebenenfalls substituierte Methylengruppen verbunden sein können,

und

G einen zweiwertigen geradkettigen oder verzweigten aliphatischen Kohlenwasserstoffrest mit 3 bis 10 Kohlenstoffatomen, der gegebenenfalls 1 bis 3 Sauerstoffbrücken enthalten kann und gegebenenfalls durch 1 oder 2 zusätzliche (Meth)-acryloyloxy-Reste substituiert sein kann, bedeutet,

A für eine Siloxankette steht, die aus m Strukturelementen der Formel

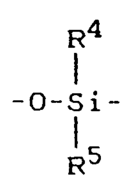

und/oder

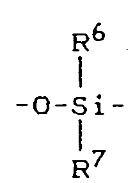

und der Endgruppe

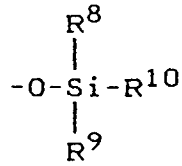

besteht,
wobei

$R^4$, $R^5$, $R^6$, $R^7$, $R^8$ und $R^9$ gleich oder verschieden sind und geradkettiges oder verzweigtes $C_1$-$C_6$-Alkyl, Cycloalkyl ($C_5$ bis $C_7$), Cycloalkyl-alkyl ($C_6$-$C_{13}$) oder gegebenenfalls substituiertes Aryl ($C_6$ bis $C_{12}$) oder Aralkyl ($C_7$ bis $C_{18}$) bedeuten,
wobei die Reste $R^5$, $R^7$ und $R^8$ auch für die Gruppe

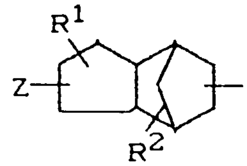

in der
$R^1$, $R^2$ und Z die obengenannte Bedeutung haben,
stehen,

$R^{10}$ für geradkettiges oder verzweigtes $C_1$-$C_6$-Alkyl steht und
wobei die Summe der Strukturelemente m unabhängig voneinander für eine Zahl von 0 bis 200 steht, und

B den Bedeutungsumfang wie A haben kann,
wobei die Reste $R^4$ bis $R^{10}$ in den Ketten A und B unterschiedlich sein können,
oder für geradkettiges oder verzweigtes $C_1$-$C_6$-Alkyl steht.

**3.** (Meth)-acrylsäureester von Tricyclodecangruppen enthaltenden Siloxanen nach den Ansprüchen 1 und 2, worin

$R^1$ und $R^2$ gleich oder verschieden sind und Wasserstoff oder geradkettiges oder verzweigtes $C_1$-$C_6$-Alkyl bedeuten,

$R^3$ geradkettiges oder verzweigtes $C_1$-$C_6$-Alkyl, Cycloalkyl ($C_5$ bis $C_7$), Cycloalkyl-alkyl ($C_6$-$C_{10}$) oder gegebenenfalls substituiertes Aryl ($C_6$ bis $C_{11}$) oder Aralkyl ($C_7$ bis $C_{11}$) bedeutet,

Z für die Gruppe

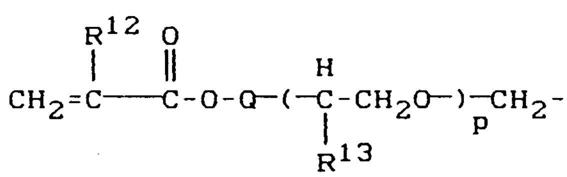

steht,
in der

$R^{12}$ und $R^{13}$ gleich oder verschieden sind und Wasserstoff oder Methyl bedeuten,

p für eine Zahl von 0 bis 4 steht und

Q für eine Einfachbindung steht
oder einen Rest der Formel

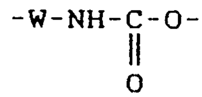

bedeutet,
in der

W eine Alkylenkette mit 2 bis 6 Kohlenstoffatomen bedeutet, oder einen Rest der Formel

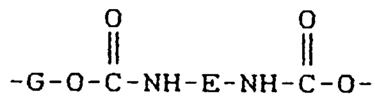

bedeutet,
in der

E ein zweiwertiger, geradkettiger oder verzweigter aliphatischer Rest mit 2 bis 8 Kohlenstoffatomen oder ein cycloaliphatischer Rest mit 6 bis 14 Kohlenstoffatomen ist
und

G einen zweiwertigen geradkettigen oder verzweigten aliphatischen Kohlenwasserstoffrest mit 3 bis 10 Kohlenstoffatomen, der gegebenenfalls 1 Sauerstoffbrücke enthalten kann und gegebenenfalls durch 1 oder 2 zusätzliche (Meth)-acryloyloxy-Reste substituiert sein kann, bedeutet,

A für eine Siloxankette steht, die aus m Strukturelementen der Formel

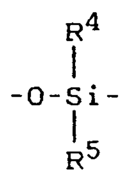

**und/oder**

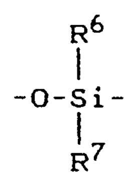

und der Endgruppe

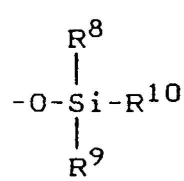

besteht,
wobei

$R^4$, $R^5$, $R^6$, $R^7$, $R^8$ und $R^9$ gleich oder verschieden sind und geradkettiges oder verzweigtes $C_1$-$C_6$-Alkyl, Cycloalkyl ($C_5$ bis $C_7$), Cycloalkyl-alkyl ($C_6$-$C_{10}$) oder gegebenenfalls substituiertes Aryl ($C_6$ bis $C_{11}$) oder Aralkyl ($C_7$ bis $C_{11}$) bedeuten,
wobei die Reste $R^5$, $R^7$ und $R^8$ auch für die Gruppe

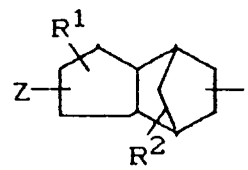

in der

$R^1$, $R^2$ und Z die obengenannte Bedeutung haben,

stehen,

$R^{10}$ für geradkettiges oder verzweigtes $C_1$-$C_6$-Alkyl steht und wobei die Summe der Strukturelemente m unabhängig voneinander für eine Zahl von 0 bis 200 steht, und

B den Bedeutungsumfang wie A haben kann, wobei die Reste $R^4$ bis $R^{10}$ in den Ketten A und B unterschiedlich sein können, oder für geradkettiges oder verzweigtes $C_1$-$C_6$-Alkyl steht.

4. (Meth)-acrylsäureester von Tricyclodecangruppen enthaltenden Siloxanen nach den Ansprüchen 1 bis 3, dadurch gekennzeichnet, daß 0,5 bis 100 Mol-% aller Siliciumatome durch Tricyclo[5.2.1.$0^{2,6}$]-decanyl-Gruppen substituiert sind.

5. (Meth)-acrylsäureester von Tricyclodecangruppen enthaltenden Siloxanen nach den Ansprüchen 1 bis 4, dadurch gekennzeichnet, daß die Anzahl m der Strukturelemente 0 bis 50 beträgt.

6. Verfahren zur Herstellung von (Meth)-acrylsäureestern von Tricyclodecangruppen enthaltenden Siloxanen der Formel

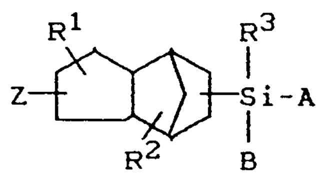

(I),

in der

$R^1$ und $R^2$ gleich oder verschieden sind und Wasserstoff, geradkettiges oder verzweigtes $C_1$-$C_6$-Alkyl, Halogen oder Trifluormethyl bedeuten,

$R^3$ geradkettiges oder verzweigtes $C_1$-$C_6$-Alkyl, Cycloalkyl, Cycloalkylalkyl oder gegebenenfalls substituiertes Aryl oder Aralkyl bedeuten,

Z für die Gruppe

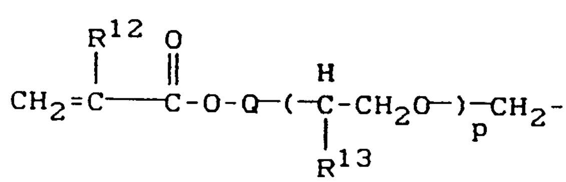

steht,

in der

$R^{12}$ und $R^{13}$ gleich oder verschieden sind und Wasserstoff oder Methyl bedeuten,

p für eine Zahl von 0 bis 20 steht und

Q für eine Einfachbindung steht oder einen Rest der Formel

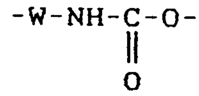

bedeutet,
in der

W — eine Alkylenkette bedeutet, oder einen Rest der Formel

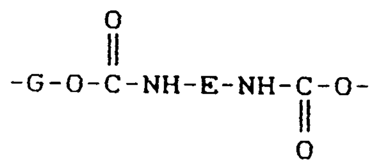

bedeutet,
in der

E — ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, aromatischen, araliphatischen und/oder cycloaliphatischen Reste über gegebenenfalls substituierte Methylengruppen verbunden sein können,
und

G — einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acryloyloxy-Reste substituiert sein kann, bedeutet,

A — für eine Siloxankette steht, die aus m Strukturelementen der Formel

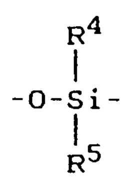

und/oder

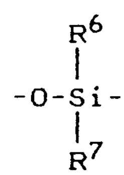

und der Endgruppe

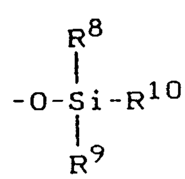

besteht,
wobei

$R^4$, $R^5$, $R^6$, $R^7$, $R^8$ und $R^9$ — gleich oder verschieden sind und geradkettiges oder verzweigtes $C_1$-$C_6$-Alkyl, Cycloalkyl, Cycloalkyl-alkyl oder gegebenenfalls substituiertes Aryl oder Aralkyl bedeuten,

wobei die Reste $R^5$, $R^7$ und $R^8$ auch für die Gruppe

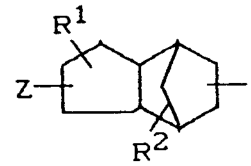

in der

$R^1$, $R^2$ und Z die obengenannte Bedeutung haben,

stehen,

$R^{10}$ für geradkettiges oder verzweigtes $C_1$-$C_6$-Alkyl steht und wobei die Summe der Strukturelemente m unabhängig voneinander für eine Zahl von 0 bis 600 steht, und

B den Bedeutungsumfang wie A haben kann, wobei die Reste $R^4$ bis $R^{10}$ in den Ketten A und B unterschiedlich sein können, oder für geradkettiges oder verzweigtes $C_1$-$C_6$-Alkyl steht,

das dadurch gekennzeichnet ist, daß man Poly[hydroxy(alkylenoxy)-methyl-tricyclo[5.2.1.0$^{2.6}$]-decanyl]-siloxane der Formel

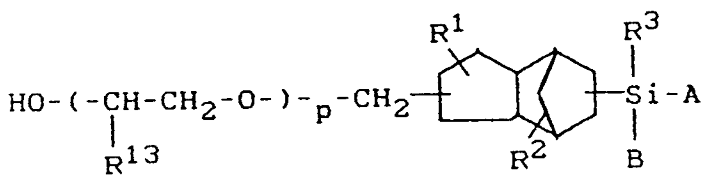 (II),

in der

$R^1$, $R^2$, $R^3$, $R^{13}$, p, A und B die obengenannte Bedeutung haben,

für den Fall der Herstellung von Verbindungen, in denen Q für eine Einfachbindung steht, mit einem (Meth)-acrylsäure-Derivat der Formel

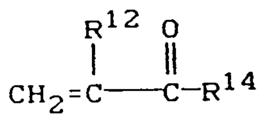 (III),

in der

$R^{12}$ die obengenannte Bedeutung hat und $R^{14}$ für Hydroxy, Chlor, Methoxy, Ethoxy oder (Meth)acryloyloxy steht, verestert

oder

für den Fall der Herstellung von Verbindungen, in denen Q für einen Rest der Formel

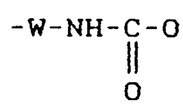

steht,

in der

W für eine Alkylenkette steht,

mit einem Isocyanat der Formel

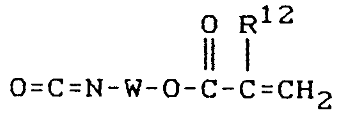 (IV),

in der

$R^{12}$ und W die obengenannte Bedeutung haben,

in einem inerten Lösungsmittel umsetzt,

oder für den Fall der Herstellung von Verbindungen, in denen Q für einen Rest der Formel

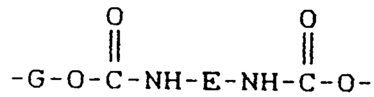

steht,

in der

E ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, aromatischen, araliphatischen und/oder cycloaliphatischen Reste über gegebenenfalls substituierte Methylengruppen verbunden sein können,

und

G einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acryloyloxy-Reste substituiert sein kann, bedeutet,

mit dem Additionsprodukt aus 1 Mol eines Diisocyanats der Formel

$$O=C=N\text{-}E\text{-}N=C=O \qquad (V),$$

in der

E die oben angegebene Bedeutung hat,

und 1 Mol eines Hydroxyalkyl(meth)acrylsäureesters der Formel

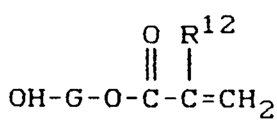 (VI),

in der

G und $R^{12}$ die oben angegebene Bedeutung haben,

in einem inerten Lösungsmittel umsetzt,

wobei eine stöchiometrische Äquivalenz zwischen den NCO-Gruppen des Adduktes aus V und VI und den OH-Gruppen von II bestehen soll.

**7.** Verfahren nach Anspruch 6, dadurch gekennzeichnet, daß man die Umsetzung mit dem (Meth)-acrylsäure-Derivat im Temperaturbereich von 50 bis 120 °C durchführt.

**8.** Verfahren nach Anspruch 6, dadurch gekennzeichnet, daß man die Umsetzung mit dem Isocyanat in Gegenwart eines Katalysators und eines Polymerisations-Inhibitors im Temperaturbereich von 20 bis 100 ° C durchführt.

**9.** Polymerisate von Monomermischungen, enthaltend (Meth)-acrylsäureester von Tricyclodecangruppen enthaltenden Siloxanen der Formel

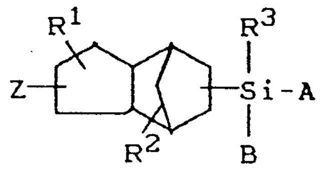

(I),

in der

$R^1$ und $R^2$ gleich oder verschieden sind und Wasserstoff, geradkettiges oder verzweigtes $C_1$-$C_6$-Alkyl, Halogen oder Trifluormethyl bedeuten,

$R^3$ geradkettiges oder verzweigtes $C_1$-$C_6$-Alkyl, Cycloalkyl, Cycloalkyl-alkyl oder gegebenenfalls substituiertes Aryl oder Aralkyl

Z für die Gruppe

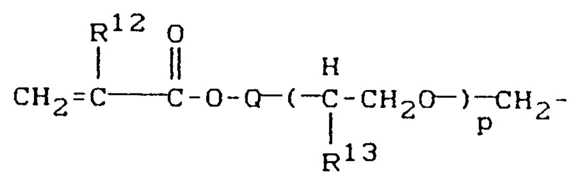

steht,

in der

$R^{12}$ und $R^{13}$ gleich oder verschieden sind und Wasserstoff oder Methyl bedeuten,

p für eine Zahl von 0 bis 20 steht und

Q für eine Einfachbindung steht oder einen Rest der Formel

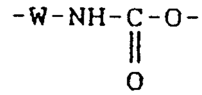

bedeutet,

in der

W eine Alkylenkette bedeutet, oder einen Rest der Formel

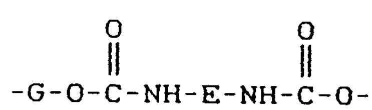

bedeutet,

in der

E ein zweiwertiger, geradkettiger oder verzweigter aliphatischer Rest mit 2 bis 24 Kohlenstoffatomen, ein aromatischer Rest mit 6 bis 26

Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, aromatischen, araliphatischen und/oder cycloaliphatischen Reste über gegebenenfalls substituierte Methylengruppen verbunden sein können,
und

G einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acryloyloxy-Reste substituiert sein kann, bedeutet,

A für eine Siloxankette steht, die aus m Strukturelementen der Formel

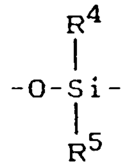

und/oder

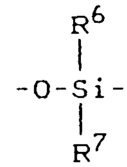

und der Endgruppe

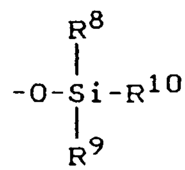

besteht,
wobei

$R^4$, $R^5$, $R^6$, $R^7$, $R^8$ und $R^9$ gleich oder verschieden sind und geradkettiges oder verzweigtes $C_1$-$C_6$-Alkyl, Cycloalkyl, Cycloalkyl-alkyl oder gegebenenfalls substituiertes Aryl oder Aralkyl bedeuten,
wobei die Reste $R^5$, $R^7$ und $R^8$ auch für die Gruppe

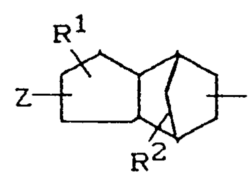

in der

$R^1$, $R^2$ und Z die obengenannte Bedeutung haben,
stehen,

$R^{10}$ für geradkettiges oder verzweigtes $C_1$-$C_6$-Alkyl steht und
wobei die Summe der Strukturelemente m unabhängig voneinander für eine Zahl von 0 bis 600 steht, und

B den Bedeutungsumfang wie A haben kann,
wobei die Reste $R^4$ bis $R^{10}$ in den Ketten A und B unterschiedlich sein können,
oder für geradkettiges oder verzweigtes $C_1$-$C_6$-Alkyl steht.

**10.** Dentalmaterial, enthaltend (Meth)-acrylsäureester von Tricyclodecangruppen enthaltenden Siloxanen

der Formel

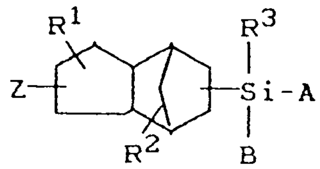

(I),

in der

$R^1$ und $R^2$ gleich oder verschieden sind und Wasserstoff, geradkettiges oder verzweigtes $C_1$-$C_6$-Alkyl, Halogen oder Trifluormethyl bedeuten,

$R^3$ geradkettiges oder verzweigtes $C_1$-$C_6$-Alkyl, Cycloalkyl, Cycloalkylalkyl oder gegebenenfalls substituiertes Aryl oder Aralkyl bedeuten,

Z für die Gruppe

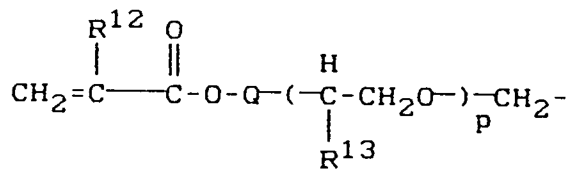

steht,
in der

$R^{12}$ und $R^{13}$ gleich oder verschieden sind und Wasserstoff oder Methyl bedeuten,

p für eine Zahl von 0 bis 20 steht und

Q für eine Einfachbindung steht
oder einen Rest der Formel

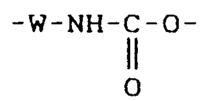

bedeutet,
in der

W eine Alkylenkette bedeutet,
oder einen Rest der Formel

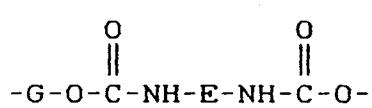

bedeutet,
in der

E ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, araliphatischen, aromatischen und/oder cycloaliphatischen Reste über gegebenenfalls

substituierte Methylengruppen verbunden sein können, und

G einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acryloyloxy-Reste substituiert sein kann, bedeutet,

A für eine Siloxankette steht, die aus m Strukturelementen der Formel

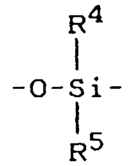

und/oder

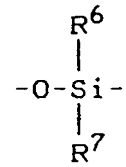

und der Endgruppe

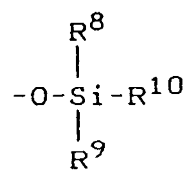

besteht, wobei

$R^4$, $R^5$, $R^6$, $R^7$, $R^8$ und $R^9$ gleich oder verschieden sind und geradkettiges oder verzweigtes $C_1$-$C_6$-Alkyl, Cycloalkyl, Cycloalkyl-alkyl oder gegebenenfalls substituiertes Aryl oder Aralkyl bedeuten, wobei die Reste $R^5$, $R^7$ und $R^8$ auch für die Gruppe

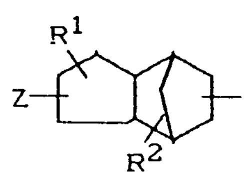

in der

$R^1$, $R^2$ und Z die obengenannte Bedeutung haben, stehen,

$R^{10}$ für geradkettiges oder verzweigtes $C_1$-$C_6$-Alkyl steht und wobei die Summe der Strukturelemente m unabhängig voneinander für eine Zahl von 0 bis 600 steht, und

B den Bedeutungsumfang wie A haben kann, wobei die Reste $R^4$ bis $R^{10}$ in den Ketten A und B unterschiedlich sein können, oder für geradkettiges oder verzweigtes $C_1$-$C_6$-Alkyl steht.

**11.** Dentalmaterial nach Anspruch 10, dadurch gekennzeichnet, daß es als Monomer 30 bis 100 Gew.-% (Meth)-acrylsäureester von Tricyclodecangruppen enthaltenden Siloxanen enthält.

**12.** Dentalmaterial nach den Ansprüchen 10 und 11, dadurch gekennzeichnet, daß es (Meth)-acrylsäureester von Tricyclodecangruppen enthaltenden Siloxanen und Hilfsstoffe enthält.

**13.** Verwendung von (Meth)-acrylsäureestern von Tricyclodecangruppen enthaltenden Siloxanen der For-

mel

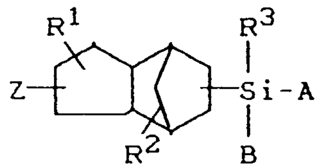 (I),

in der

R¹ und R² — gleich oder verschieden sind und Wasserstoff, geradkettiges oder verzweigtes $C_1$-$C_6$-Alkyl, Halogen oder Trifluormethyl bedeuten,

$R^3$ — geradkettiges oder verzweigtes $C_1$-$C_6$-Alkyl, Cycloalkyl, Cycloalkyl-alkyl oder gegebenenfalls substituiertes Aryl oder Aralkyl bedeutet,

Z — für die Gruppe

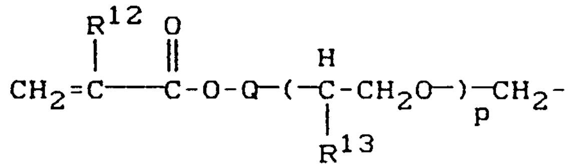

steht,
in der

$R^{12}$ und $R^{13}$ — gleich oder verschieden sind und Wasserstoff oder Methyl bedeuten,

p — für eine Zahl von 0 bis 20 steht und

Q — für eine Einfachbindung steht
oder einen Rest der Formel

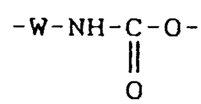

bedeutet,
in der

W — eine Alkylenkette bedeutet,
oder einen Rest der Formel

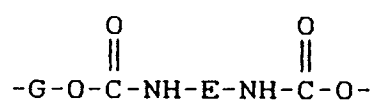

bedeutet,
in der

E — ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, araliphatischen, aromatischen und/oder cycloaliphatischen Reste über gegebenenfalls

substituierte Methylengruppen verbunden sein können,
und

G — einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acryloyloxy-Reste substituiert sein kann, bedeutet,

A — für eine Siloxankette steht, die aus m Strukturelementen der Formel

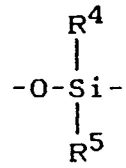
und/oder
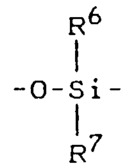

und der Endgruppe

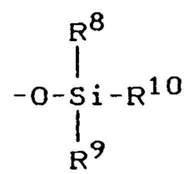

besteht,
wobei

$R^4$, $R^5$, $R^6$, $R^7$, $R^8$ und $R^9$ — gleich oder verschieden sind und geradkettiges oder verzweigtes $C_1$-$C_6$-Alkyl, Cycloalkyl, Cycloalkyl-alkyl oder gegebenenfalls substituiertes Aryl oder Aralkyl bedeuten,
wobei die Reste $R^5$, $R^7$ und $R^8$ auch für die Gruppe

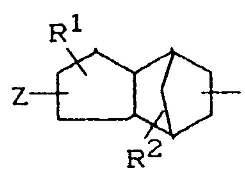

in der

$R^1$, $R^2$ und Z — die obengenannte Bedeutung haben,
stehen,

$R^{10}$ — für geradkettiges oder verzweigtes $C_1$-$C_6$-Alkyl steht und
wobei die Summe der Strukturelemente m unabhängig voneinander für eine Zahl von 0 bis 600 steht, und

B — den Bedeutungsumfang wie A haben kann,
wobei die Reste $R^4$ bis $R^{10}$ in den Ketten A und B unterschiedlich sein können,
oder für geradkettiges oder verzweigtes $C_1$-$C_6$-Alkyl steht,
in Dentalmaterialien.

**14.** Verwendung nach Anspruch 13 in Füllungsmaterialien für Zahne.

**15.** Verwendung nach Anspruch 13 in Beschichtungsmitteln für Zähne.

**16.** Verwendung nach Anspruch 13, als Komponente bei der Herstellung von Zahnersatz.

**17.** Verwendung nach Anspruch 13, als Komponente zur Herstellung von Dentalformkörpern.

**Patentansprüche für folgenden Vertragsstaat : ES**

**1.** Verfahren zur Herstellung von (Meth)-acrylsäureestern von Tricyclodecangruppen enthaltenden Siloxanen der Formel

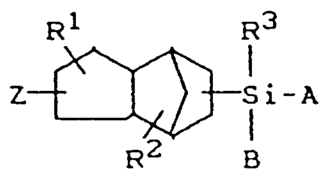

(I),

in der

| | |
|---|---|
| $R^1$ und $R^2$ | gleich oder verschieden sind und Wasserstoff, geradkettiges oder verzweigtes $C_1$-$C_6$-Alkyl, Halogen oder Trifluormethyl bedeuten, |
| $R^3$ | geradkettiges oder verzweigtes $C_1$-$C_6$-Alkyl, Cycloalkyl, Cycloalkylalkyl oder gegebenenfalls substituiertes Aryl oder Aralkyl bedeuten, |
| Z | für die Gruppe |

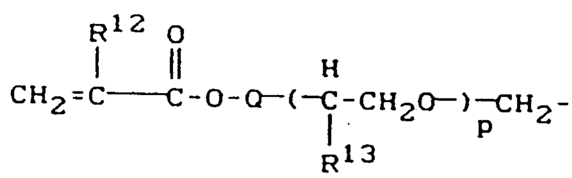

steht,
in der

| | |
|---|---|
| $R^{12}$ und $R^{13}$ | gleich oder verschieden sind und Wasserstoff oder Methyl bedeuten, |
| p | für eine Zahl von 0 bis 20 steht und |
| Q | für eine Einfachbindung steht oder einen Rest der Formel |

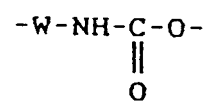

bedeutet,
in der

| | |
|---|---|
| W | eine Alkylenkette bedeutet, oder einen Rest der Formel |

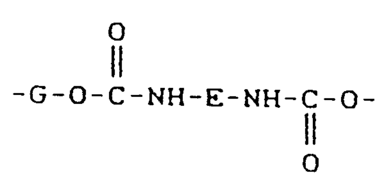

bedeutet,
in der

| | |
|---|---|
| E | ein zweiwertiger, geradkettiger oder verzweigter aliphatischer Rest mit |

2 bis 24 Kohlenstoffatomen, ein aromatischer Rest mit 6 bis 26 Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, aromatischen, araliphatischen und/oder cycloaliphatischen Reste über gegebenenfalls substituierte Methylengruppen verbunden sein können,

und

G — einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acryloyloxy-Reste substituiert sein kann, bedeutet,

A — für eine Siloxankette steht, die aus m Strukturelementen der Formel

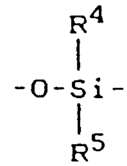

und/oder

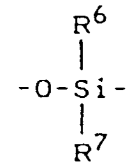

und der Endgruppe

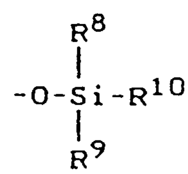

besteht,

wobei

$R^4$, $R^5$, $R^6$, $R^7$, $R^8$ und $R^9$ — gleich oder verschieden sind und geradkettiges oder verzweigtes $C_1$-$C_6$-Alkyl, Cycloalkyl, Cycloalkyl-alkyl oder gegebenenfalls substituiertes Aryl oder Aralkyl bedeuten,

wobei die Reste $R^5$, $R^7$ und $R^8$ auch für die Gruppe

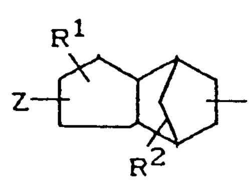

in der

$R^1$, $R^2$ und Z — die obengenannte Bedeutung haben,

stehen,

$R^{10}$ — für geradkettiges oder verzweigtes $C_1$-$C_6$-Alkyl steht und

wobei die Summe der Strukturelemente m unabhängig voneinander für eine Zahl von 0 bis 600 steht, und

B — den Bedeutungsumfang wie A haben kann,

wobei die Reste $R^4$ bis $R^{10}$ in den Ketten A und B unterschiedlich sein können,

oder für geradkettiges oder verzweigtes $C_1$-$C_6$-Alkyl steht,
das dadurch gekennzeichnet ist, daß man Poly[hydroxy(alkylenoxy)-methyl-tricyclo[5.2.1.$0^{2.6}$]-decanyl]-siloxane der Formel

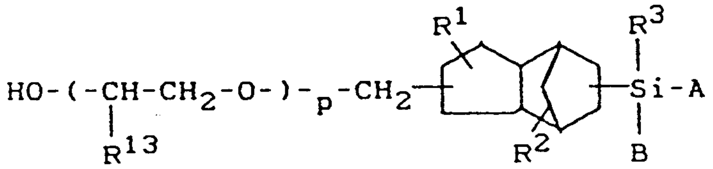

(II),

in der

$R^1$, $R^2$, $R^3$, $R^{13}$, p, A und B die obengenannte Bedeutung haben,
für den Fall der Herstellung von Verbindungen, in denen Q für eine Einfachbindung steht, mit einem (Meth)-acrylsäure-Derivat der Formel

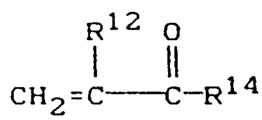

(III),

in der

$R^{12}$ die obengenannte Bedeutung hat und $R^{14}$ für Hydroxy, Chlor, Methoxy, Ethoxy oder (Meth)acryloyloxy steht, verestert
oder
für den Fall der Herstellung von Verbindungen, in denen Q für einen Rest der Formel

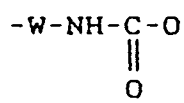

steht,
in der

W für eine Alkylenkette steht,
mit einem Isocyanat der Formel

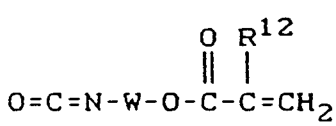

(IV),

in der

$R^{12}$ und W die obengenannte Bedeutung haben,
in einem inerten Lösungsmittel umsetzt,
oder für den Fall der Herstellung von Verbindungen, in denen Q für einen Rest der Formel

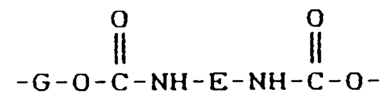

steht,
in der

E ein zweiwertiger, geradkettiger oder verzweigter aliphatischer Rest mit 2 bis 24 Kohlenstoffatomen, ein aromatischer Rest mit 6 bis 26 Kohlenstoffatomen, ein araliphatischer Rest mit 7 bis 26 Kohlenstoffatomen oder ein cycloaliphatischer Rest mit 6 bis 26 Kohlenstoffatomen ist, wobei die aliphatischen, aromatischen, araliphatischen und/oder cycloaliphatischen Reste 1 oder 2 Sauerstoffbrücken enthalten können, und wobei mehrere der aliphatischen, aromatischen, araliphatischen und/oder cycloaliphatischen Reste über gegebenenfalls substituierte Methylengruppen verbunden sein können,
und

G einen zweiwertigen geradkettigen oder verzweigten aliphatischen Kohlenwasserstoffrest mit 3 bis 15 Kohlenstoffatomen, der gegebenenfalls 1 bis 3 Sauerstoffbrücken enthalten kann und gegebenenfalls durch 1 bis 4 zusätzliche (Meth)-acryloyloxy-Reste substituiert sein kann, bedeutet,

mit dem Additionsprodukt aus 1 Mol eines Diisocyanats der Formel

$$O=C=N\text{-}E\text{-}N=C=O \qquad (V),$$

in der

E die oben angegebene Bedeutung hat,

und 1 Mol eines Hydroxyalkyl(meth)acrylsäureesters der Formel

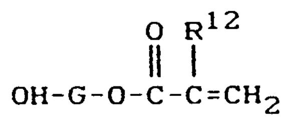 (VI),

in der

G und $R^{12}$ die oben angegebene Bedeutung haben,

in einem inerten Lösungsmittel umsetzt,
wobei eine stöchiometrische Äquivalenz zwischen den NCO-Gruppen des Adduktes aus V und VI und den OH-Gruppen von II bestehen soll.

**2.** Verfahren nach Anspruch 1, dadurch gekennzeichnet, daß man die Umsetzung mit dem (Meth)-acrylsäure-Derivat im Temperaturbereich von 50 bis 120 ° C durchführt.

## Claims

## Claims for the following Contracting States : AT, BE, CH, DE, FR, GB, IT, LI, NL, SE

**1.** (Meth)-acrylates of siloxanes containing tricyclodecane groups, of the formula

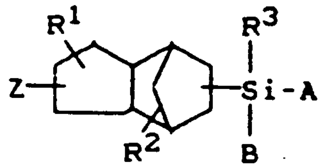 (I),

in which

$R^1$ and $R^2$ are identical or different and denote hydrogen, lower alkyl, halogen or trifluoromethyl,

$R^3$ denotes straight-chain or branched $C_1$-$C_6$-alkyl, cycloalkyl, cycloalkyl-alkyl or optionally substituted aryl or aralkyl,

Z represents the

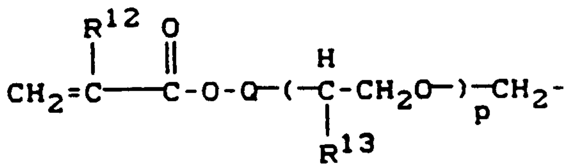

group,
in which

$R^{12}$ and $R^{13}$ are identical or different and denote hydrogen or methyl,

p represents a number from 0 to 20 and

Q represents a single bond, or denotes a radical of the formula

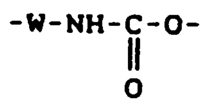

in which

W denotes an alkylene chain,
or denotes a radical of the formula

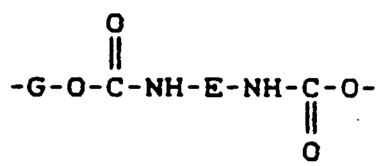

in which

E is a divalent, straight-chain or branched aliphatic radical having 2 to 24 carbon atoms, an aromatic radical having 6 to 26 carbon atoms, an araliphatic radical having 7 to 26 carbon atoms, or a cycloaliphatic radical having 6 to 26 carbon atoms, it being possible for the aliphatic, aromatic, araliphatic and/or cycloaliphatic radicals to contain 1 or 2 oxygen bridges, and it being possible for several of the aliphatic, aromatic, araliphatic and/or cycloaliphatic radicals to be connected via optionally substituted methylene groups,

and

G denotes a divalent straight-chain or branched aliphatic hydrocarbon radical, having 3 to 15 carbon atoms, which may optionally contain 1

to 3 oxygen bridges and may optionally be substituted by 1 to 4 additional (meth)acryloyloxy radicals,

A — represents a siloxane chain which comprises m structural elements of the formula

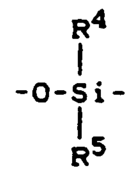

and/or

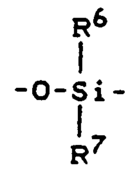

and the terminal group

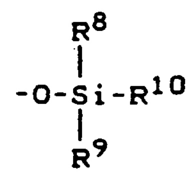

where

$R^4$, $R^5$, $R^6$, $R^7$, $R^8$ and $R^9$ — are identical or different and denote straight-chain or branched $C_1$-$C_6$-alkyl, cycloalkyl, cycloalkyl-alkyl or optionally substituted aryl or aralkyl, where the radicals $R^5$, $R^7$ and $R^8$ alternatively represent the

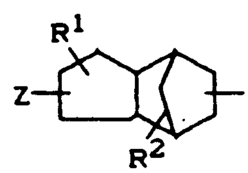

group, in which

$R^1$, $R^2$ and Z — have the abovementioned meaning,

$R^{10}$ — represents straight-chain or branched $C_1$-$C_6$-alkyl, and where the total number of structural elements m, independently of one another, represents a number from 0 to 600, and

B — can have the same range of meanings as A, it being possible for the radicals $R^4$ to $R^{10}$ in the chains A and B to be different, or represents straight-chain or branched $C_1$-$C_6$-alkyl.

**2.** (Meth)acrylates of siloxanes containing tricyclodecane groups, according to Claim 1, in which

$R^1$ and $R^2$ — are identical or different and denote hydrogen, straight-chain or branched $C_1$-$C_6$-alkyl, halogen or trifluoromethyl,

$R^3$ — denotes straight-chain or branched $C_1$-$C_6$-alkyl, cycloalkyl ($C_5$ to $C_7$), cycloalkyl-alkyl ($C_6$ to $C_{13}$) or optionally substituted aryl ($C_6$ to $C_{12}$) or aralkyl ($C_7$ to $C_{18}$),

Z — represents the

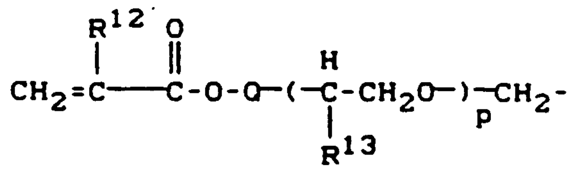

group,

in which

$R^{12}$ and $R^{13}$ are identical or different and denote hydrogen or methyl,

p represents a number from 0 to 20, and

Q represents a single bond or denotes a radical of the formula

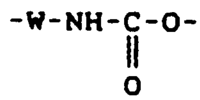

in which

W denotes an alkylene chain having 2 to 10 carbon atoms, or denotes a radical of the formula

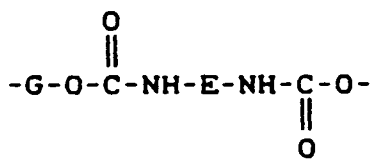

in which

E is a divalent, straight-chain or branched aliphatic radical having 2 to 12 carbon atoms, an aromatic radical having 6 to 18 carbon atoms, or a cycloaliphatic radical having 6 to 14 carbon atoms, it being possible for the aliphatic, aromatic and/or cycloaliphatic radicals to contain 1 or 2 oxygen bridges, and it being possible for several of the aliphatic, aromatic and/or cycloaliphatic radicals to be connected via optionally substituted methylene groups,

and

G denotes a divalent straight-chain or branched aliphatic hydrocarbon radical, having 3 to 10 carbon atoms, which can optionally contain 1 to 3 oxygen bridges and can optionally be substituted by 1 or 2 additional (meth)acryloyloxy radicals,

A represents a siloxane chain which comprises m structural elements of the formula

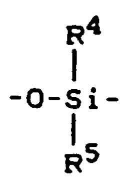

and/or

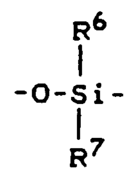

and the terminal group

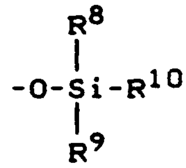

where

$R^4$, $R^5$, $R^6$, $R^7$, $R^8$ and $R^9$ are identical or different and denote straight-chain or branched $C_1$-$C_6$-alkyl, cycloalkyl ($C_5$ to $C_7$), cycloalkyl-alkyl ($C_6$-$C_{13}$) or optionally substituted aryl ($C_6$ to $C_{12}$) or aralkyl ($C_7$ to $C_{18}$),

where the radicals $R^5$, $R^7$ and $R^8$ alternatively represent the

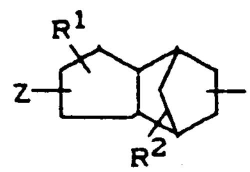

group,

in which

$R^1$, $R^2$ and Z have the abovementioned meaning,

$R^{10}$ represents straight-chain or branched $C_1$-$C_6$-alkyl, and where the total number of structural elements m, independently of one another, represents a number from 0 to 200, and

B can have the same range of meanings as A, it being possible for the radicals $R^4$ to $R^{10}$ in the chains A and B to be different, or represents straight-chain or branched $C_1$-$C_6$-alkyl.

**3.** (Meth)-acrylates of siloxanes containing tricyclodecane groups, according to Claims 1 and 2, in which

$R^1$ and $R^2$ are identical or different and denote hydrogen or straight-chain or branched $C_1$-$C_6$-alkyl,

$R^3$ denotes straight-chain or branched $C_1$-$C_6$-alkyl, cycloalkyl ($C_5$ to $C_7$), cycloalkyl-alkyl ($C_6$ to $C_{10}$), or optionally substituted aryl ($C_6$ to $C_{11}$) or aralkyl ($C_7$ to $C_{11}$),

Z represents the

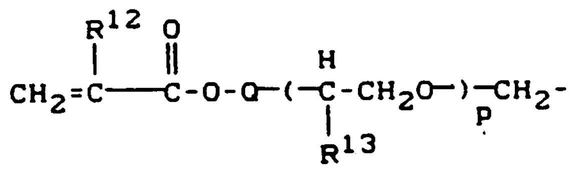

group,

in which

$R^{12}$ and $R^{13}$ are identical or different and denote hydrogen or methyl,

p represents a number from 0 to 4, and

Q represents a single bond or denotes a radical of the formula

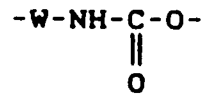

in which

W denotes an alkylene chain having 2 to 6 carbon atoms, or denotes a radical of the formula

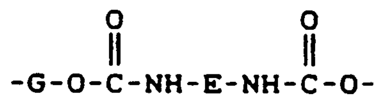

in which

E is a divalent, straight-chain or branched aliphatic radical having 2 to 8 carbon atoms or a cycloaliphatic radical having 6 to 14 carbon atoms, and

G denotes a divalent straight-chain or branched aliphatic hydrocarbon radical, having 3 to 10 carbon atoms, which may optionally contain 1 oxygen bridge and may optionally be substituted by 1 or 2 additional (meth)-acryloyloxy radicals,

A represents a siloxane chain which comprises m structural elements of the formula

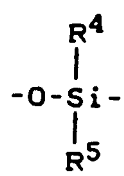

and/or

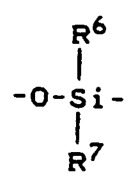

and the terminal group

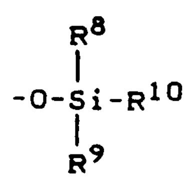

where

$R^4$, $R^5$, $R^6$, $R^7$, $R^8$ and $R^9$ are identical or different and denote straight-chain or branched $C_1$-$C_6$-alkyl, cycloalkyl ($C_5$ to $C_7$), cycloalkyl-alkyl ($C_6$ to $C_{10}$), or optionally substituted aryl ($C_6$ to $C_{11}$) or aralkyl ($C_7$ to $C_{11}$), where the radicals $R^5$, $R^7$ and $R^8$ alternatively represent the

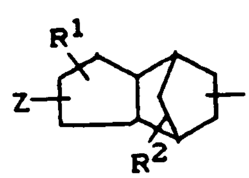

group

in which

$R^1$, $R^2$ and Z — have the abovementioned meaning,
$R^{10}$ — represents straight-chain or branched $C_1$-$C_6$-alkyl, and where the total number of structural elements m, independently of one another, represents a number from 0 to 200, and
B — can have the same range of meanings as A, it being possible for the radicals $R^4$ to $R^{10}$ in the chains A and B to be different, or represents straight-chain or branched $C_1$-$C_6$-alkyl.

**4.** (Meth)-acrylates of siloxanes containing tricyclodecane groups, according to Claims 1 to 3, characterised in that 0.5 to 100 mol % of all silicon atoms are substituted by tricyclo[5.2.1.$0^{2.6}$]decanyl groups.

**5.** (Meth)-acrylates of siloxanes containing tricyclodecane groups, according to Claims 1 to 4, characterised in that the number m of structural elements is 0 to 50.

**6.** Process for the preparation of (meth)-acrylates of siloxanes containing tricyclodecane groups, of the formula

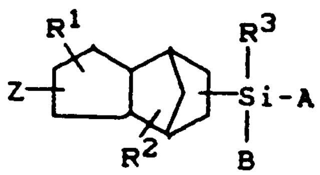

(I),

in which

$R^1$ and $R^2$ — are identical or different and denote hydrogen, straight-chain or branched $C_1$-$C_6$-alkyl, halogen or trifluoromethyl,
$R^3$ — denotes straight-chain or branched $C_1$-$C_6$-alkyl, cycloalkyl, cycloalkylalkyl or optionally substituted aryl or aralkyl,
Z — represents the

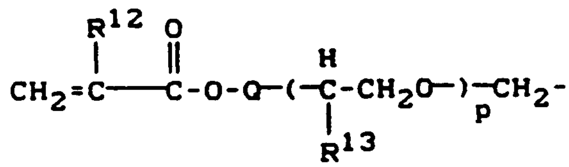

group,
in which
$R^{12}$ and $R^{13}$ — are identical or different and denote hydrogen or methyl,
p — represents a number from 0 to 20 and
Q — represents a single bond, or denotes a radical of the formula

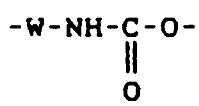

in which
W — denotes an alkylene chain, or denotes a radical of the formula

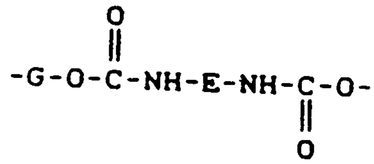

in which

E is a divalent, straight-chain or branched aliphatic radical having 2 to 24 carbon atoms, an aromatic radical having 6 to 26 carbon atoms, an araliphatic radical having 7 to 26 carbon atoms, or a cycloaliphatic radical having 6 to 26 carbon atoms, it being possible for the aliphatic, aromatic, araliphatic and/or cycloaliphatic radicals to contain 1 or 2 oxygen bridges, and it being possible for several of the aliphatic, aromatic, araliphatic and/or cycloaliphatic radicals to be connected via optionally substituted methylene groups,

and

G denotes a divalent straight-chain or branched aliphatic hydrocarbon radical, having 3 to 15 carbon atoms, which may optionally contain 1 to 3 oxygen bridges and may optionally be substituted by 1 to 4 additional (meth)acryloyloxy radicals,

A represents a siloxane chain which comprises m structural elements of the formula

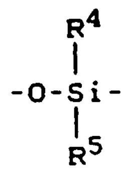

and/or

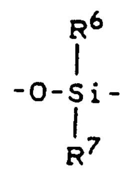

and the terminal group

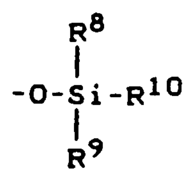

where

$R^4$, $R^5$, $R^6$, $R^7$, $R^8$ and $R^9$ are identical or different and denote straight-chain or branched $C_1$-$C_6$-alkyl, cycloalkyl, cycloalkyl-alkyl or optionally substituted aryl or aralkyl,

where the radicals $R^5$, $R^7$ and $R^8$ alternatively represent the

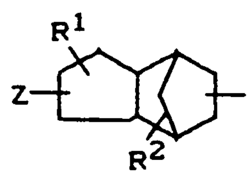

group,

in which

$R^1$, $R^2$ and Z have the abovementioned meaning,

$R^{10}$ represents straight-chain or branched $C_1$-$C_6$-alkyl, and where the total number of structural elements m, independently of one another, represents a number from 0 to 600, and

B can have the same range of meanings as A, it being possible for the radicals $R^4$ to $R^{10}$ in the chains A and B to be different, or represents straight-chain or branched $C_1$-$C_6$-alkyl,

which is characterised in that poly[hydroxy(alkyleneoxy)methyl-tricyclo[5.2.1.$0^{2.6}$]-decanyl]-siloxanes of the formula

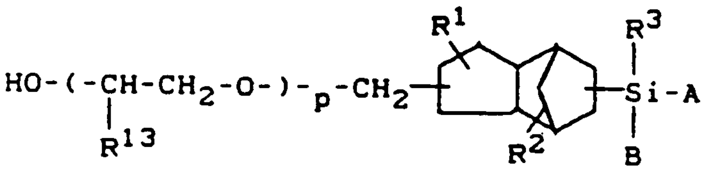

(II)

in which

$R^1$, $R^2$, $R^3$, $R^{13}$, p, A and B have the abovementioned meaning,

in the case of the preparation of compounds in which Q represents a single bond, are esterified using a (meth)acrylic acid derivative of the formula

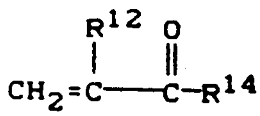

(III)

in which

$R^{12}$ has the abovementioned meaning, and

$R^{14}$ represents hydroxyl, chlorine, methoxy, ethoxy or (meth)acryloyloxy,

or

in the case of the preparation of compounds in which Q represents a radical of the formula

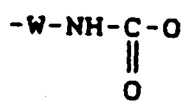

in which

W represents an alkylene chain,

are reacted with an isocyanate of the formula

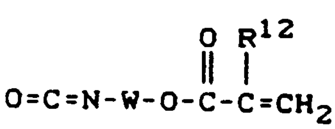

(IV)

in which

$R^{12}$ and W have the abovementioned meaning,

in an inert solvent,

or, in the case of the preparation of compounds in which Q represents a radical of the formula

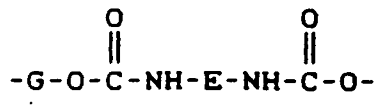

in which

E is a divalent, straight-chain or branched aliphatic radical having 2 to 24 carbon atoms, an aromatic radical having 6 to 26 carbon atoms, an araliphatic radical having 7 to 26 carbon atoms or a cycloaliphatic radical having 6 to 26 carbon atoms, it being possible for the aliphatic, aromatic, araliphatic and/or cycloaliphatic radicals to contain 1 or 2 oxygen bridges, and it being possible for several of the aliphatic, aromatic, araliphatic and/or cycloaliphatic radicals to be connected via optionally substituted methylene groups,

and

G denotes a divalent straight-chain or branched aliphatic hydrocarbon radical, having 3 to 15 carbon atoms, which may optionally contain 1 to 3 oxygen bridges and may optionally be substituted by 1 to 4 additional (meth)acryloyloxy radicals,

are reacted with the product of the addition reaction of 1 mole of a diisocyanate of the formula

O = C = N-E-N = C = O (V),

in which

E has the abovementioned meaning,

and 1 mole of a hydroxyalkyl (meth)acrylate of the formula

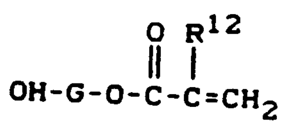

(VI)

in which

G and $R^{12}$ have the abovementioned meaning,

where a stoichiometric equivalence should exist between the NCO groups of the adduct of V and VI and the OH groups of II, in an inert solvent.

7. Process according to Claim 6, characterised in that the reaction with the (meth)acrylic acid derivative is carried out in the temperature range 50 to 120 ° C.

8. Process according to Claim 6, characterised in that the reaction with the isocyanate is carried out in the presence of a catalyst and a polymerisation inhibitor in the temperature range 20 to 100 ° C.

9. Polymers of monomer mixtures, containing (meth)-acrylates of siloxanes containing tricyclodecane groups of the formula

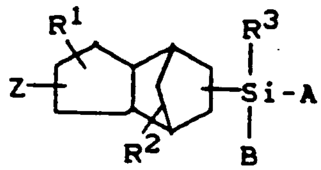

I

in which

- $R^1$ and $R^2$ — are identical or different and denote hydrogen, straight-chain or branched $C_1$-$C_6$-alkyl, halogen or trifluoromethyl,
- $R^3$ — denotes straight-chain or branched $C_1$-$C_6$-alkyl, cycloalkyl, cycloalkyl-alkyl or optionally substituted aryl or aralkyl,
- Z — represents the

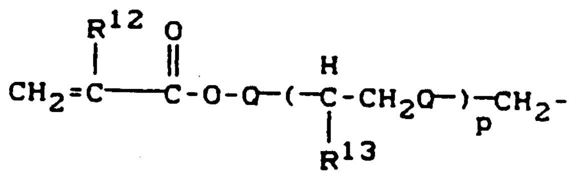

group,
in which

- $R^{12}$ and $R^{13}$ — are identical or different and denote hydrogen or methyl,
- p — represents a number from 0 to 20 and
- Q — represents a single bond, or denotes a radical of the formula

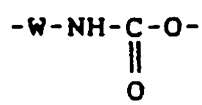

in which

- W — denotes an alkylene chain, or denotes a radical of the formula

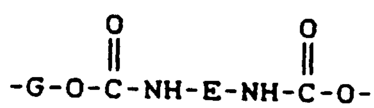

in which

- E — is a divalent, straight-chain or branched aliphatic radical having 2 to 24 carbon atoms, an aromatic radical having 6 to 26 carbon atoms, an araliphatic radical having 7 to 26 carbon atoms or a cycloaliphatic radical having 6 to 26 carbon atoms, it being possible for the aliphatic, aromatic, araliphatic and/or cycloaliphatic radicals to contain 1 or 2 oxygen bridges, and it being possible for several of the aliphatic, aromatic, araliphatic and/or cycloaliphatic radicals to be connected via optionally substituted methylene groups,

and

- G — denotes a divalent straight-chain or branched aliphatic hydrocarbon radical, having 3 to 5 carbon atoms, which may optionally contain 1 to 3 oxygen bridges and may optionally be substituted by 1 to 4

additional (meth)acryloyloxy radicals,

A — represents a siloxane chain which comprises m structural elements of the formula

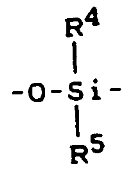

and/or

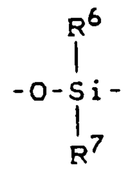

and the terminal group

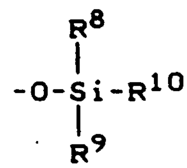

where

$R^4$, $R^5$, $R^6$, $R^7$, $R^8$ and $R^9$ — are identical or different and denote straight-chain or branched $C_1$-$C_6$-alkyl, cycloalkyl, cycloalkyl-alkyl or optionally substituted aryl or aralkyl,
where the radicals $R^5$, $R^7$ and $R^8$ alternatively represent the

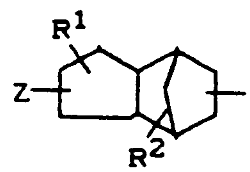

group,
in which

$R^1$, $R^2$ and Z — have the abovementioned meaning,

$R^{10}$ — represents straight-chain or branched $C_1$-$C_6$-alkyl, and
where the total number of structural elements m, independently of one another, represents a number from 0 to 600, and

B — can have the same range of meanings as A,
it being possible for the radicals $R^4$ to $R^{10}$ in the chains A and B to be different,
or represents straight-chain or branched $C_1$-$C_6$-alkyl.

**10.** Dental material containing (meth)-acrylates of siloxanes containing tricyclodecane groups, of the formula

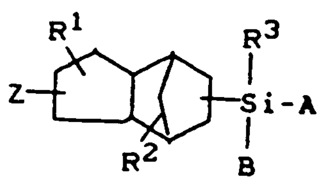

(I)

in which

$R^1$ and $R^2$ — are identical or different and denote hydrogen, straight-chain or

branched $C_1$-$C_6$-alkyl, halogen or trifluoromethyl,

$R^3$ denotes straight-chain or branched $C_1$-$C_6$-alkyl, cycloalkyl, cycloalkyl-alkyl or optionally substituted aryl or aralkyl,

Z represents the

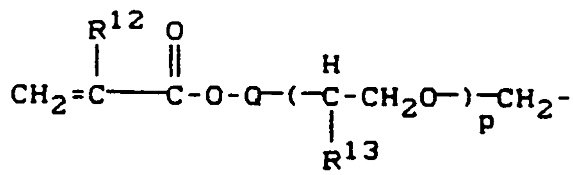

group,

in which

$R^{12}$ and $R^{13}$ are identical or different and denote hydrogen or methyl,

p represents a number from 0 to 20 and

Q represents a single bond, or denotes a radical of the formula

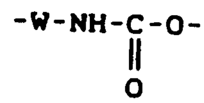

in which

W denotes an alkylene chain,

or denotes a radical of the formula

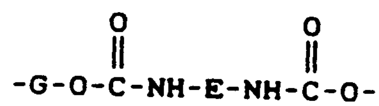

in which

E is a divalent, straight-chain or branched aliphatic radical having 2 to 24 carbon atoms, an aromatic radical having 6 to 26 carbon atoms, an araliphatic radical having 7 to 26 carbon atoms or a cycloaliphatic radical having 6 to 26 carbon atoms, it being possible for the aliphatic, aromatic, araliphatic and/or cycloaliphatic radicals to contain 1 or 2 oxygen bridges, and it being possible for several of the aliphatic, araliphatic, aromatic and/or cycloaliphatic radicals to be connected via optionally substituted methylene groups,

and

G denotes a divalent straight-chain or branched aliphatic hydrocarbon radical, having 3 to 15 carbon atoms, which may optionally contain 1 to 3 oxygen bridges and may optionally be substituted by 1 to 4 additional (meth)acryloyloxy radicals,

A represents a siloxane chain which comprises m structural elements of the formula

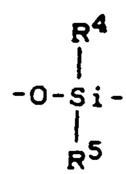

and/or

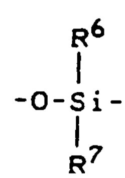

and the terminal group

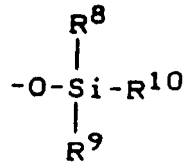

where

$R_4$, $R^5$, $R^6$, $R^7$, $R^8$ and $R^9$ are identical or different and denote straight-chain or branched $C_1$-$C_6$-alkyl, cycloalkyl, cycloalkyl-alkyl or optionally substituted aryl or aralkyl,

where the radicals $R^5$, $R^7$ and $R^8$ alternatively represent the

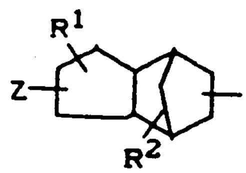

group,

in which

$R^1$, $R^2$ and Z have the abovementioned meaning,

$R^{10}$ represents straight-chain or branched $C_1$-$C_6$-alkyl, and where the total number of structural elements m, independently of one another, represents a number from 0 to 600, and

B can have the same range of meanings as A, it being possible for the radicals $R^4$ to $R^{10}$ in the chains A and B to be different, or represents straight-chain or branched $C_1$-$C_6$-alkyl.

**11.** Dental material according to Claim 10, characterised in that it contains, as monomer, 30 to 100% by weight of (meth)-acrylates of siloxanes containing tricyclodecane groups.

**12.** Dental material according to Claims 10 and 11, characterised in that it contains (meth)-acrylates of siloxanes containing tricyclodecane groups, and auxiliaries.

**13.** Use of (meth)-acrylates of siloxanes containing tricyclodecane groups, of the formula

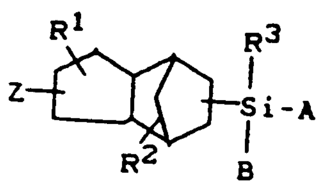

(I)

in which

$R^1$ and $R^2$ are identical or different and denote hydrogen, straight-chain or branched $C_1$-$C_6$-alkyl, halogen or trifluoromethyl,

$R^3$ denotes straight-chain or branched $C_1$-$C_6$-alkyl, cycloalkyl, cycloalkyl-alkyl or optionally substituted aryl or aralkyl,

Z represents the

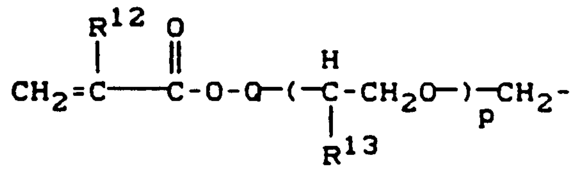

group,
in which

$R^{12}$ and $R^{13}$ are identical or different and denote hydrogen or methyl,

p represents a number from 0 to 20 and

Q represents a single bond, or denotes a radical of the formula

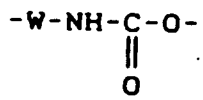

in which

W denotes an alkylene chain,
or denotes a radical of the formula

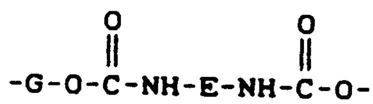

in which

E is a divalent, straight-chain or branched aliphatic radical having 2 to 24 carbon atoms, an aromatic radical having 6 to 26 carbon atoms, an araliphatic radical having 7 to 26 carbon atoms or a cycloaliphatic radical having 6 to 26 carbon atoms, it being possible for the aliphatic, aromatic, araliphatic and/or cycloaliphatic radicals to contain 1 or 2 oxygen bridges, and it being possible for several of the aliphatic, araliphatic, aromatic and/or cycloaliphatic radicals to be connected via optionally substituted methylene groups,
and

G denotes a divalent straight-chain or branched aliphatic hydrocarbon radical, having 3 to 15 carbon atoms, which may optionally contain 1 to 3 oxygen bridges and may optionally be substituted by 1 to 4 additional (meth)acryloyloxy radicals,

A represents a siloxane chain which comprises m structural elements of the formula

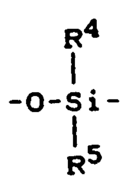

and/or

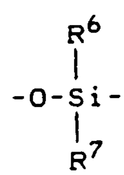

and the terminal group

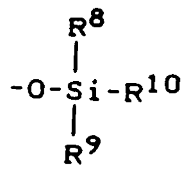

where

$R^4$, $R^5$, $R^6$, $R^7$, $R^8$ and $R^9$ are identical or different and denote straight-chain or branched $C_1$-$C_6$-alkyl, cycloalkyl, cycloalkyl-alkyl or optionally substituted aryl or aralkyl,

where the radicals $R^5$, $R^7$ and $R^8$ alternatively represent the

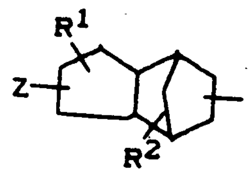

group,

in which

$R^1$, $R^2$ and Z have the abovementioned meaning,

$R^{10}$ represents straight-chain or branched $C_1$-$C_6$-alkyl, and

where the total number of structural elements m, independently of one another, represents a number from 0 to 600, and

B can have the same range of meanings as A,

it being possible for the radicals $R^4$ to $R^{10}$ in the chains A and B to be different,

or represents straight-chain or branched $C_1$-$C_6$-alkyl,

in dental materials.

**14.** Use according to Claim 13 in filling materials for teeth.

**15.** Use according to Claim 13 in coating agents for teeth.

**16.** Use according to Claim 13 as components in the production of tooth replacements.

**17.** Use according to Claim 13 as components for the production of moulded dental elements.

**Claims for the following Contracting State : ES**

**1.** Process for the preparation of (meth)-acrylates of siloxanes containing tricyclodecane groups, of the formula

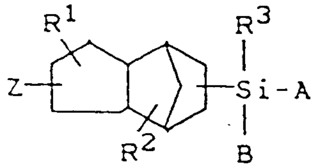

(I)

in which

$R^1$ and $R^2$ are identical or different and denote hydrogen, straight-chain or branched $C_1$-$C_6$-alkyl, halogen or trifluoromethyl,

$R^3$ denotes straight-chain or branched $C_1$-$C_6$-alkyl, cycloalkyl, cycloalkylalkyl or optionally substituted aryl or aralkyl,

Z represents the

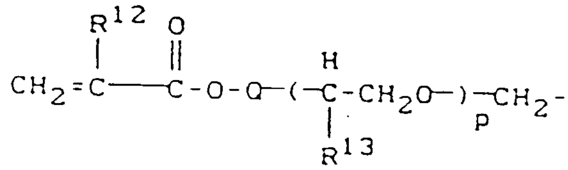

group,

in which

$R^{12}$ and $R^{13}$ are identical or different and denote hydrogen or methyl,

p represents a number from 0 to 20 and

Q represents a single bond, or denotes a radical of the formula

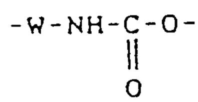

in which

W denotes an alkylene chain,

or denotes a radical of the formula

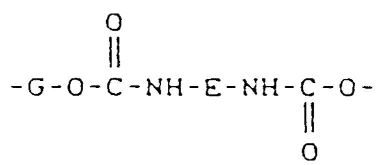

in which

E is a divalent, straight-chain or branched aliphatic radical having 2 to 24 carbon atoms, an aromatic radical having 6 to 26 carbon atoms, an araliphatic radical having 7 to 26 carbon atoms, or a cycloaliphatic radical having 6 to 26 carbon atoms, it being possible for the aliphatic, aromatic, araliphatic and/or cycloaliphatic radicals to contain 1 or 2 oxygen bridges, and it being possible for several of the aliphatic, aromatic, araliphatic and/or cycloaliphatic radicals to be connected via optionally substituted methylene groups,

and

G denotes a divalent straight-chain or branched aliphatic hydrocarbon radical, having 3 to 15 carbon atoms, which may optionally contain 1 to 3 oxygen bridges and may optionally be substituted by 1 to 4 additional (meth)acryloyloxy radicals,

A represents a siloxane chain which comprises m structural elements of the formula

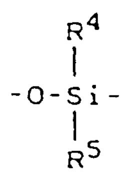

and/or

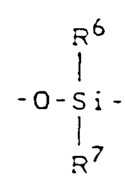

and the terminal group

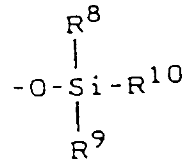

where

$R^4$, $R^5$, $R^6$, $R^7$, $R^8$ and $R^9$ are identical or different and denote straight-chain or branched $C_1$-$C_6$-alkyl, cycloalkyl, cycloalkyl-alkyl or optionally substituted aryl or aralkyl,

where the radicals $R^5$, $R^7$ and $R^8$ alternatively represent the

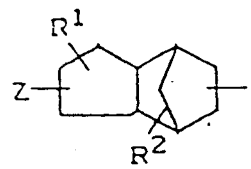

group,

in which

$R^1$, $R^2$ and Z have the abovementioned meaning,

$R^{10}$ represents straight-chain or branched $C_1$-$C_6$-alkyl, and

where the total number of structural elements m, independently of one another, represents a number from 0 to 600, and

B can have the same range of meanings as A,

it being possible for the radicals $R^4$ to $R^{10}$ in the chains A and B to be different,

or represents straight-chain or branched $C_1$-$C_6$-alkyl,

which is characterised in that poly[hydroxy(alkyleneoxy)methyl-tricyclo[5.2.1.$0^{2.6}$]-decanyl]-siloxanes of the formula

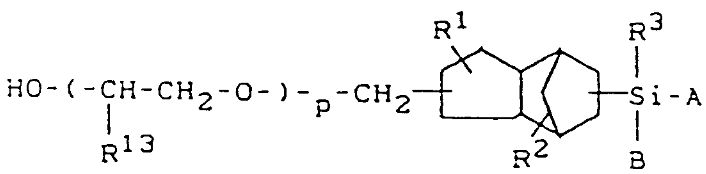

(II)

in which

$R^1$, $R^2$, $R^3$, $R^{13}$, p, A and B have the abovementioned meaning,

in the case of the preparation of compounds in which Q represents a single bond, are esterified using a (meth)acrylic acid derivative of the formula

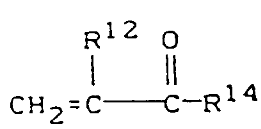

(III)

in which

$R^{12}$ has the abovementioned meaning, and

$R^{14}$ represents hydroxyl, chlorine, methoxy, ethoxy or (meth)acryloyloxy, or in the case of the preparation of compounds in which Q represents a radical of the formula

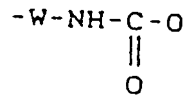

in which

W represents an alkylene chain,

are reacted with an isocyanate of the formula

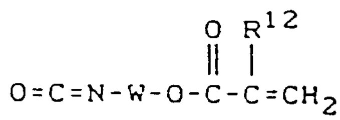

(IV)

in which

$R^{12}$ and W have the abovementioned meaning,

in an inert solvent,

or, in the case of the preparation of compounds in which Q represents a radical of the formula

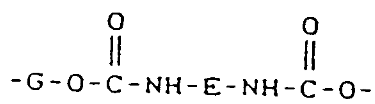

in which

E is a divalent, straight-chain or branched aliphatic radical having 2 to 24 carbon atoms, an aromatic radical having 6 to 26 carbon atoms, an araliphatic radical having 7 to 26 carbon atoms or a cycloaliphatic radical having 6 to 26 carbon atoms, it being possible for the aliphatic, aromatic, araliphatic and/or cycloaliphatic radicals to contain 1 or 2 oxygen bridges, and it being possible for several of the aliphatic, aromatic, araliphatic and/or cycloaliphatic radicals to be connected via optionally substituted methylene groups,

and

G denotes a divalent straight-chain or branched aliphatic hydrocarbon radical, having 3 to 15 carbon atoms, which may optionally contain 1 to 3 oxygen bridges and may optionally be substituted by 1 to 4 additional (meth)acryloyloxy radicals,

are reacted with the product of the addition reaction of 1 mole of a diisocyanate of the formula

O = C = N-E-N = C = O (V),

in which

E has the abovementioned meaning,

and 1 mole of a hydroxyalkyl (meth)acrylate of the formula

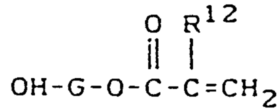 (VI)

in which

G and $R^{12}$ have the abovementioned meaning,

where a stoichiometric equivalence should exist between the NCO groups of the adduct of V and VI and the OH groups of II, in an inert solvent.

2. Process according to Claim 1, characterised in that the reaction with the (meth)acrylic acid derivative is carried out in the temperature range 50 to 120 °C.

**Revendications**
**Revendications pour les Etats contractants suivants : AT, BE, CH, DE, FR, GB, IT, LI, NL, SE**

1. Esters (méth)-acryliques de siloxanes contenant des groupes tricyclodécane, répondant à la formule :

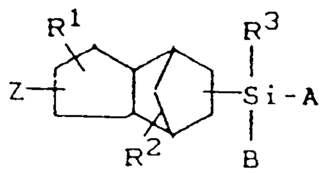 (I),

dans laquelle

$R^1$ et $R^2$ sont identiques ou différents et représentent un atome d'hydrogène, un groupe alkyle inférieur, un atome d'halogène ou un groupe trifluorométhyle,

$R^3$ représente un groupe alkyle en $C_1$-$C_6$ à chaîne droite ou ramifiée, un groupe cycloalkyle, un groupe cycloalkylalkyle, ou encore un groupe aryle ou un groupe aralkyle éventuellement substitué,

Z représente le groupe

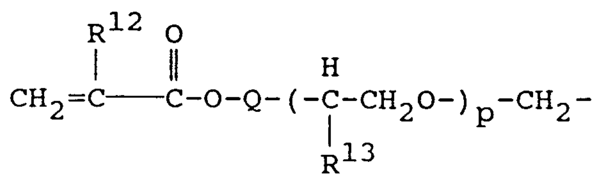

dans lequel

$R^{12}$ et $R^{13}$ sont identiques ou différents et représentent un atome d'hydrogène ou un groupe méthyle,

p représente un nombre de 0 à 20 et

Q représente une liaison simple ou un radical de formule

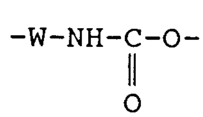

dans laquelle

W représente une chaîne alkylène ou bien représente un radical de formule

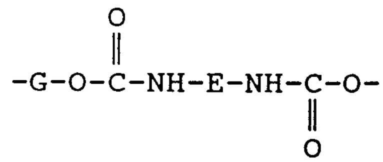

dans laquelle

E représente un radical aliphatique bivalent à chaîne droite ou ramifiée contenant de 2 à 24 atomes de carbone, un radical aromatique contenant de 6 à 26 atomes de carbone, un radical araliphatique contenant de 7 à 26 atomes de carbone ou un radical cycloaliphatique contenant de 6 à 26 atomes de carbone, les radicaux aliphatiques, aromatiques, araliphatiques et/ou cycloaliphatiques pouvant contenir 1 ou 2 ponts d'oxygène, et plusieurs des radicaux aliphatiques, aromatiques, araliphatiques et/ou cycloaliphatiques pouvant être reliés à l'intervention de groupes méthylène éventuellement substitués,

et

G représente un radical d'hydrocarbure aliphatique bivalent à chaîne droite ou ramifiée contenant de 3 à 15 atomes de carbone, qui peut éventuellement contenir de 1 à 3 ponts d'oxygène et qui peut être éventuellement substitué par 1 à 4 radicaux (méth)-acryloyloxy supplémentaires,

A représente une chaîne siloxane qui est constituée par m éléments de structure de formules

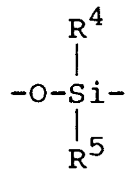

et/ou

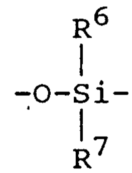

et par le groupe terminal

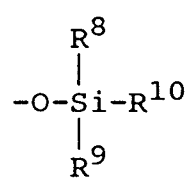

dans lesquels

$R^4$, $R^5$, $R^6$, $R^7$, $R^8$ et $R^9$ sont identiques ou différents et représentent un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué,

les radicaux $R^5$, $R^7$ et $R^8$ représentent également le groupe

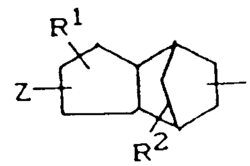

| | |
|---|---|
| | dans lequel |
| $R^1$, $R^2$ et Z | ont la signification mentionnée ci-dessus, |
| $R^{10}$ | représente un groupe alkyle en $C_1$-$C_6$ à chaîne droite ou ramifiée et la somme des éléments de structure m représentant, de manière mutuellement indépendante, un nombre de 0 à 600, et |
| B | peut avoir le domaine de signification analogue à celui de A, les radicaux $R^4$ à $R^{10}$ dans les chaînes A et B pouvant être différents, ou représente un groupe alkyle en $C_1$-$C_6$ à chaîne droite ou ramifiée. |

**2.** Esters (méth)-acryliques de siloxanes contenant des groupes tricyclodécane, selon la revendication 1, dans lesquels

| | |
|---|---|
| $R^1$ et $R^2$ | sont identiques ou différents et représentent un atome d'hydrogène, un groupe alkyle en $C_1$-$C_6$ à chaîne droite ou ramifiée, un atome d'halogène ou un groupe trifluorométhyle, |
| $R^3$ | représente un groupe alkyle en $C_1$-$C_6$ à chaîne droite ou ramifiée, un groupe cycloalkyle en $C_5$-$C_7$, un groupe cycloalkyl-alkyle en $C_6$-$C_{13}$, ou encore un groupe aryle en $C_6$-$C_{12}$ ou un groupe aralkyle en $C_7$-$C_{18}$ éventuellement substitué, |
| Z | représente le groupe |

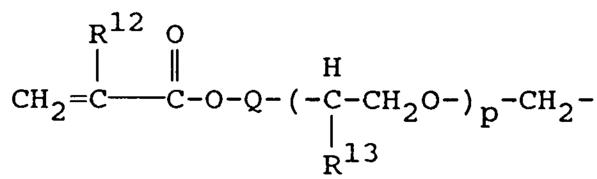

| | |
|---|---|
| | dans lequel |
| $R^{12}$ et $R^{13}$ | sont identiques ou différents et représentent un atome d'hydrogène ou un groupe méthyle, |
| p | représente un nombre de 0 à 20 et |
| Q | représente une liaison simple ou un radical de formule |

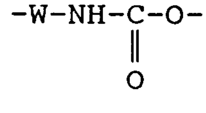

| | |
|---|---|
| | dans laquelle |
| W | représente une chaîne alkylène contenant de 2 à 10 atomes de carbone, ou bien représente un radical de formule |

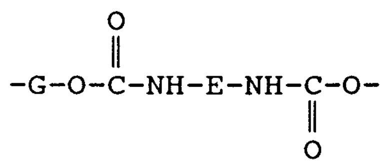

dans laquelle

E représente un radical aliphatique bivalent à chaîne droite ou ramifiée contenant de 2 à 12 atomes de carbone, un radical aromatique contenant de 6 à 18 atomes de carbone ou un radical cycloaliphatique contenant de 6 à 14 atomes de carbone, les radicaux aliphatiques, aromatiques et/ou cycloaliphatiques pouvant contenir 1 ou 2 ponts d'oxygène et plusieurs des radicaux aliphatiques, aromatiques et/ou cycloaliphatiques pouvant être reliés à l'intervention de groupes méthylène éventuellement substitués,

et

G représente un radical d'hydrocarbure aliphatique bivalent à chaîne droite ou ramifiée contenant de 3 à 10 atomes de carbone, qui peut éventuellement contenir de 1 à 3 ponts d'oxygène et qui peut être éventuellement substitué par 1 ou 2 radicaux (méth)-acryloyloxy supplémentaires,

A représente une chaîne siloxane qui est constituée par m éléments de structure de formules

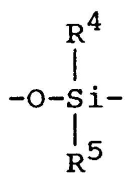

et/ou

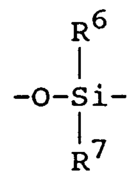

et par le groupe terminal

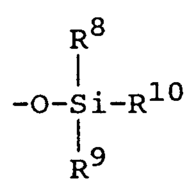

dans lesquels

$R^4$, $R^5$, $R^6$, $R^7$, $R^8$ et $R^9$ sont identiques ou différents et représentent un groupe alkyle en $C_1$-$C_6$ à chaîne droite ou ramifiée, un groupe cycloalkyle en $C_5$-$C_7$, un groupe cycloalkyl-alkyle en $C_6$-$C_{13}$, ou encore un groupe aryle en $C_6$-$C_{12}$ ou un groupe aralkyle en $C_7$-$C_{18}$ éventuellement substitué,

les radicaux $R^5$, $R^7$ et $R^8$ représentant également le groupe

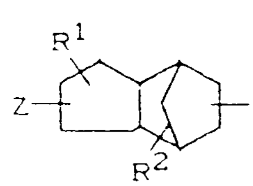

dans lequel

$R^1$, $R^2$ et Z — ont la signification mentionnée ci-dessus,

$R^{10}$ — représente un groupe alkyle en $C_1$-$C_6$ à chaîne droite ou ramifiée et la somme des éléments de structure m représentant, de manière mutuellement indépendante, un nombre de 0 à 200, et

B — peut avoir le domaine de signification analogue à celui de A, les radicaux $R^4$ à $R^{10}$ dans les chaînes A et B pouvant être différents, ou représente un groupe alkyle en $C_1$-$C_6$ à chaîne droite ou ramifiée.

**3.** Esters (méth)-acryliques de siloxanes contenant des groupes tricyclodécane, selon les revendications 1 et 2, dans lesquels

$R^1$ et $R^2$ — sont identiques ou différents et représentent un atome d'hydrogène ou un groupe alkyle en $C_1$-$C_6$ à chaîne droite ou ramifiée,

$R^3$ — représente un groupe alkyle en $C_1$-$C_6$ à chaîne droite ou ramifiée, un groupe cycloalkyle en $C_5$-$C_7$, un groupe cycloalkyl-alkyle en $C_6$-$C_{10}$, ou encore un groupe aryle en $C_6$-$C_{11}$ ou un groupe aralkyle en $C_7$-$C_{11}$ éventuellement substitué,

Z — représente le groupe

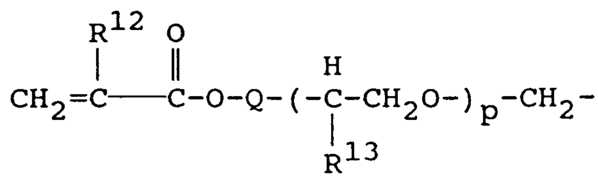

dans lequel

$R^{12}$ et $R^{13}$ — sont identiques ou différents et représentent un atome d'hydrogène ou un groupe méthyle,

p — représente un nombre de 0 à 4 et

Q — représente une liaison simple ou un radical de formule

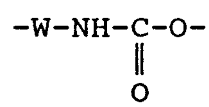

dans laquelle

W — représente une chaîne alkylène contenant de 2 à 6 atomes de carbone, ou bien représente un radical de formule

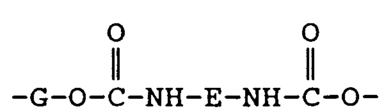

dans laquelle

E — représente un radical aliphatique bivalent à chaîne droite ou ramifiée contenant de 2 à 8 atomes de carbone ou un radical cycloaliphatique contenant de 6 à 14 atomes de carbone,

et

G — représente un radical d'hydrocarbure aliphatique bivalent à chaîne droite ou ramifiée contenant de 3 à 10 atomes de carbone, qui peut éventuellement contenir 1 pont d'oxygène et qui peut être éventuellement substitué par 1 ou 2 radicaux (méth)-acryloyloxy supplémentaires,

A représente une chaîne siloxane qui est constituée par m éléments de structure de formules

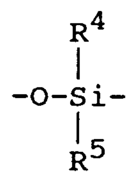

et/ou

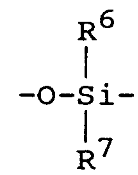

et par le groupe terminal

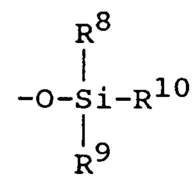

dans lesquelles

$R^4$, $R^5$, $R^6$, $R^7$, $R^8$ et $R^9$ sont identiques ou différents et représentent un groupe alkyle en $C_1$-$C_6$ à chaîne droite ou ramifiée, un groupe cycloalkyle en $C_5$-$C_7$, un groupe cycloalkyl-alkyle en $C_6$-$C_{10}$, ou encore un groupe aryle en $C_6$-$C_{11}$ ou un groupe aralkyle en $C_7$-$C_{11}$ éventuellement substitué,
les radicaux $R^5$, $R^7$ et $R^8$ représentant également le groupe

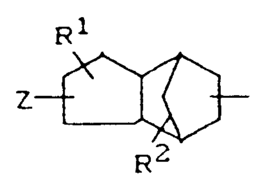

dans lequel

$R^1$, $R^2$ et Z ont la signification mentionnée ci-dessus,

$R^{10}$ représente un groupe alkyle en $C_1$-$C_6$ à chaîne droite ou ramifiée et la somme des éléments de structure m représentant, de manière mutuellement indépendante, un nombre de 0 à 200, et

B peut avoir le domaine de signification analogue à celui de A, les radicaux $R^4$ à $R^{10}$ dans les chaînes A et B pouvant être différents, ou représente un groupe alkyle en $C_1$-$C_6$ à chaîne droite ou ramifiée.

**4.** Esters (méth)-acryliques de siloxanes contenant des groupes tricyclodécane, selon les revendications 1 à 3, caractérisés en ce que de 0,5 à 100 moles % de tous les atomes de silicium sont substitués par des groupes tricyclo[5.2.1.$0^{2,6}$]décanyle.

**5.** Esters (méth)-acryliques de siloxanes contenant des groupes tricyclodécane, selon les revendications 1 à 4, caractérisés en ce que le nombre m des éléments de structure s'élève de 0 à 50.

**6.** Procédé pour la préparation d'esters (méth)-acryliques de siloxanes contenant des groupes tricyclodécane, répondant à la formule :

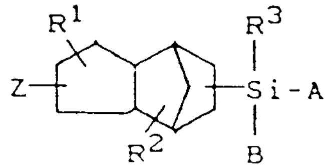 (I),

dans laquelle

$R^1$ et $R^2$ sont identiques ou différents et représentent un atome d'hydrogène, un groupe alkyle en $C_1$-$C_6$ à chaîne droite ou ramifiée, un atome d'halogène ou un groupe trifluorométhyle,

$R^3$ représente un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué,

Z représente le groupe

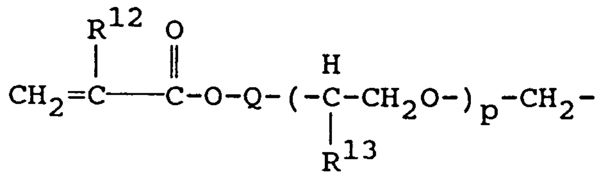

dans lequel

$R^{12}$ et $R^{13}$ sont identiques ou différents et représentent un atome d'hydrogène ou un groupe méthyle,

p représente un nombre de 0 à 20 et

Q représente une liaison simple ou un radical de formule

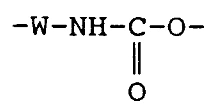

dans laquelle

W représente une chaîne alkylène ou bien représente un radical de formule

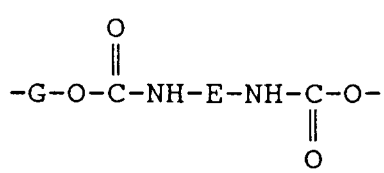

dans laquelle

E représente un radical aliphatique bivalent à chaîne droite ou ramifiée contenant de 2 à 24 atomes de carbone, un radical aromatique contenant de 6 à 26 atomes de carbone, un radical araliphatique contenant de 7 à 26 atomes de carbone ou un radical cycloaliphatique contenant de 6 à 26 atomes de carbone, les radicaux aliphatiques, aromatiques, araliphatiques et/ou cycloaliphatiques pouvant contenir 1 ou 2 ponts d'oxygène, et plusieurs des radicaux aliphatiques, aromatiques, araliphatiques et/ou cycloaliphatiques pouvant être reliés à l'intervention de

groupes méthylène éventuellement substitués, et

G représente un radical d'hydrocarbure aliphatique bivalent à chaîne droite ou ramifiée contenant de 3 à 15 atomes de carbone, qui peut éventuellement contenir de 1 à 3 ponts d'oxygène et qui peut être éventuellement substitué par 1 à 4 radicaux (méth)-acryloyloxy supplémentaires,

A représente une chaîne siloxane qui est constituée par m éléments de structure de formules

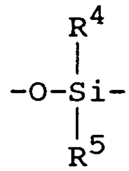

et/ou

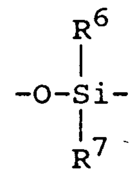

et par le groupe terminal

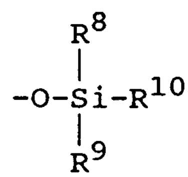

dans lesquels

$R^4$, $R^5$, $R^6$, $R^7$, $R^8$ et $R^9$ sont identiques ou différents et représentent un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué,
les radicaux $R^5$, $R^7$ et $R^8$ représentant également le groupe

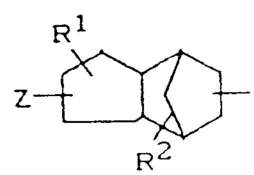

dans lequel

$R^1$, $R^2$ et Z ont la signification mentionnée ci-dessus,

$R^{10}$ représente un groupe alkyle en $C_1$-$C_6$ à chaîne droite ou ramifiée et la somme des éléments de structure m représentant, de manière mutuellement indépendante, un nombre de 0 à 600, et

B peut avoir le domaine de signification analogue à celui de A, les radicaux $R^4$ à $R^{10}$ dans les chaînes A et B pouvant être différents, ou représente un groupe alkyle en $C_1$-$C_6$ à chaîne droite ou ramifiée,

qui se caractérise par le fait qu'on estérifie des poly[hydroxy-(alkylènoxy)méthyl-tricyclo-[5.2.1.$0^{2,6}$]-décanyl]-siloxanes répondant à la formule

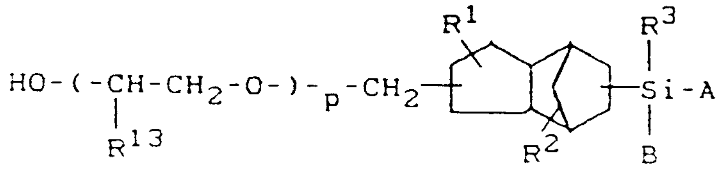 (II),

dans laquelle

$R^1$, $R^2$, $R^3$, $R^{13}$, p, A et B ont la signification mentionnée ci-dessus,

dans le cas d'une préparation de composés dans lesquels Q représente une liaison simple, avec un dérivé de l'acide (méth)-acrylique de formule

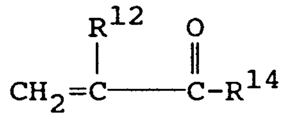 (III)

dans laquelle

$R^{12}$ a la signification mentionnée ci-dessus et $R^{14}$ représente un groupe hydroxyle, un atome de chlore, un groupe méthoxy, un groupe éthoxy ou un groupe (méth)-acryloyloxy,

ou bien

dans le cas d'une préparation de composés dans lesquels Q représente un radical de formule

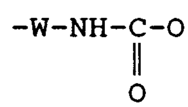

dans laquelle

W représente une chaîne alkylène,

on les fait réagir avec un isocyanate de formule

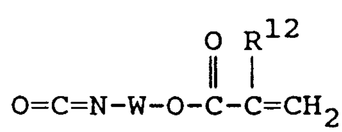 (IV)

dans laquelle

$R^{12}$ et W ont la signification mentionnée ci-dessus,

dans un solvant inerte,

ou bien

dans le cas d'une préparation de composés dans lesquels Q représente un radical de formule

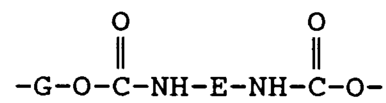

dans laquelle

E représente un radical aliphatique bivalent à chaîne droite ou ramifiée contenant de 2 à 24 atomes de carbone, un radical aromatique contenant de 6 à 26 atomes de carbone, un radical araliphatique contenant de 7 à 26 atomes de carbone ou un radical cycloaliphatique contenant de 6 à 26 atomes de carbone, les radicaux aliphatiques, aromatiques, araliphatiques et/ou cycloaliphatiques pouvant contenir 1 ou 2 ponts d'oxygène, et plusieurs des radicaux aliphatiques, aromatiques, araliphatiques et/ou cycloaliphatiques pouvant être reliés à l'intervention de groupes méthylène éventuellement substitués,

et

G représente un radical d'hydrocarbure aliphatique bivalent à chaîne droite ou ramifiée contenant de 3 à 15 atomes de carbone, qui peut éventuellement contenir de 1 à 3 ponts d'oxygène et qui peut être éventuellement substitué par 1 à 4 radicaux (méth)-acryloyloxy supplémentaires,

on les fait réagir avec le produit d'addition de 1 mole d'un diisocyanate de formule

$$O=C=N\text{-}E\text{-}N=C=O \qquad (V),$$

dans laquelle

E a la signification mentionnée ci-dessus,

et de 1 mole d'un ester hydroxyalkyl(méth)-acrylique de formule

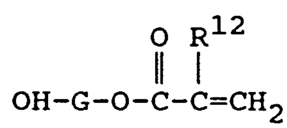

(VI),

dans laquelle

G et $R^{12}$ ont la signification mentionnée ci-dessus,

dans un solvant inerte,

une équivalence stoechiométrique entre les groupes NCO de l'adduit de V et de VI et les groupes OH de II devant exister.

**7.** Procédé selon la revendication 6, caractérisé en ce qu'on effectue la mise en réaction avec le dérivé de l'acide (méth)-acrylique dans un domaine de température de 50 à 120 ° C.

**8.** Procédé selon la revendication 6, caractérisé en ce qu'on effectue la mise en réaction avec l'isocyanate en présence d'un catalyseur et d'un inhibiteur de polymérisation dans un domaine de température de 20 à 100 ° C.

**9.** Polymères de mélanges de monomères contenant des esters (méth)-acryliques de siloxanes contenant des groupes tricyclodécane, répondant à la formule :

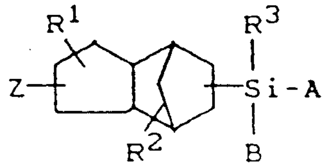 (I),

dans laquelle

$R^1$ et $R^2$ sont identiques ou différents et représentent un atome d'hydrogène, un groupe alkyle en $C_1$-$C_6$ à chaîne droite ou ramifiée, un atome d'halogène ou un groupe trifluorométhyle,

$R^3$ représente un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué,

Z représente le groupe

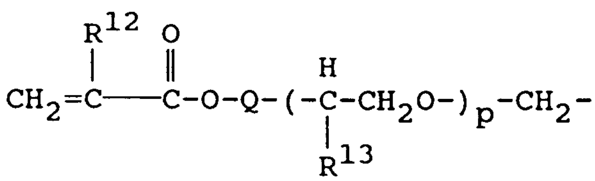

dans lequel

$R^{12}$ et $R^{13}$ sont identiques ou différents et représentent un atome d'hydrogène ou un groupe méthyle,

p représente un nombre de 0 à 20 et

Q représente une liaison simple ou un radical de formule

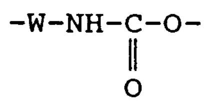

dans laquelle

W représente une chaîne alkylène ou bien représente un radical de formule

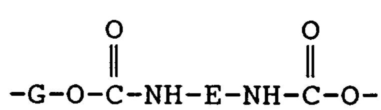

dans laquelle

E représente un radical aliphatique bivalent à chaîne droite ou ramifiée contenant de 2 à 24 atomes de carbone, un radical aromatique contenant de 6 à 26 atomes de carbone, un radical araliphatique contenant de 7 à 26 atomes de carbone ou un radical cycloaliphatique contenant de 6 à 26 atomes de carbone, les radicaux aliphatiques, aromatiques, araliphatiques et/ou cycloaliphatiques pouvant contenir 1 ou 2 ponts d'oxygène, et plusieurs des radicaux aliphatiques, aromatiques, araliphatiques et/ou cycloaliphatiques pouvant être reliés à l'intervention de groupes méthylène éventuellement substitués,

et

G représente un radical d'hydrocarbure aliphatique bivalent à chaîne droite ou ramifiée contenant de 3 à 15 atomes de carbone, qui peut éventuellement contenir de 1 à 3 ponts d'oxygène et qui peut être éventuellement substitué par 1 à 4 radicaux (méth)-acryloyloxy supplémentaires,

A représente une chaîne siloxane qui est constituée par m éléments de structure de formules

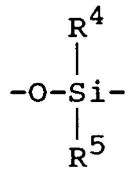

et/ou

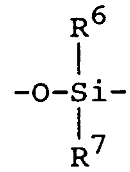

et par le groupe terminal

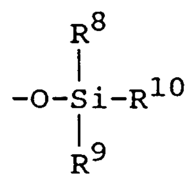

dans lesquels

$R^4$, $R^5$, $R^6$, $R^7$, $R^8$ et $R^9$ sont identiques ou différents et représentent un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué,
les radicaux $R^5$, $R^7$ et $R^8$ représentant également le groupe

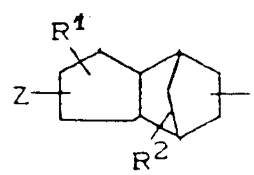

dans lequel

$R^1$, $R^2$ et Z ont la signification mentionnée ci-dessus,

$R^{10}$ représente un groupe alkyle en $C_1$-$C_6$ à chaîne droite ou ramifiée et la somme des éléments de structure m représentant, de manière mutuellement indépendante, un nombre de 0 à 600, et

B peut avoir le domaine de signification analogue à celui de A, les radicaux $R^4$ à $R^{10}$ dans les chaînes A et B pouvant être différents, ou représente un groupe alkyle en $C_1$-$C_6$ à chaîne droite ou ramifiée.

**10.** Matière dentaire contenant des esters (méth)-acryliques de siloxanes contenant des groupes tricyclodécane, répondant à la formule :

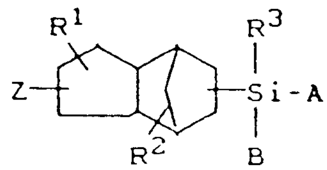 (I),

dans laquelle

$R^1$ et $R^2$ sont identiques ou différents et représentent un atome d'hydrogène, un groupe alkyle en $C_1$-$C_6$ à chaîne droite ou ramifiée, un atome d'halogène ou un groupe trifluorométhyle,

$R^3$ représente un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué,

Z représente le groupe

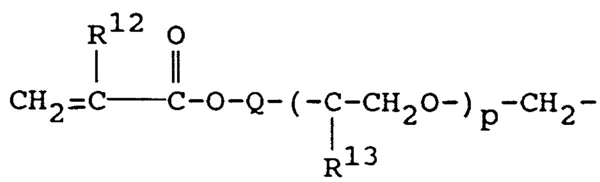

dans lequel

$R^{12}$ et $R^{13}$ sont identiques ou différents et représentent un atome d'hydrogène ou un groupe méthyle,

p représente un nombre de 0 à 20 et

Q représente une liaison simple ou un radical de formule

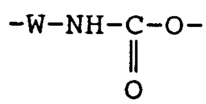

dans laquelle

W représente une chaîne alkylène ou bien représente un radical de formule

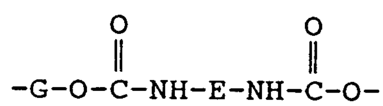

dans laquelle

E représente un radical aliphatique bivalent à chaîne droite ou ramifiée contenant de 2 à 24 atomes de carbone, un radical aromatique contenant de 6 à 26 atomes de carbone, un radical araliphatique contenant de 7 à 26 atomes de carbone ou un radical cycloaliphatique contenant de 6 à 26 atomes de carbone, les radicaux aliphatiques, aromatiques, araliphatiques et/ou cycloaliphatiques pouvant contenir 1 ou 2 ponts d'oxygène, et plusieurs des radicaux aliphatiques, aromatiques, araliphatiques et/ou cycloaliphatiques pouvant être reliés à l'intervention de groupes méthylène éventuellement substitués,

et

G représente un radical d'hydrocarbure aliphatique bivalent à chaîne droite ou ramifiée contenant de 3 à 15 atomes de carbone, qui peut éventuellement contenir de 1 à 3 ponts d'oxygène et qui peut être éventuellement substitué par 1 à 4 radicaux (méth)-acryloyloxy supplémentaires,

A représente une chaîne siloxane qui est constituée par m éléments de structure de formules

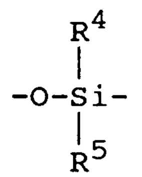

et/ou

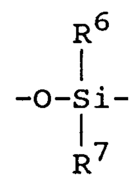

et par le groupe terminal

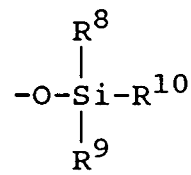

dans lesquels

$R^4$, $R^5$, $R^6$, $R^7$, $R^8$ et $R^9$ sont identiques ou différents et représentent un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué,

les radicaux $R^5$, $R^7$ et $R^8$ représentant également le groupe

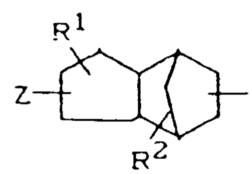

dans lequel

$R^1$, $R^2$ et Z ont la signification mentionnée ci-dessus,

$R^{10}$ représente un groupe alkyle en $C_1$-$C_6$ à chaîne droite ou ramifiée et

la somme des éléments de structure m représentant, de manière mutuellement indépendante, un nombre de 0 à 600, et

B peut avoir le domaine de signification analogue à celui de A,

les radicaux $R^4$ à $R^{10}$ dans les chaînes A et B pouvant être différents,

ou représente un groupe alkyle en $C_1$-$C_6$ à chaîne droite ou ramifiée.

**11.** Matière dentaire selon la revendication 10, caractérisée en ce qu'elle contient, comme monomère, de 30 à 100 % en poids d'esters (méth)-acryliques de siloxanes contenant des groupes tricyclodécane.

**12.** Matière dentaire selon les revendications 10 et 11, caractérisée en ce qu'elle contient des esters (méth)-acryliques de siloxanes contenant des groupes tricyclodécane, ainsi que des substances auxiliaires.

**13.** Utilisation d'esters (méth)-acryliques de siloxanes contenant des groupes tricyclodécane, répondant à la formule

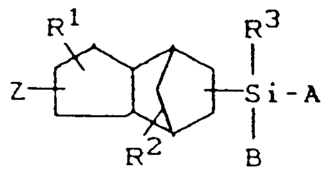

(I),

dans laquelle

$R^1$ et $R^2$ sont identiques ou différents et représentent un atome d'hydrogène, un groupe alkyle en $C_1$-$C_6$ à chaîne droite ou ramifiée, un atome d'halogène ou un groupe trifluorométhyle,

$R^3$ représente un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué,

Z représente le groupe

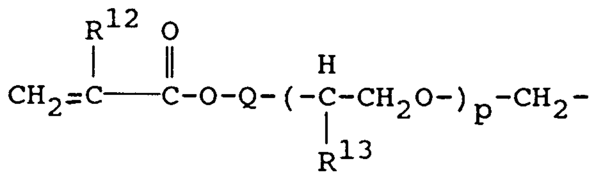

dans lequel

$R^{12}$ et $R^{13}$ sont identiques ou différents et représentent un atome d'hydrogène ou un groupe méthyle,

p représente un nombre de 0 à 20 et

Q représente une liaison simple ou un radical de formule

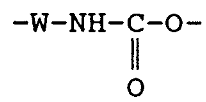

dans laquelle

W représente une chaîne alkylène ou bien représente un radical de formule

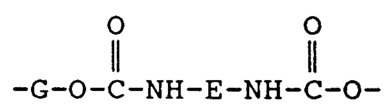

dans laquelle

E représente un radical aliphatique bivalent à chaîne droite ou ramifiée contenant de 2 à 24 atomes de carbone, un radical aromatique contenant de 6 à 26 atomes de carbone, un radical araliphatique contenant de 7 à 26 atomes de carbone ou un radical cycloaliphatique contenant de 6 à 26 atomes de carbone, les radicaux aliphatiques, aromatiques, araliphatiques et/ou cycloaliphatiques pouvant contenir 1 ou 2 ponts d'oxygène, et plusieurs des radicaux aliphatiques, aromatiques, araliphatiques et/ou cycloaliphatiques pouvant être reliés à l'intervention de groupes méthylène éventuellement substitués,

et

G représente un radical d'hydrocarbure aliphatique bivalent à chaîne droite ou ramifiée contenant de 3 à 15 atomes de carbone, qui peut éventuellement contenir de 1 à 3 ponts d'oxygène et qui peut être éventuellement substitué par 1 à 4 radicaux (méth)-acryloyloxy supplémentaires,

A représente une chaîne siloxane qui est constituée par m éléments de structure de formules

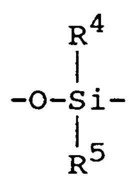

et/ou

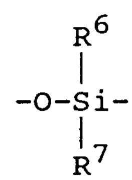

et par le groupe terminal

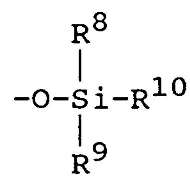

dans lesquels

$R^4$, $R^5$, $R^6$, $R^7$, $R^8$ et $R^9$ sont identiques ou différents et représentent un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué,

les radicaux $R^5$, $R^7$ et $R^8$ représentant également le groupe

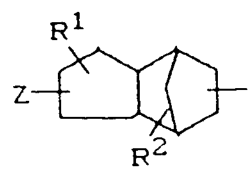

dans lequel

$R^1$, $R^2$ et Z ont la signification mentionnée ci-dessus,

$R^{10}$ représente un groupe alkyle en $C_1$-$C_6$ à chaîne droite ou ramifiée et la somme des éléments de structure m représentant, de manière mutuellement indépendante, un nombre de 0 à 600, et

B peut avoir le domaine de signification analogue à celui de A, les radicaux $R^4$ à $R^{10}$ dans les chaînes A et B pouvant être différents, ou représente un groupe alkyle en $C_1$-$C_6$ à chaîne droite ou ramifiée,

dans des matières dentaires.

**14.** Utilisation selon la revendication 13 dans des matières d'obturation dentaire.

**15.** Utilisation selon la revendication 13 dans des agents d'enduction pour les dents.

**16.** Utilisation selon la revendication 13 comme composant lors de la préparation de prothèses dentaires.

**17.** Utilisation selon la revendication 13 comme composant pour la préparation de corps moulés pour les dents.

**Revendications pour l'Etat contractant suivant : ES**

**1.** Procédé pour la préparation d'esters (méth)-acryliques de siloxanes contenant des groupes tricyclodécane, répondant à la formule :

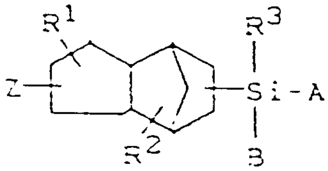 (I),

dans laquelle

| | |
|---|---|
| $R^1$ et $R^2$ | sont identiques ou différents et représentent un atome d'hydrogène, un groupe alkyle en $C_1$-$C_6$ à chaîne droite ou ramifiée, un atome d'halogène ou un groupe trifluorométhyle, |
| $R^3$ | représente un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué, |
| Z | représente le groupe |

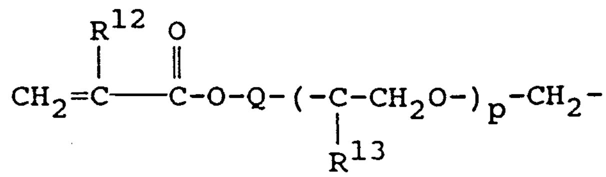

| | |
|---|---|
| | dans lequel |
| $R^{12}$ et $R^{13}$ | sont identiques ou différents et représentent un atome d'hydrogène ou un groupe méthyle, |
| p | représente un nombre de 0 à 20 et |
| Q | représente une liaison simple ou un radical de formule |

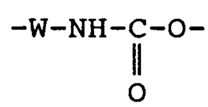

| | |
|---|---|
| | dans laquelle |
| W | représente une chaîne alkylène ou bien représente un radical de formule |

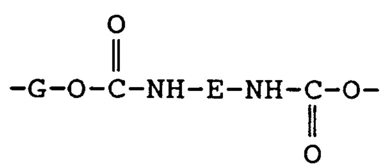

| | |
|---|---|
| | dans laquelle |
| E | représente un radical aliphatique bivalent à chaîne droite ou ramifiée contenant de 2 à 24 atomes de carbone, un radical aromatique contenant de 6 à 26 atomes de carbone, un radical araliphatique contenant de 7 à 26 atomes de carbone ou un radical cycloaliphatique contenant |

de 6 à 26 atomes de carbone, les radicaux aliphatiques, aromatiques, araliphatiques et/ou cycloaliphatiques pouvant contenir 1 ou 2 ponts d'oxygène, et plusieurs des radicaux aliphatiques, aromatiques, araliphatiques et/ou cycloaliphatiques pouvant être reliés à l'intervention de groupes méthylène éventuellement substitués,

et

G représente un radical d'hydrocarbure aliphatique bivalent à chaîne droite ou ramifiée contenant de 3 à 15 atomes de carbone, qui peut éventuellement contenir de 1 à 3 ponts d'oxygène et qui peut être éventuellement substitué par 1 à 4 radicaux (méth)-acryloyloxy supplémentaires,

A représente une chaîne siloxane qui est constituée par m éléments de structure de formules

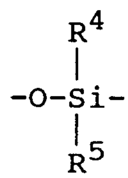

et/ou

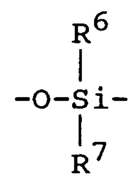

et par le groupe terminal

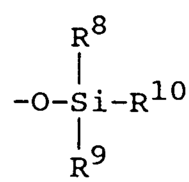

dans lesquels

$R^4$, $R^5$, $R^6$, $R^7$, $R^8$ et $R^9$ sont identiques ou différents et représentent un groupe alkyle en $C_1$-$C_6$ à chaîne droite ou ramifiée, un groupe cycloalkyle, un groupe cycloalkyl-alkyle, ou encore un groupe aryle ou un groupe aralkyle éventuellement substitué,

les radicaux $R^5$, $R^7$ et $R^8$ représentant également le groupe

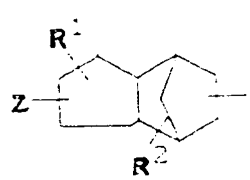

dans lequel

$R^1$, $R^2$ et Z ont la signification mentionnée ci-dessus,

$R^{10}$ représente un groupe alkyle en $C_1$-$C_6$ à chaîne droite ou ramifiée et la somme des éléments de structure m représentant, de manière mutuellement indépendante, un nombre de 0 à 600, et

B peut avoir le domaine de signification analogue à celui de A, les radicaux $R^4$ à $R^{10}$ dans les chaînes A et B pouvant être différents, ou représente un groupe alkyle en $C_1$-$C_6$ à chaîne droite ou ramifiée,

qui se caractérise par le fait qu'on estérifie des poly[hydroxy-(alkylènoxy)méthyl-tricyclo-[5.2.1.0$^{2,6}$]-décanyl]-siloxanes répondant à la formule

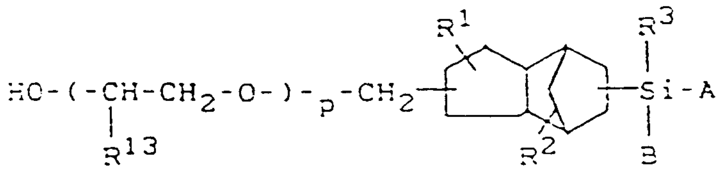

(II),

dans laquelle

$R^1$, $R^2$, $R^3$, $R^{13}$, p, A et B ont la signification mentionnée ci-dessus,

dans le cas d'une préparation de composés dans lesquels Q représente une liaison simple, avec un dérivé de l'acide (méth)-acrylique de formule

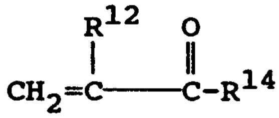

(III)

dans laquelle

$R^{12}$ a la signification mentionnée ci-dessus et $R^{14}$ représente un groupe hydroxyle, un atome de chlore, un groupe méthoxy, un groupe éthoxy, ou un groupe (méth)-acryloyloxy,

ou bien

dans le cas d'une préparation de composés dans lesquels Q représente un radical de formule

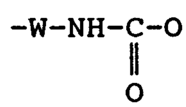

dans laquelle

W représente une chaîne alkylène,

on les fait réagir avec un isocyanate de formule

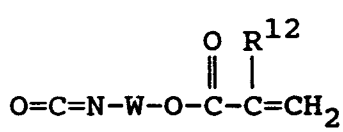

(IV)

dans laquelle

$R^{12}$ et W ont la signification mentionnée ci-dessus,

dans un solvant inerte,

ou bien

dans le cas d'une préparation de composés dans lesquels Q représente un radical de formule

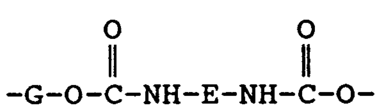

dans laquelle

E représente un radical aliphatique bivalent à chaîne droite ou ramifiée contenant de 2 à 24 atomes de carbone, un radical aromatique contenant de 6 à 26 atomes de carbone, un radical araliphatique contenant de 7 à 26 atomes de carbone ou radical cycloaliphatique contenant de 6 à 26 atomes de carbone, les radicaux aliphatiques, aromatiques, araliphatiques et/ou cycloaliphatiques pouvant contenir 1 ou 2 ponts d'oxygène, et plusieurs des radicaux aliphatiques, aromatiques, araliphatiques et/ou cycloaliphatiques pouvant être reliés à l'intervention de groupes méthylène éventuellement substitués,

et

G représente un radical d'hydrocarbure aliphatique bivalent à chaîne droite ou ramifiée contenant de 3 à 15 atomes de carbone, qui peut éventuellement contenir de 1 à 3 ponts d'oxygène et qui peut être éventuellement substitué par 1 à 4 radicaux (méth)-acryloyloxy supplémentaires,

on les fait réagir avec le produit d'addition de 1 mole d'un diisocyanate de formule

O = C = N-E-N = C = O (V),

dans laquelle

E a la signification mentionnée ci-dessus,

et de 1 mole d'un ester hydroxyalkyl(méth)-acrylique de formule

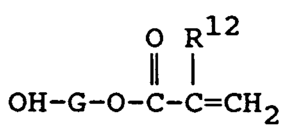

(VI),

dans laquelle

G et $R^{12}$ ont la signification mentionnée ci-dessus,

dans un solvant inerte,

une équivalence stoechiométrique entre les groupes NCO de l'adduit de V et de VI et les groupes OH de II devant exister.

**2.** Procédé selon la revendication 1, caractérisé en ce qu'on effectue la mise en réaction avec le dérivé de l'acide (méth)-acrylique dans un domaine de température de 50 à 120 ° C.